# EUROPEAN PATENT APPLICATION

(11) **EP 4 092 103 A1**
(43) Date of publication of application: **23.11.2022**
(21) Application number: 21741347.5
(22) Date of filing: 18.01.2021
(51) Int. Cl.: C12N 1/20, A61K 35/741, A61P 5/24, A61P 17/10, A61P 17/14, A61P 25/08, A61P 29/00, A61P 31/04, A61P 35/00, C12N 9/04, C12N 15/31, C12N 15/53, C12P 7/40, C12Q 1/00, G01N 33/48, G01N 33/50

(54) **COMPOSITION FOR PRODUCING BILE ACIDS**

(30) Priority: 16.01.2020 US 202062961797 P; 09.04.2020 US 202063007708 P; 21.05.2020 US 202063028077 P; 19.11.2020 US 202063115666 P
(71) Applicant: Keio University, Tokyo, 108-8345 (JP); JSR Corporation, Tokyo 105-8640 (JP)
(72) Inventor: HIROSE Nobuyoshi, Tokyo 160-8582 (JP); HONDA Kenya, Tokyo 160-8582 (JP); SATO Yuko, Tokyo 160-8582 (JP); ATARASHI Koji, Tokyo 160-8582 (JP); NARUSHIMA Seiko, Tokyo 160-8582 (JP); ARAI Yasumichi, Tokyo 160-8582 (JP); TAKESHITA Kozue, Tokyo 160-8582 (JP); SASAJIMA Satoshi, Tokyo 160-8582 (JP)
(74) Representative: J A Kemp LLP
(86) International application number: PCT/JP2021/001434
(87) International publication number: WO 2021/145458

(57) **Abstract**

The present inventors have found that the content ratios of isoalloLCA, 3-oxoLCA, alloLCA, and 3-oxoalloLCA in the feces of centenarians are higher than those of younger ones, and have also identified gut microbiomes peculiar to centenarians involved in the production of these bile acids. Furthermore, it has been found that these bile acids, enzymes involved in the production thereof, and bacteria producing the enzymes reduce the risk of infection with pathogens, prostate cancer, and the like, and are involved in longevity.

## Description

### [Technical Field]

The present invention relates to a composition for producing bile acids. More specifically, the present invention relates to a composition containing a bacterium that produces bile acids related to centenarianism (life span of 100 years or more) and the like as an active ingredient. The present invention also relates to an antibacterial use of the composition against gram-positive bacteria, a therapeutic or preventive use of prostate cancer, and the like.

### [Background Art]

Microbiome (a concept that integrates the genes and functions of microbiota) has been considered to be an important factor that influences the health condition of the elderly, such as resistance to pathogenic bacterial infection, immunity, neural activity, bone density, and digestive and absorptive functions (NPLs 1 to 5). Phenomena of individual disparity and diversity are often found in the microbiota of the elderly, which are thought to be associated with aging of immune functions, chronic systemic inflammation, and senility (NPLs 6 and 7). For the restoration and maintenance of general health related to aging and homeostasis of tissues, it is essential to comprehensively understand the dynamic harmony of the functions of each bacterium constituting microbiota, and to construct a strategy for logically manipulating them.

It is known that centenarians, who are the elderly of age 100 years or older, achieve longevity because they are less susceptible to diseases such as hypertension, diabetes, obesity, and cancer (NPLs 8 and 9). Furthermore, many centenarians have survived infectious diseases such as influenza, pulmonary tuberculosis, dysentery, and salmonella, as well as starvation (NPL 10). This suggests that the microbiota possessed by centenarians not only contributes to longevity, but also to aging health, resilience, and maintenance of homeostasis (NPLs 6 and 11 to 13).

However, the beneficial symbiotic bacteria possessed by centenarians, which contribute to resistance to pathogenic bacterial infection and environmental load, have not yet been clarified.

### [Citation List]

### [Non Patent Literature]

[NPL 1] Nicoletti, C. Age-associated changes of the intestinal epithelial barrier: Local and systemic implications. Expert Rev. Gastroenterol. Hepatol. 9, 1467 to 1469 (2015) .
[NPL 2] Huang, Z. & Kraus, V. B. Does lipopolysaccharide-mediated inflammation have a role in OA? Nat. Rev. Rheumatol. 12, 123 to 129(2016).
[NPL 3] Morais, L. H., Schreiber, H. L. & Mazmanian, S. K. Thegut microbiota-brain axis in behaviour and brain disorders. Nat. Rev. Microbiol. (2020).
[NPL 4] Franceschi, C., Garagnani, P., Parini, P., Giuliani, C. & Santoro, A. Inflammaging: a new immune-metabolic viewpoint for age-related diseases. Nat. Rev. Endocrinol. 14, 576 to 590(2018).
[NPL 5] Santoro, A. et al. Microbiomes other than the gut: inflammaging and age-related diseases. Semin. Immunopathol. (2020).
[NPL 6] Claesson, M. J. et al. Gut microbiota composition correlates with diet and health in the elderly. Nature 488, 178 to 184(2012).
[NPL 7] Claesson, M. J. et al. Composition, variability, and temporal stability of the intestinal microbiota of the elderly. Proc. Natl. Acad. Sci. U. S. A. 108, 4586 to 4591(2011) .
[NPL 8] Andersen, S. L., Sebastiani, P., Dworkis, D. A., Feldman, L. & Perls, T. T. Health span approximates life span among many supercentenarians: Compression of morbidity at the approximate limit of life span. Journals Gerontol. -Ser. A Biol. Sci. Med. Sci. 67A, 395 to 405(2012) .
[NPL 9] Hirata, T. et al. Associations of cardiovascular biomarkers and plasma albumin with exceptional survival to the highest ages. Nat. Commun. 11, 1 to 17(2020).
[NPL 10] Bloom, D. E. & Cadarette, D. Infectious disease threats in the twenty-first century: Strengthening the global response. Front. Immunol. 10, 549(2019).
[NPL 11] Barcena, C. et al. Healthspan and lifespan extension by fecal microbiota transplantation into progeroid mice. Nat. Med. 25, 1234 to 1242(2019).
[NPL 12] Biagi, E. et al. Gut Microbiota and Extreme Longevity. Curr. Biol. 26, 1480 to 1485(2016).
[NPL 13] Wu, L. et al. A cross-sectional study of compositional and functional profiles of gut microbiota in Sardinian centenarians. mSystems 4, e00325-19(2019).

### [Summary of Invention]

### [Technical Problem]

An object of the present invention to be achieved is to identify a beneficial symbiotic bacterium peculiar to centenarians and to provide a method for preventing or treating pathogenic bacterial infections using the bacterium or the like.

### [Solution to Problem]

Centenarians have a low susceptibility to chronic inflammation and aging-related diseases, resulting in longevity. The intestinal microbiota is considered to be an important factor for longevity and health.

In view of the above, the present inventors have conducted intensive studies to achieve the aforementioned object, and have found as a result that, compared to the elderly (85 to 89 years old) and the young (21 to 55 years old), centenarians (average 107 years old) have gut microbiomes that specifically produce secondary bile acids such as isoallo lithocholic acid (lithocholic acid, LCA), isoLCA, 3-oxoLCA, alloLCA, and 3-oxoalloLCA. In particular, the biochemical synthetic pathway that induces isoalloLCA has not been reported so far.

Furthermore, it has been revealed that among 68 bacterial strains isolated from the feces of centenarians, the strains belonging to Parabacteroides merdae and Odoribacteraceae effectively induce isoalloLCA. Furthermore, experiments using a gene-deficient strain of P. merdae have revealed that 3-oxo-5a-steroid-4-dehydrogenase (also known as 5α-reductase (5AR)) and 3β-hydroxysteroid dehydrogenase (3βHSDH) are responsible for the production of isoalloLCA.

It has also been found that these secondary bile acid derivatives have extremely high antibacterial activity against gram-positive multidrug-resistant bacteria such as Clostridioides difficile and vancomycin-resistant Enterococcus faecium.

The present inventors have also found that the above 5AR and 3βHSDH are involved not only in the production of isoalloLCA but also in the metabolism of testosterone. These enzymes, or the bacteria that produce them, deplete tumor-induced testosterone and 5α-dihydrotestosterone (DHT) while producing metastasis-resistant 3β-androstanediol, exerting a therapeutic effect on prostate cancer and the like.

As described above, the present inventors have found that specific secondary bile acid metabolites, enzymes involved in their production, and bacteria that produce these enzymes reduce the risk of pathogenic bacterial infections, prostate cancer, and the like, and contribute to longevity. Thus, the present invention has been completed.

Specifically, the present invention provides the following.
<1> A composition for converting 3-oxo-Δ⁴-LCA to 3-oxoalloLCA, comprising: a bacterium having a polynucleotide encoding 3-oxo-5α-steroid-4-dehydrogenase (5AR) as an active ingredient.
   The present invention also relates to the use of a bacterium having a polynucleotide encoding 5AR for producing a composition for converting 3-oxo-Δ⁴-LCA to 3-oxoalloLCA.
<2> A composition for converting 3-oxoalloLCA to isoalloLCA, comprising: a bacterium having a polynucleotide encoding 3β-hydroxysteroid dehydrogenase (3βHSDH) as an active ingredient.
   The present invention also relates to the use of a bacterium having a polynucleotide encoding 3β hydroxysteroid dehydrogenase (3βHSDH) for producing a composition for converting 3-oxoalloLCA to isoalloLCA.
<3> A composition for producing isoalloLCA from 3-oxo-Δ⁴-LCA, comprising: a bacterium having a polynucleotide encoding 5AR and a bacterium having a polynucleotide encoding 3βHSDH as active ingredients.
   The present invention also relates to the use of a bacterium having a polynucleotide encoding 5AR and a bacterium having a polynucleotide encoding 3βHSDH for producing a composition for producing isoalloLCA from 3-oxo-Δ⁴-LCA.
<4> A composition for producing isoalloLCA from 3-oxo-Δ⁴-LCA, comprising: a bacterium having a polynucleotide encoding 5AR and a polynucleotide encoding 3βHSDH as an active ingredient.
   The present invention also relates to the use of a bacterium having a polynucleotide encoding 5AR and a polynucleotide encoding 3βHSDH for producing a composition for producing isoalloLCA from 3-oxo-Δ⁴-LCA.
<5> A bacterium comprising: a polynucleotide encoding 3-oxo-5β-steroid-4-dehydrogenase (5BR).
<6> A composition for converting 3-oxo-Δ⁴-LCA to 3-oxoLCA, comprising: a bacterium having a polynucleotide encoding 5BR as an active ingredient.
   The present invention also relates to the use of a bacterium having a polynucleotide encoding 5BR for producing a composition for converting 3-oxo-Δ⁴-LCA to 3-oxoLCA.
<7> A composition for producing isoalloLCA from isoLCA or 3-oxoLCA, comprising: a bacterium having a polynucleotide encoding 5AR, a polynucleotide encoding 3βHSDH, and a polynucleotide encoding 5BR as an active ingredient.
   The present invention also relates to the use of a bacterium having a polynucleotide encoding 5AR, a polynucleotide encoding 3βHSDH, and a polynucleotide encoding 5BR for producing a composition for producing isoalloLCA from isoLCA or 3-oxoLCA.
<8> The composition according to any one of <1> to <7>, which is an antibacterial composition against gram-positive bacteria.
<9> An antibacterial composition against gram-positive bacteria, comprising: isoalloLCA or isoLCA as an active ingredient.
<10> An antibacterial composition against gram-positive bacteria, comprising: at least one enzyme selected from 5AR, 3βHSDH, and 5BR as an active ingredient.
<11> The composition according to any one of <8> to <10>, which is a composition for treating or preventing infectious diseases of gram-positive bacteria.
<12> The composition according to any one of <8> to <10>, which is a composition for treating or preventing infectious diseases of Clostridium difficile.
<13> The composition according to any one of <8> to <10>, which is a composition for treating or preventing at least one disease selected from the group consisting of sepsis and acute respiratory distress syndrome (ARDS/ALI) having sepsis as an underlying disease.

The present invention also relates to the use of at least one bacterium selected from the group consisting of a bacterium having a polynucleotide encoding 5AR, a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, and a bacterium having a polynucleotide encoding 5AR, a polynucleotide encoding 3βHSDH, and a polynucleotide encoding 5BR

for producing an antibacterial composition against gram-positive bacteria, a composition for treating or preventing infectious diseases of gram-positive bacteria, a composition for treating or preventing infectious diseases of Clostridium difficile, or a composition for treating or preventing at least one disease selected from the group consisting of sepsis and ARDS/ALI having sepsis as an underlying disease.

The present invention also relates to the use of at least one bacterium selected from the group consisting of a bacterium having a polynucleotide encoding 5AR, a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, and a bacterium having a polynucleotide encoding 5AR, a polynucleotide encoding 3βHSDH, and a polynucleotide encoding 5BR
for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease.

The present invention also relates to at least one bacterium selected from the group consisting of a bacterium having a polynucleotide encoding 5AR, a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, and a bacterium having a polynucleotide encoding 5AR, a polynucleotide encoding 3βHSDH, and a polynucleotide encoding 5BR
for use in growth suppression of gram-positive bacteria, treatment or prevention of infectious diseases of gram-positive bacteria, treatment or prevention of infectious diseases of Clostridium difficile, or treatment or prevention of at least one disease selected from the group consisting of sepsis and ARDS/ALI having sepsis as an underlying disease.

The present invention also relates to a method for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease
which administers to a subject an effective amount of at least one bacterium selected from the group consisting of a bacterium having a polynucleotide encoding 5AR, a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, and a bacterium having a polynucleotide encoding 5AR, a polynucleotide encoding 3βHSDH, and a polynucleotide encoding 5BR.

The present invention also relates to the use of isoalloLCA or isoLCA for producing an antibacterial composition against gram-positive bacteria, a composition for treating or preventing infectious diseases of gram-positive bacteria, a composition for treating or preventing infectious diseases of Clostridium difficile, or a composition for treating or preventing at least one disease selected from the group consisting of sepsis and ARDS/ALI having sepsis as an underlying disease.

The present invention also relates to the use of isoalloLCA or isoLCA for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease.

The present invention also relates to isoalloLCA or isoLCA for use in growth suppression of gram-positive bacteria, treatment or prevention of infectious diseases of gram-positive bacteria, treatment or prevention of infectious diseases of Clostridium difficile, or treatment or prevention of at least one disease selected from the group consisting of sepsis and ARDS/ALI having sepsis as an underlying disease.

The present invention also relates to a method for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease which administers to a subject an effective amount of isoalloLCA or isoLCA.

The present invention also relates to the use of at least one enzyme selected from 5AR, 3βHSDH, and 5BR for producing an antibacterial composition against gram-positive bacteria, a composition for treating or preventing infectious diseases of gram-positive bacteria, a composition for treating or preventing infectious diseases of Clostridium difficile, or a composition for treating or preventing at least one disease selected from the group consisting of sepsis and ARDS/ALI having sepsis as an underlying disease.

The present invention also relates to the use of at least one enzyme selected from 5AR, 3βHSDH, and 5BR for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease.

The present invention also relates to the use of at least one enzyme selected from 5AR, 3βHSDH, and 5BR for use in growth suppression of gram-positive bacteria, treatment or prevention of infectious diseases of gram-positive bacteria, treatment or prevention of infectious diseases of Clostridium difficile, or treatment or prevention of at least one disease selected from the group consisting of sepsis and ARDS/ALI having sepsis as an underlying disease.

The present invention also relates to a method for suppressing the growth of gram-positive bacteria, for treating or preventing infectious diseases of gram-positive bacteria, for treating or preventing infectious diseases of Clostridium difficile, or for treating or preventing at least one disease selected from the group consisting of sepsis and ARDS/ALI having sepsis as an underlying disease which administers to a subject an effective amount of at least one enzyme selected from 5AR, 3βHSDH, and 5BR.
<14> A composition for converting 5α-dihydrotestosterone (DHT) to 3β-androstanediol, comprising: a bacterium having a polynucleotide encoding 3βHSDH as an active ingredient.

The present invention also relates to the use of a bacterium having a polynucleotide encoding 3βHSDH for producing a composition for converting DHT to 3β-androstanediol.
<15> A composition for producing 3β-androstanediol from testosterone, comprising: a bacterium having a polynucleotide encoding 5AR and a bacterium having a polynucleotide encoding 3βHSDH as active ingredients.

The present invention also relates to the use of a bacterium having a polynucleotide encoding 5AR and a bacterium having a polynucleotide encoding 3βHSDH for producing a composition for producing 3β-androstanediol from testosterone.
<16> A composition for producing 3β-androstanediol from testosterone, comprising: a bacterium having a polynucleotide encoding 5AR and a polynucleotide encoding 3βHSDH as an active ingredient.

The present invention also relates to the use of a bacterium having a polynucleotide encoding 5AR and a polynucleotide encoding 3βHSDH for producing a composition for producing 3β-androstanediol from testosterone.
<17> A composition for producing 3β-androstanediol from DHT, comprising: 3βHSDH as an active ingredient.

The present invention also relates to the use of 3βHSDH for producing a composition for producing 3β-androstanediol from DHT.
<18> A composition for producing 3β-androstanediol from testosterone, comprising: 5AR and 3βHSDH as active ingredients.

The present invention also relates to the use of 5AR and 3βHSDH for producing a composition for producing 3β-androstanediol from testosterone.
<19> The composition according to any one of <14> to <18>, which is a composition for treating or preventing at least one disease selected from the following disease group
disease group:
prostate cancer, androgenetic alopecia, polycystic ovary syndrome, acne, hirsutism, ovarian endothelial cancer, neuroinflammation, and GABAA receptor-mediated epilepsy.

The present invention also relates to the use of at least one substance selected from the group consisting of a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, 3βHSDH, 5AR, and 3βHSDH for producing a composition for treating or preventing at least one disease selected from the disease group.

The present invention also relates to the use of at least one substance selected from the group consisting of a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, 3βHSDH, 5AR, and 3βHSDH for treating or preventing at least one disease selected from the disease group.

The present invention also relates to at least one substance selected from the group consisting of a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, 3βHSDH, 5AR, and 3βHSDH for use in the treatment or prevention of at least one disease selected from the disease group.

The present invention also relates to a method for treating or preventing at least one disease selected from the disease group, including administering to a subject an effective amount of at least one substance selected from the group consisting of a bacterium having a polynucleotide encoding 3βHSDH, a bacterium having a polynucleotide encoding 5AR and a bacterium having a polynucleotide encoding 3βHSDH, a bacterium having a polynucleotide encoding 5AR and a polynucleotide encoding 3βHSDH, 3βHSDH, 5AR, and 3βHSDH.
<20> A method for evaluating at least one disease selected from the following disease group
   disease group:
   prostate cancer, androgenetic alopecia, polycystic ovary syndrome, acne, hirsutism, ovarian endothelial cancer, neuroinflammation, and GABAA receptor-mediated epilepsy, comprising:
   (1) quantifying bacteria that produce 3βHSDH in feces of a subject;
   (2) comparing a value obtained by quantifying in step (1) with a corresponding value obtained by quantifying the bacteria of a centenarian; and
   (3) as a result of the comparison in step (2),
      determining that the subject is unlikely to catch the disease when the quantitative value in the feces of the subject is equal to or higher than the corresponding value, and
      determining that the subject is highly likely to catch or highly likely to have the disease when the quantitative value in the feces of the subject is lower than the corresponding value.
<21> A method for preventing or treating at least one disease selected from the following disease group
   disease group:
      prostate cancer, androgenetic alopecia, polycystic ovary syndrome, acne, hirsutism, ovarian endothelial cancer, neuroinflammation, and GABAA receptor-mediated epilepsy,
   comprising:
      in the method according to <20>, administering a bacterium that produces 3βHSDH to the subject who is determined to be highly likely to catch or highly likely to have the disease.
<22> A method for evaluating a possibility of survival over 100 years old, comprising:
   (1) quantifying at least one bile acid selected from the group consisting of 3-oxoLCA, isoalloLCA, 3-oxoalloLCA, alloLCA, 3-oxoLCA, and isoLCA in feces of a subject;
   (2) comparing a value obtained by quantifying in step (1) with a corresponding value obtained by quantifying the bile acid of a centenarian; and
   (3) as a result of the comparison in step (2),
      determining that the subject has a high possibility of survival over 100 years old when the quantitative value in the feces of the subject is equal to or higher than the corresponding value, and
      determining that the subject has a low possibility of survival over 100 years old when the quantitative value in the feces of the subject is lower than the corresponding value.
<23> A method for evaluating a possibility of survival over 100 years old, comprising:
   (1) quantifying bacteria that produce at least one enzyme selected from 5AR, 3βHSDH, and 5BR in feces of a subject;
   (2) comparing a value obtained by quantifying in step (1) with a corresponding value obtained by quantifying the bacteria of a centenarian; and
   (3) as a result of the comparison in step (2),
      determining that the subject has a high possibility of survival over 100 years old when the quantitative value in the feces of the subject is equal to or higher than the corresponding value, and
      determining that the subject has a low possibility of survival over 100 years old when the quantitative value in the feces of the subject is lower than the corresponding value.
<24> A method for increasing a possibility of survival over 100 years old, comprising:
   in the method according to <22> or <23>, administering at least one selected from the group consisting of 3-oxoLCA, isoalloLCA, 3-oxoalloLCA, alloLCA, 3-oxoLCA, isoLCA, and bacteria that produce at least one enzyme selected from 5AR, 3βHSDH, and 5BR to the subject who is determined to have a low possibility of survival over 100 years old.
<25> A method for evaluating resistance to gram-positive bacteria, comprising:
   (1) quantifying at least one bile acid selected from the group consisting of 3-oxoLCA and isoalloLCA in feces of a subject;
   (2) comparing a value obtained by quantifying in step (1) with a corresponding value obtained by quantifying the bile acid of a centenarian; and
   (3) as a result of the comparison in step (2),
      determining that the subject is highly resistant to gram-positive bacteria when the quantitative value in the feces of the subject is equal to or higher than the corresponding value, and
      determining that the subject is lowly resistant to gram-positive bacteria when the quantitative value in the feces of the subject is lower than the corresponding value.
<26> A method for evaluating resistance to gram-positive bacteria, comprising:
   (1) quantifying bacteria that produce at least one enzyme selected from 5AR, 3βHSDH, and 5BR in feces of a subject;
   (2) comparing a value obtained by quantifying in step (1) with a corresponding value obtained by quantifying the bacteria of a centenarian; and
   (3) as a result of the comparison in step (2),
      determining that the subject is highly resistant to gram-positive bacteria when the quantitative value in the feces of the subject is equal to or higher than the corresponding value, and
      determining that the subject is lowly resistant to gram-positive bacteria when the quantitative value in the feces of the subject is lower than the corresponding value.
<27> A method for preventing infectious diseases of gram-positive bacteria, comprising:
   in the method according to <25> or <26>, administering at least one selected from the group consisting of 3-oxoLCA, isoalloLCA, and bacteria that produce at least one enzyme selected from 5AR, 3βHSDH, and 5BR to the subject who is determined to be lowly resistant to gram-positive bacteria.
<28> A method for producing isoalloLCA, comprising: culturing a bacterium having a polynucleotide encoding 3βHSDH in the presence of 3-oxoalloLCA and collecting isoalloLCA produced in the bacterium and/or a culture product thereof.
<29> A method for producing isoalloLCA, comprising: culturing a bacterium having a polynucleotide encoding 5AR and a bacterium having a polynucleotide encoding 3βHSDH in the presence of 3-oxo-Δ⁴-LCA and collecting isoalloLCA produced in the bacteria and/or culture products thereof.
<30> A method for producing isoalloLCA, comprising: culturing a bacterium having a polynucleotide encoding 5AR and a polynucleotide encoding 3βHSDH in the presence of 3-oxo-Δ⁴-LCA and collecting isoalloLCA produced in the bacterium and/or a culture product thereof.
<31> A method for producing isoalloLCA, comprising: culturing a bacterium having a polynucleotide encoding 5AR, a polynucleotide encoding 3βHSDH, and a polynucleotide encoding 5BR in the presence of isoLCA or 3-oxoLCA and collecting isoalloLCA produced in the bacterium and/or a culture product thereof.
<32> A method for producing isoalloLCA, including producing isoalloLCA from 3-oxoalloLCA and collecting the isoalloLCA in the presence of 3βHSDH.
<33> A method for producing isoalloLCA, including producing isoalloLCA from 3-oxo-Δ⁴-LCA and collecting the isoalloLCA in the presence of 5AR and 3βHSDH.
<34> A method for producing isoalloLCA, including producing isoalloLCA from isoLCA or 3-oxoLCA and collecting the isoalloLCA in the presence of 5AR, 3βHSDH, and 5BR.

### [Advantageous Effects of Invention]

The present invention makes it possible to produce bile acids related to centenarians. In addition, the bile acids, enzymes involved in their production, bacteria that produce the enzymes, and the like make it possible to treat or prevent infectious diseases of gram-positive bacteria, prostate cancer, and the like. Furthermore, the present invention also makes it possible to evaluate the resistance to infectious diseases of gram-positive bacteria, the possibility of suffering from prostate cancer and the like, and the possibility of survival over 100 years old, by using the amount of these bacteria or the like in feces as an index.

### [Brief Description of Drawings]

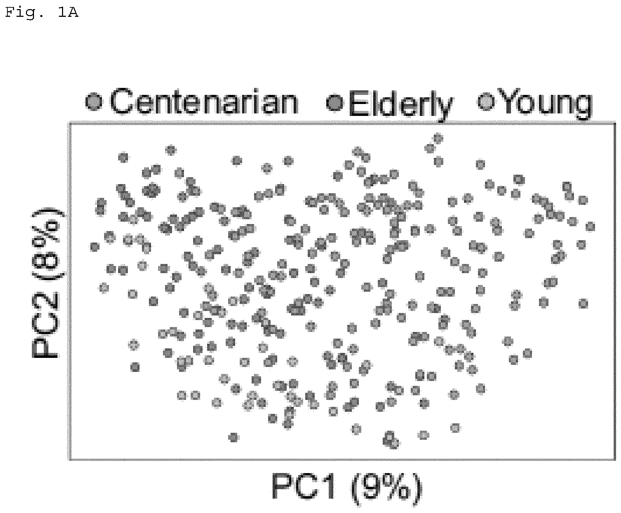

[Fig. 1A] Fig. 1A is a PCoA plot of β-diversity showing the dissimilarity of Bray-Curtis. It is shown that the dots gradually separate from the intestinal microbiota of younger subjects (elderly and young) to the intestinal microbiota of centenarians. In the figure, "Centenarian (or CE)", "Elderly", and "Young" indicate centenarians, the elderly, and the young, respectively (the same applies to the following drawings). Figs. 1A to 1G show the characteristics of the gut microbiome of subjects (Japanese long-lived people aged 100 years or older (centenarians), the elderly, and the young) based on whole metagenomic shotgun sequencing and de novo assembly analysis.

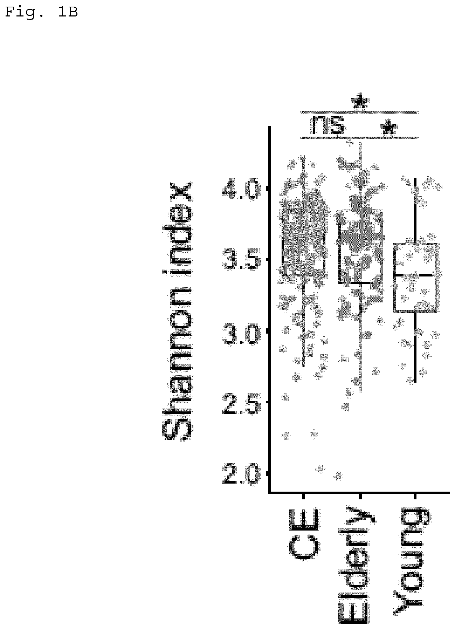

[Fig. 1B] Fig. 1B is a dot plot diagram showing the Shannon diversity index, which was significantly increased in centenarian and elderly subjects compared to the young (P < 0.05, linear model).

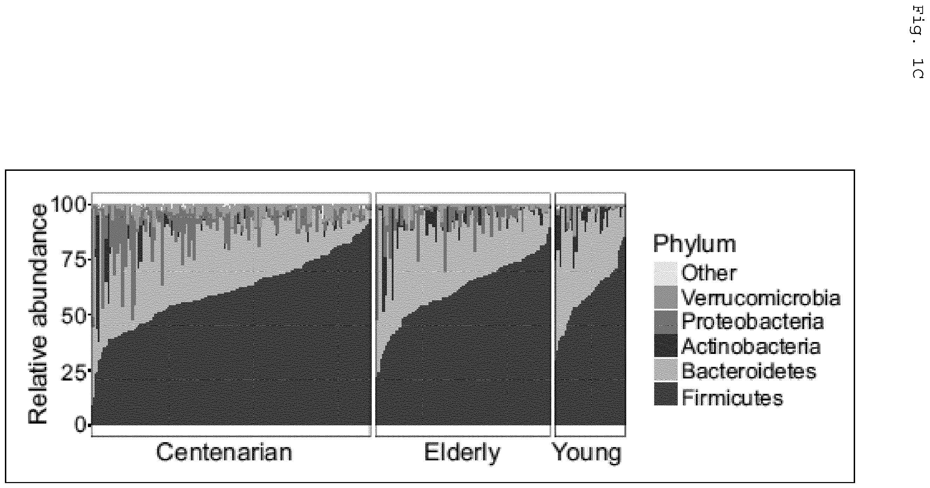

[Fig. 1C] Fig. 1C is a graph showing the relative abundance of five bacterial phyla having a large abundance.

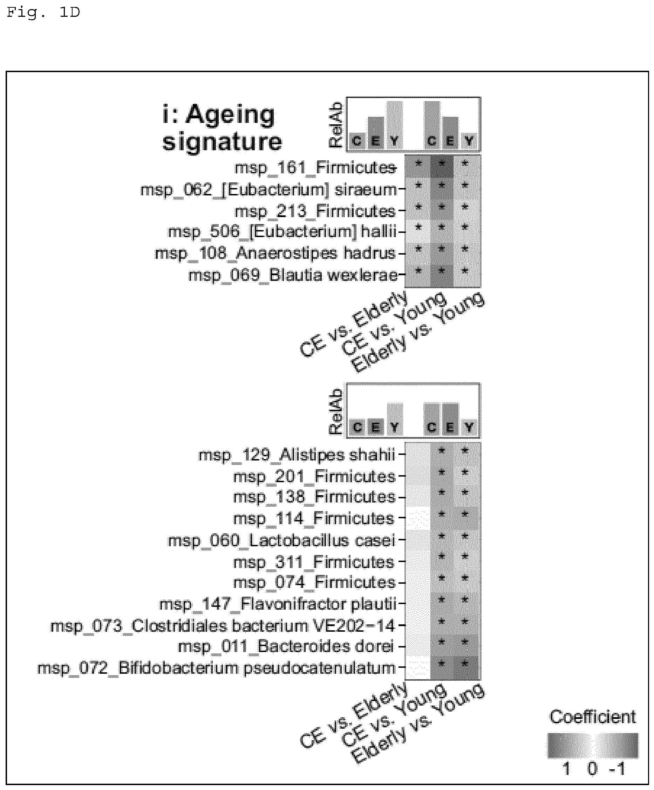

[Fig. 1D] Fig. 1D is a graph showing changes in relative abundance (RelAb) of gut microbiome species (MSPs) between centenarian, elderly, and young controls, grouped according to the characteristics of old age for different abundances.

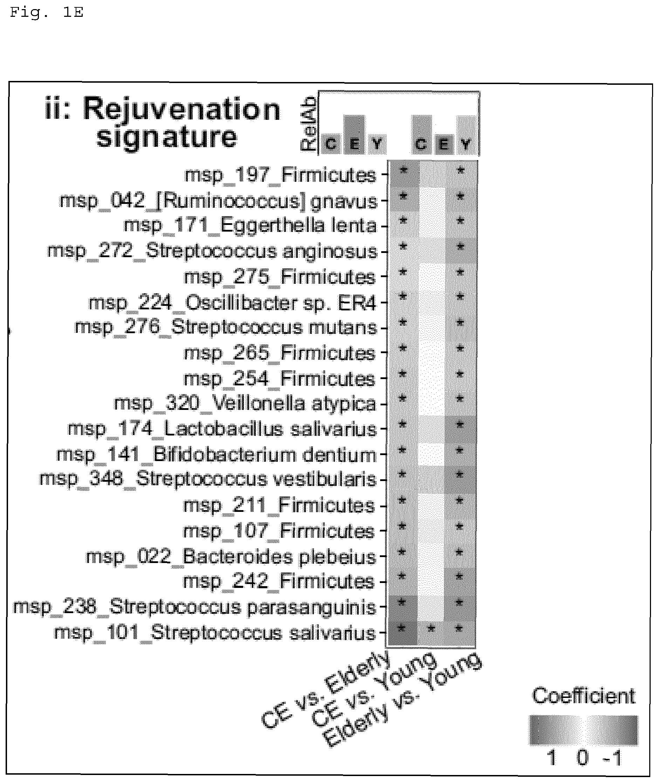

[Fig. 1E] Fig. 1E is a graph showing changes in relative abundance of gut microbiome species between centenarian, elderly, and young controls, grouped according to the characteristics of young age for different abundances.

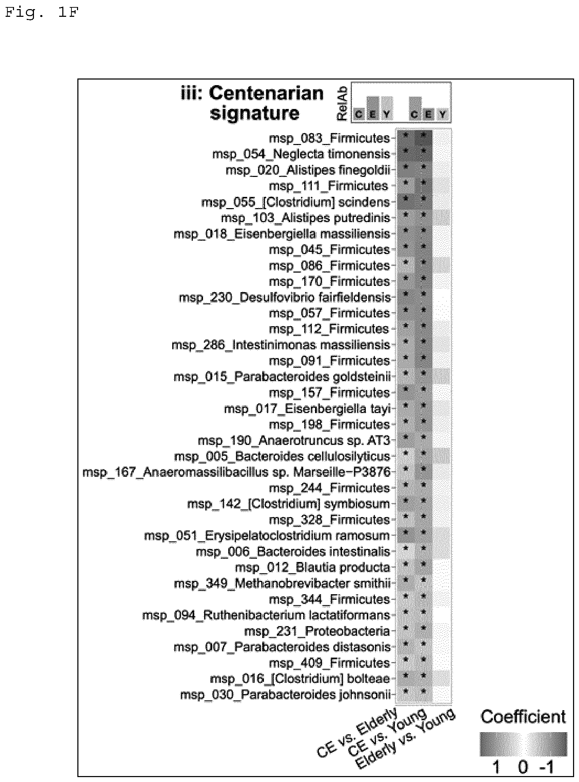

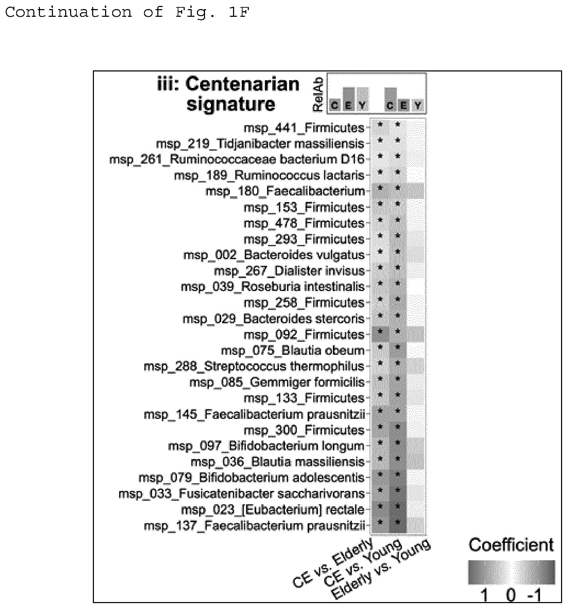

[Fig. 1F] Fig. 1F is a graph showing changes in relative abundance of gut microbiome species between centenarian, elderly, and young controls, grouped according to the characteristics of centenarianism for different abundances. In Figs. 1D to 1F, each characteristic shows a model representing different relative abundance situations for species belonging to a predetermined trajectory within a group of centenarians, the elderly, and the young. The color scale represents the coefficients from the linear model and shows the increase ("red" under color display) and decrease ("blue" under color display) of the species in each comparison. Centenarians compared to the elderly, centenarians compared to the young, elderly compared to the young; in each case the latter group was used as a reference model. Bacterial species with significantly different abundances at FDR P < 0.05 are indicated by asterisks.

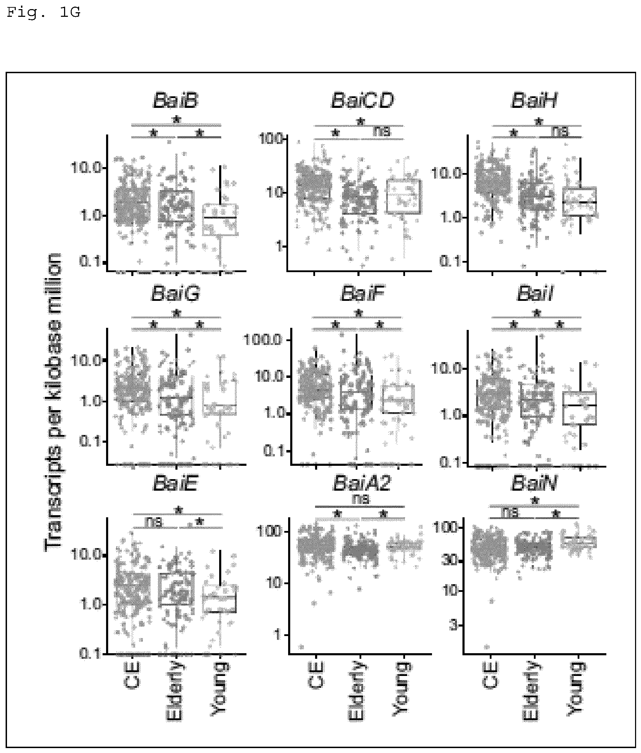

[Fig. 1G] Fig. 1G is a dot plot diagram showing the abundance of homologs for the genes of the C. scindens bai operons (excluding baiA2 and baiN) of the centenarian, elderly, and young groups. Each asterisk indicates that the abundance is significantly different in FDR P < 0.05 based on the Wilcoxon rank sum test. Number of subjects: Centenarian [n = 176 (153 people)], elderly (n = 110), young (n = 44). Each dot indicates an individual subject.

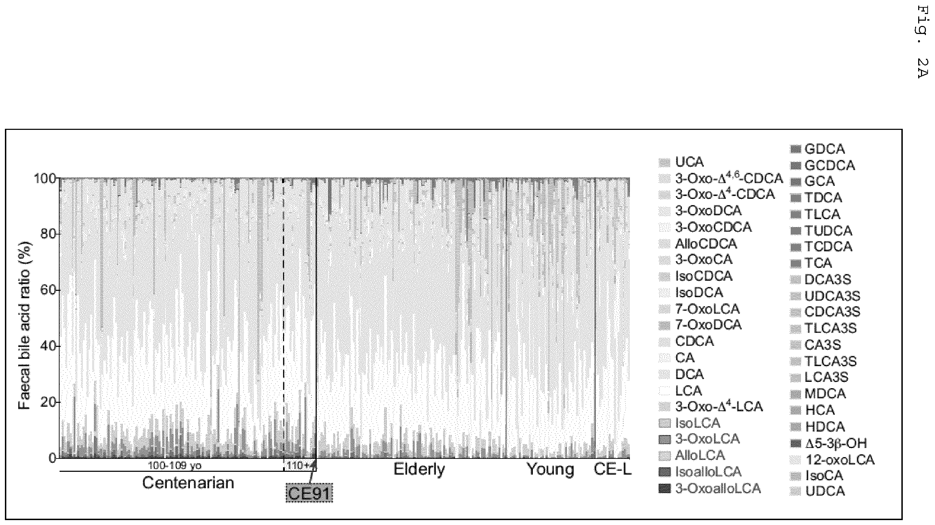

[Fig. 2A] Fig. 2A is a graph showing the results of analyzing and quantifying bile acids in the feces of individual subjects by LC-MS/MS for centenarians (n = 127), the elderly (n = 108), the young (n = 47), and lineal descendants of centenarians (denoted as "CE-L" in the figure (hereinafter the same), n = 18). Under color display, the ratio of 3-oxoalloLCA is shown in blue, the ratio of isoalloLCA is shown in red, the ratio of alloLCA is shown in green, the ratio of 3-oxoLCA is shown in light blue, and the ratio of isoLCA is shown in yellow. The results are shown in order of age within each group. Centenarian 91 (CE91) that the present inventors paid attention to is shown by a dotted line frame. Figs. 2A to 2G show that the feces of centenarians have significantly higher contents of isoLCA, 3-oxoLCA, alloLCA, isoalloLCA, and 3-oxoalloLCA.

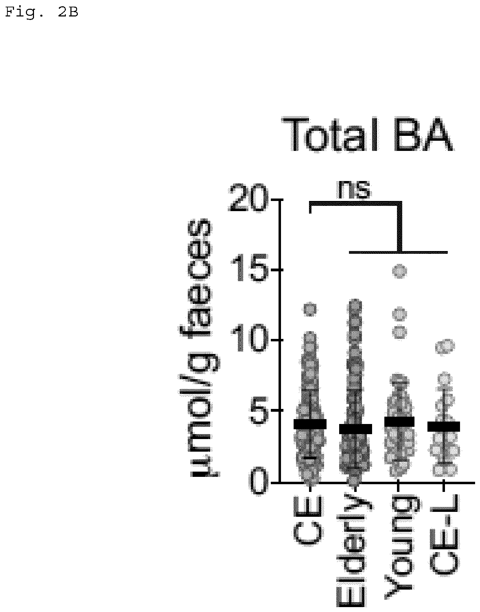

[Fig. 2B] Fig. 2B is a dot plot diagram showing the results of quantifying the total amount of bile acid compounds in feces for individuals of four age groups.

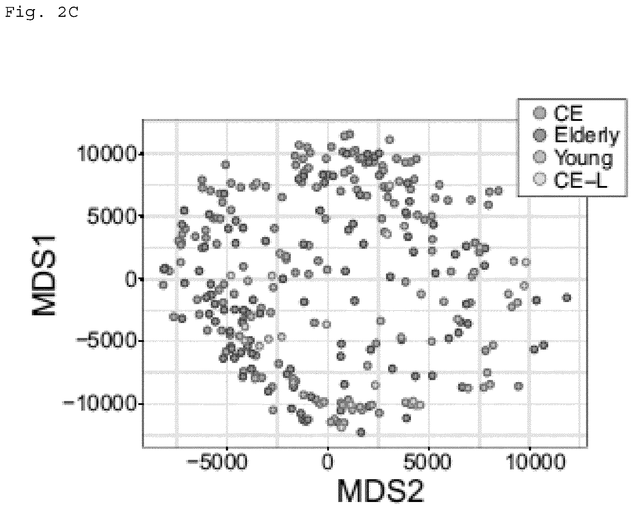

[Fig. 2C] Fig. 2C is a multidimensional scaling plot (MDS) using Spearman's correlation showing differences between the results of analyzing the bile acids of individuals. Each dot shows an individual donor color-coded by age group (centenarian vs. elderly; P = 4.27E-9, centenarian vs. young; P = 2.72E-12, centenarian vs. lineal descendant of centenarian; P = 4.18E-6, elderly vs. young; P = 0.00123, Wilcoxon test).

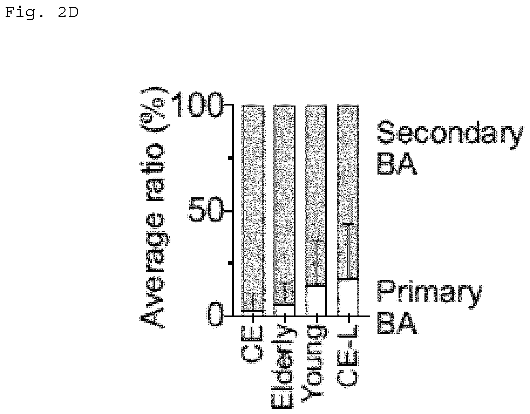

[Fig. 2D] Fig. 2D is a graph showing the average ratio of total primary bile acid (Primary BA) and total secondary bile acid (Secondary BA) metabolized by gut microbiomes.

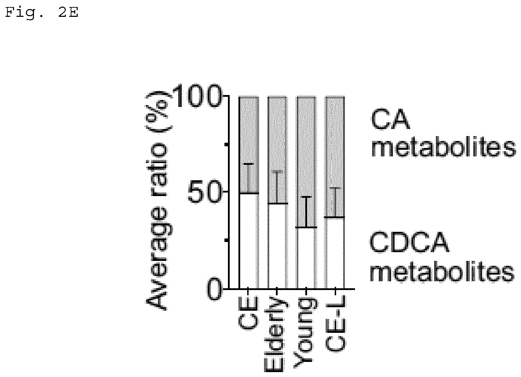

[Fig. 2E] Fig. 2E is a graph showing the average ratio of CA-based bile acid (having 7α- and 12α-OH groups) and CDCA-based bile acid (having 7α-OH groups).

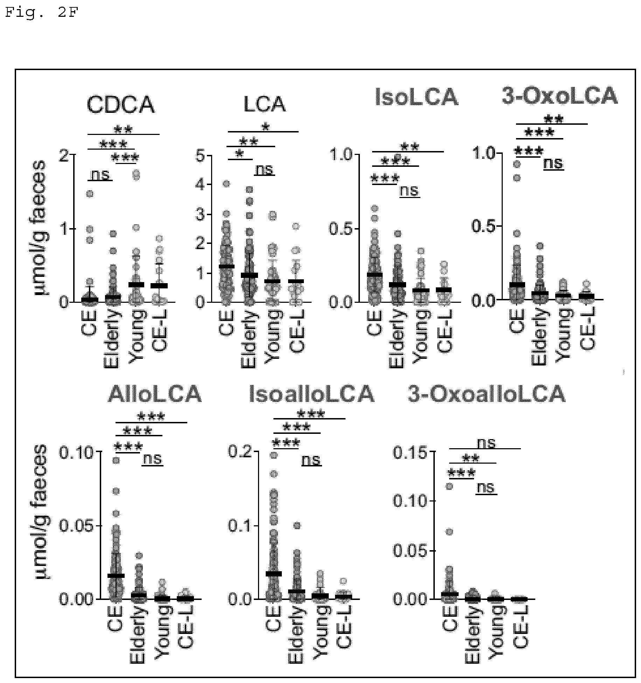

[Fig. 2F] Fig. 2F is a graph showing the quantitative results of each bile acid compound in feces. In Figs. 2B and 2D to 2F, the data is shown as the mean + standard deviation. *** P < 0.001; ** P < 0.01; * P < 0.05; one-way analysis of variance with Tukey's test. ns indicates that there is no significant difference. Each dot indicates an individual. The total amount of bile acid in feces is represented in µmol/g based on the wet weight of feces.

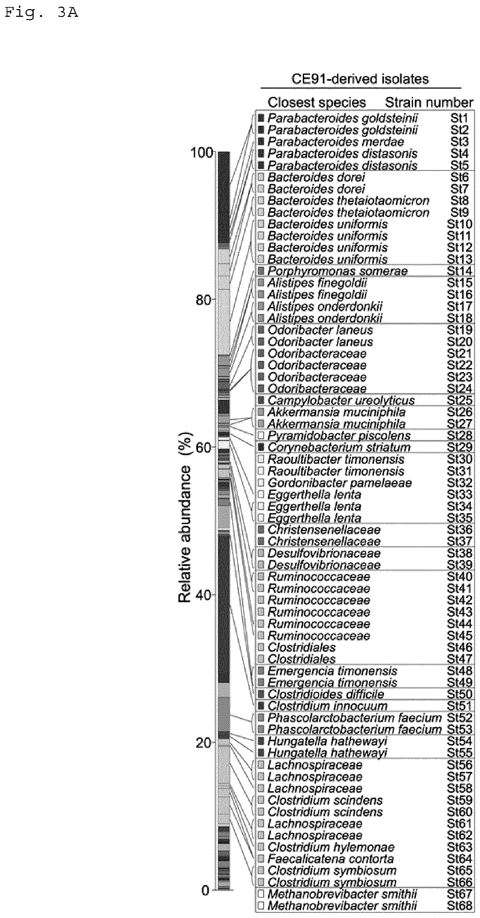

[Fig. 3A] Fig. 3A is a diagram showing the fecal bacterial composition of CE91. The 68 bacterial strains isolated from the feces of CE91 are shown along with their relative abundance. Figs. 3A to 3E show the identified bacterial strains and genes involved in the production of isoLCA, 3-oxoLCA, and isoalloLCA.

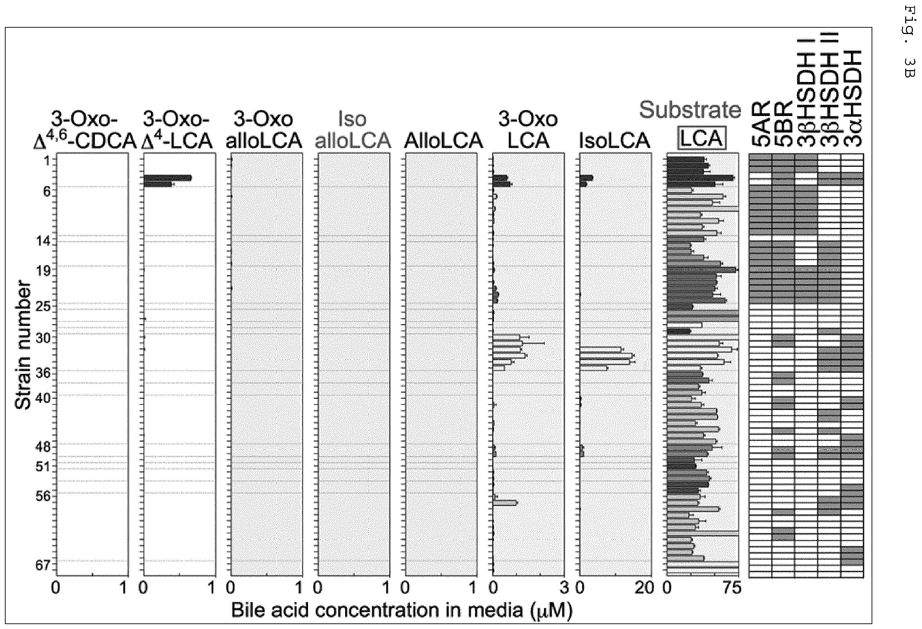

[Fig. 3B] Fig. 3B is a graph showing the results of bile acid metabolism using 50 µM LCA as a substrate by 68 strains derived from CE91.

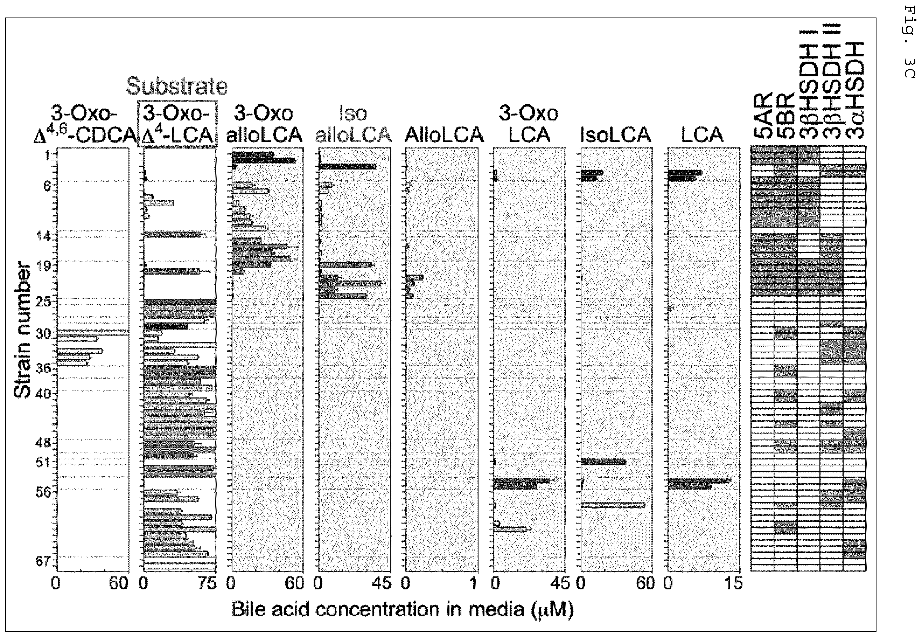

[Fig. 3C] Fig. 3C is a graph showing the results of bile acid metabolism using 50 µM 3-oxo-Δ⁴-LCA as a substrate by 68 strains derived from CE91. In Figs. 3B and 3C, the isolated bacteria were cultured under anaerobic conditions in a medium adjusted to pH 9 for 48 hours at 37°C. In these drawings, the presence or absence of the predicted bile acid metabolizing enzyme gene homologs is shown in the waffle chart on the right side. Homologue was defined as <1e-12 E value;> 30% sequence matching degree; > 60% query coverage. Further, in these drawings, a graph region lightly colored in red under color display indicates a compound having a trans-type chemical structure, and blue indicates a compound having a cis-type chemical structure.

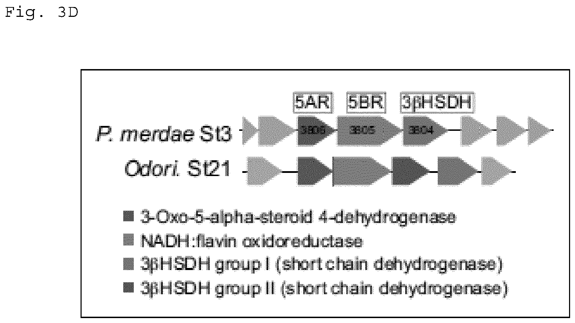

[Fig. 3D] Fig. 3D is a schematic diagram of predicted bile acid metabolizing enzyme genes in P. merdae St3 and Odoribacteraceae St21. The arrows indicate the coding sequence. In addition, they are color-coded based on the identified function under color display.

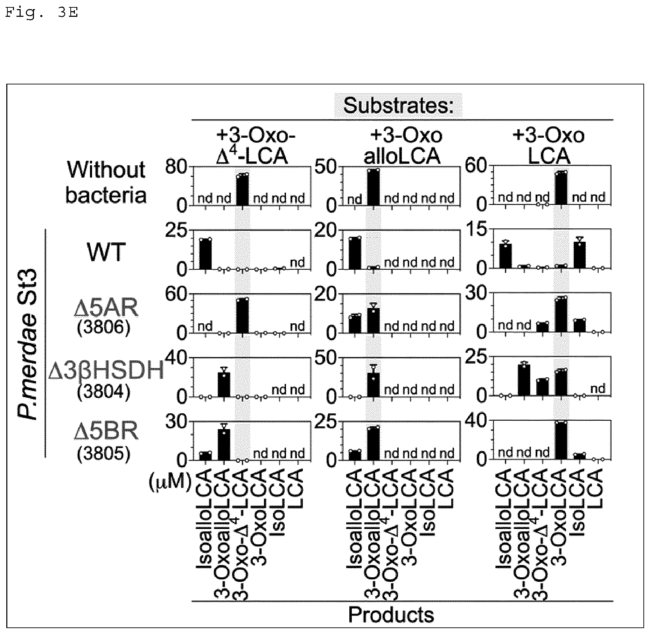

[Fig. 3E] Fig. 3E is a graph showing the results of a bile acid metabolism assay in a 5AR, 3βHSDH, or 5BR gene-deficient strain of P. merdae St3. To a medium adjusted to pH 9, 50 µM 3-oxoalloLCA, 3-oxo-Δ⁴-LCA, or 3-oxoLCA was added as a substrate, which was cultured for 48 hours at 37°C. Under color display, the detected substrate concentration is shown in light yellow. nd indicates undetected. Figs. 3B, 3C, and 3E show bile acid concentrations determined by LC-MS/MS. The data is shown as the mean + standard deviation of N = 2 samples.

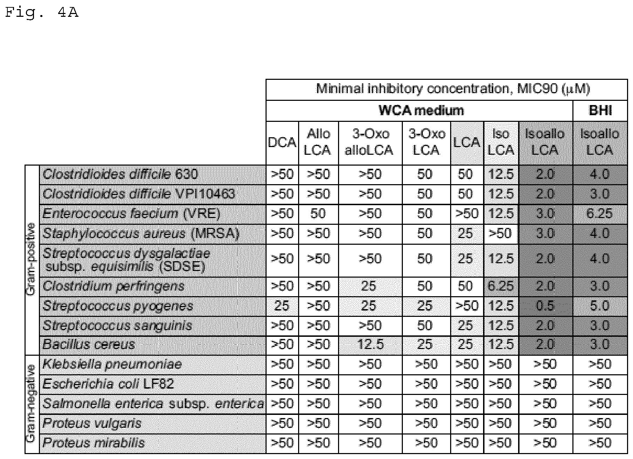

[Fig. 4A] Fig. 4A shows the minimum inhibitory concentration (MIC90) of secondary bile acids in WCA or BHI medium for gram-positive pathogens and gram-negative pathogens.

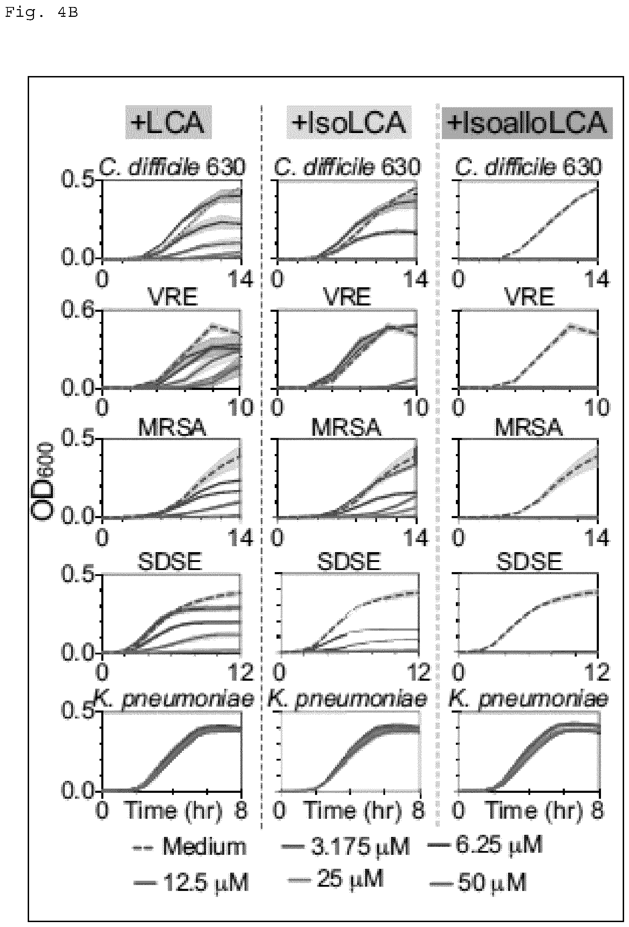

[Fig. 4B] Fig. 4B is a graph showing growth curves of pathogens in a WCA medium in which the concentration of each secondary bile acid is changed. Under color display, LCA, isoLCA, and isoalloLCA are shown in blue, yellow, and green, respectively. The data is shown as the mean and standard deviation. Error bars are represented by filled areas.

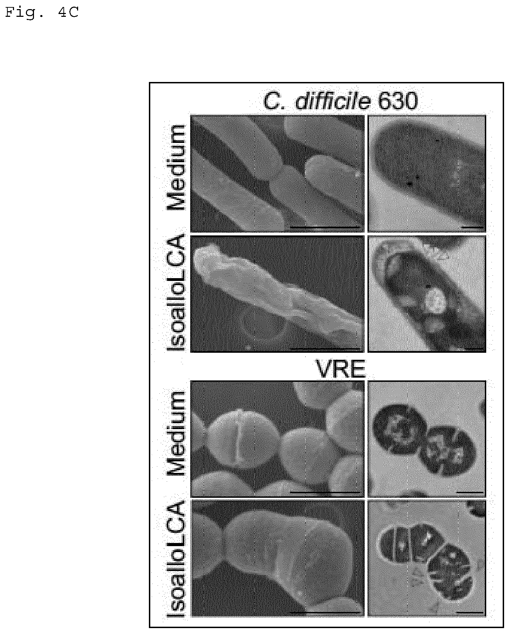

[Fig. 4C] Fig. 4C is typical scanning electron microscope images (SEM, left panels) and transmission electron microscope images (TEM, right panels) when C. difficile 630 and VRE were cultured for 5 hours in a WCA medium containing or not containing 8 µM isoalloLCA. The scale bars indicate 1.0 µm (SEM), 200 nm (C. difficile 630 TEM), and 500 nm (VRE TEM). The arrows indicate the shape change after isoalloLCA treatment.

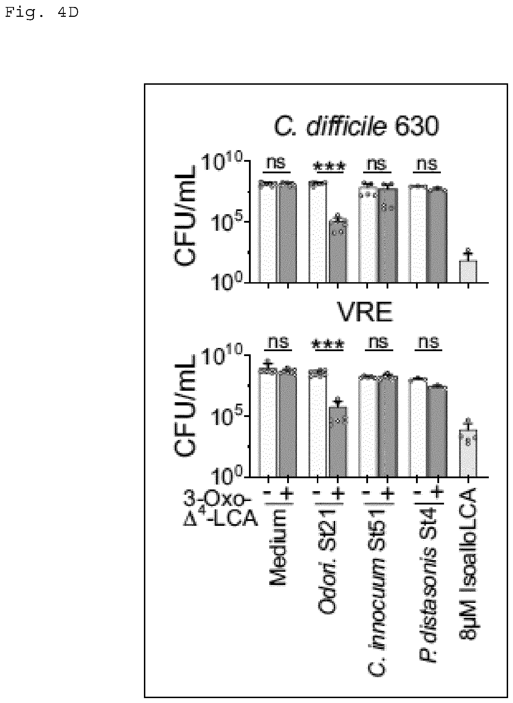

[Fig. 4D] Fig. 4D is a graph showing the results of suppressing in vitro growth of C. difficile 630 or VRE when co-cultured with Odoribacteraceae St21, C. innocuum St51, or P. distasonis St4 derived from CE91 in a WCA medium containing 12.5 µM 3-oxo-Δ⁴-LCA. CFU when cultured overnight is shown (n = 6).

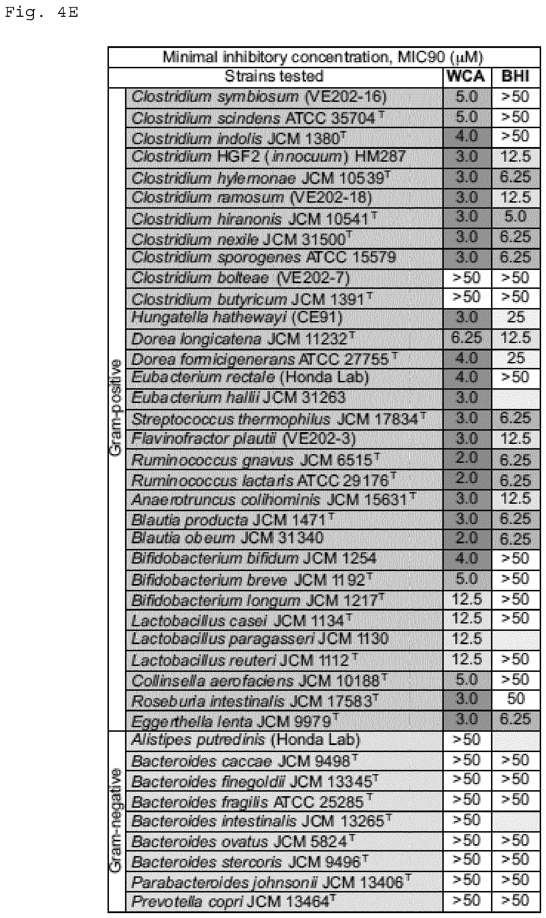

[Fig. 4E] Fig. 4E is a diagram showing MIC90 of isoalloLCA for a symbiotic strain in a WCA medium or BHI medium.

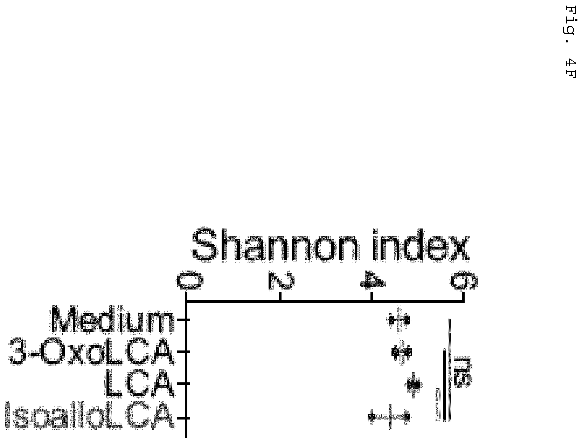

[Fig. 4F] Fig. 4F is a dot plot diagram showing the Shannon index for the diversity of fecal culture broths after secondary bile acid treatment. The results are shown of incubation of human fecal samples of healthy young donors in improved WCA medium supplemented with 3-oxoLCA, LCA, or isoalloLCA (50 µM) for 48 hours. In Figs. 4D and 4F, the data is shown as the mean + standard deviation. *** indicates that P < 0.001. Fig. 4D shows the results of the Mann-Whitney test with Welch correction. Fig. 4F shows the results of the Kruskal-Wallis test with Dunn's test. ns indicates that there is no significant difference. Each dot shows the result of one microtiter plate in Fig. 4D and the result of culturing the donors' feces in Fig. 4F.

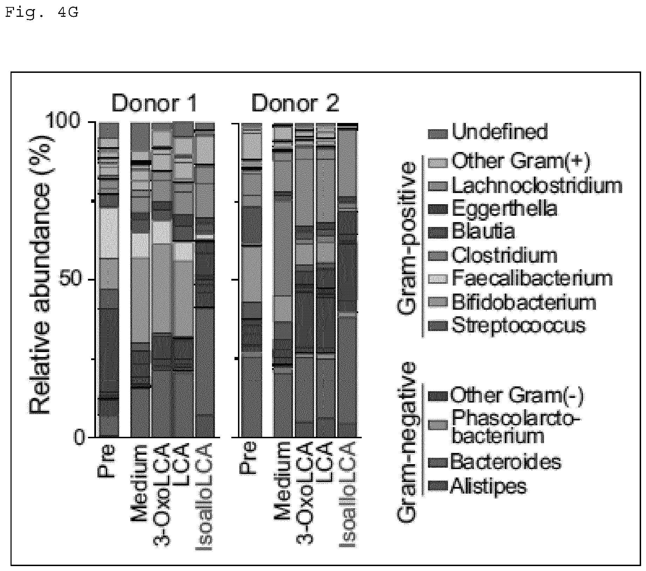

[Fig. 4G] Fig. 4G is a graph showing changes in the composition of intestinal microbiota at the genus level. The results are shown of incubation of human fecal samples of healthy young donors in improved WCA medium supplemented with 3-oxoLCA, LCA, or isoalloLCA (50 µM each) for 48 hours.

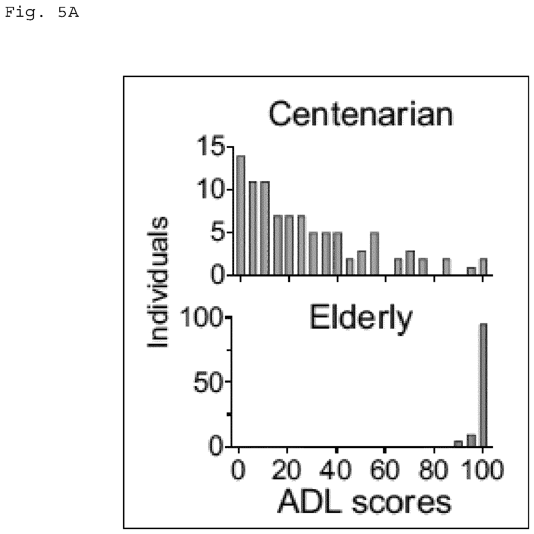

[Fig. 5A] Fig. 5A is a graph showing activities of daily living (ADL) estimated by the Barthel indices of the centenarian group ("upper" in the figure, ADL, n = 94) and the elderly group ("lower" in the figure, ADL, n = 112).

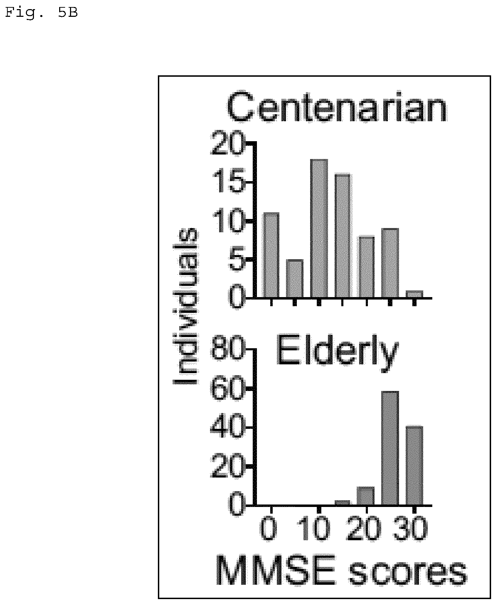

[Fig. 5B] Fig. 5B is a graph showing the mini-mental state examination (MMSE) scores estimated by the Barthel indices of the centenarian group ("upper" in the figure, MMSE, n = 68) and the elderly group ("lower" in the figure, MMSE, n = 111).

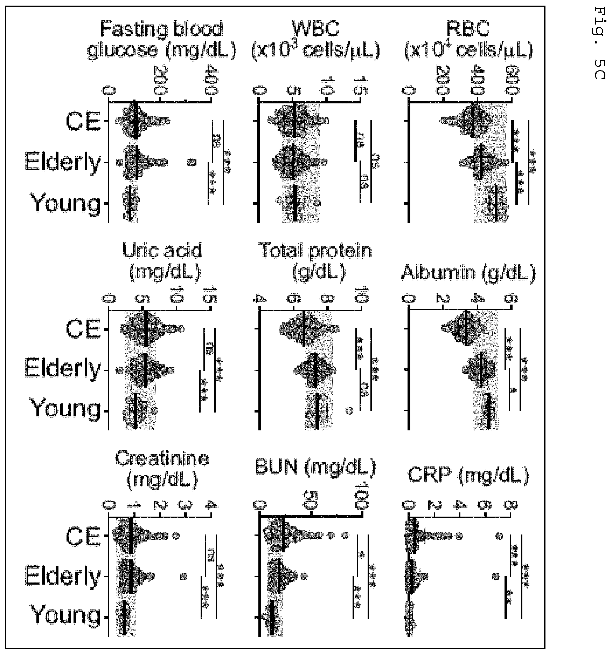

[Fig. 5C] Fig. 5C is a graph showing the results of blood tests of centenarians (n = 149-153), the elderly (n = 111-112), and the young (n = 15-39). Red blood cell count (RBC); albumin; C-reactive protein (CRP); white blood cell count (WBC); total protein; blood urea nitrogen (BUN); fasting blood glucose; uric acid; creatinine.

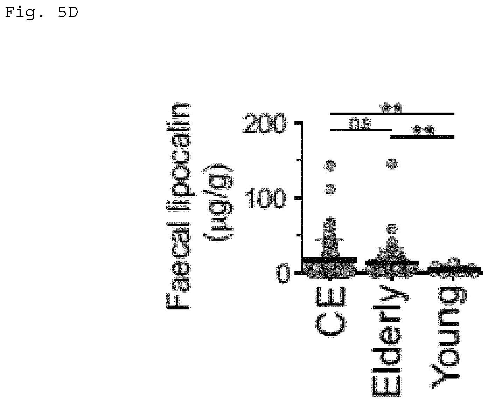

[Fig. 5D] Fig. 5D is a graph showing lipocalin levels in the feces of individual centenarians (n = 122), elderly (n = 67), and young (n = 19) quantified by ELISA.

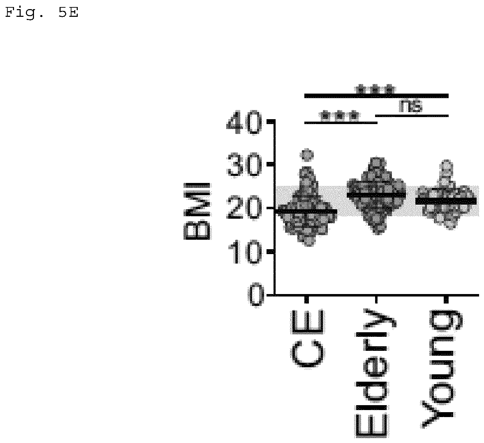

[Fig. 5E] Fig. 5E is a graph showing BMI values of individual centenarians (n = 89), elderly (n = 112), and young (n = 43). In Figs. 5C to 5E, the data is shown as the mean + standard deviation. *** P < 0.001; ** P < 0.01; * P < 0.05; Kruskal-Wallis test with Dunn's test. ns indicates that there is no significant difference. Gray regions indicate the range of normal values.

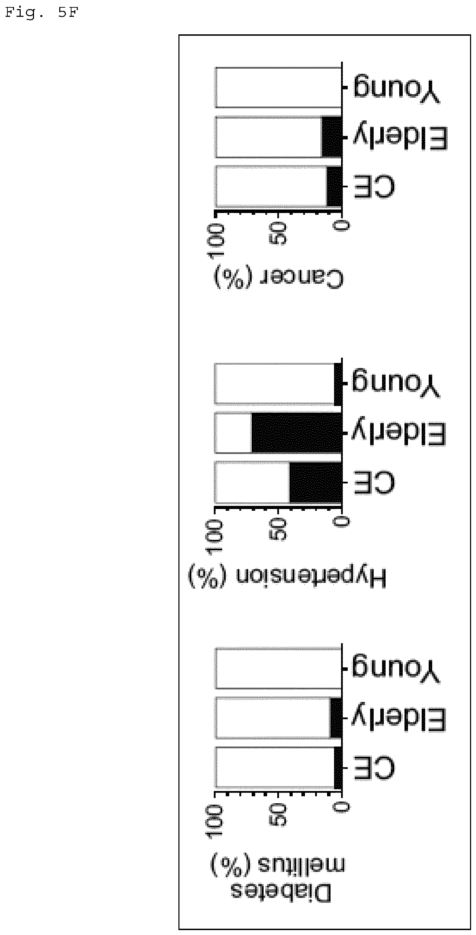

[Fig. 5F] Fig. 5F is a graph showing the proportion of individuals with a history of diabetes, hypertension, and cancer. Black indicates the proportion of people with these disease records. The medical history of 135 centenarians, 112 elderly, and 43 young people was investigated.

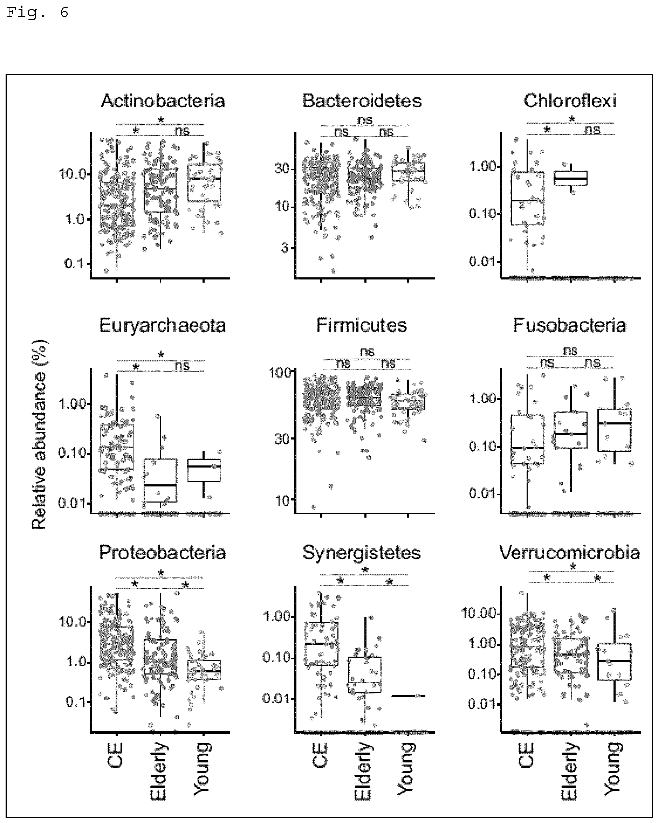

[Fig. 6] Fig. 6 is a dot plot diagram showing the relative abundance of all phyla in the assembled intestinal metagenomes of centenarian, elderly, and young controls. Groups with a significant difference in FDR P < 0.05 in the linear model are indicated by asterisks. Each dot indicates an individual.

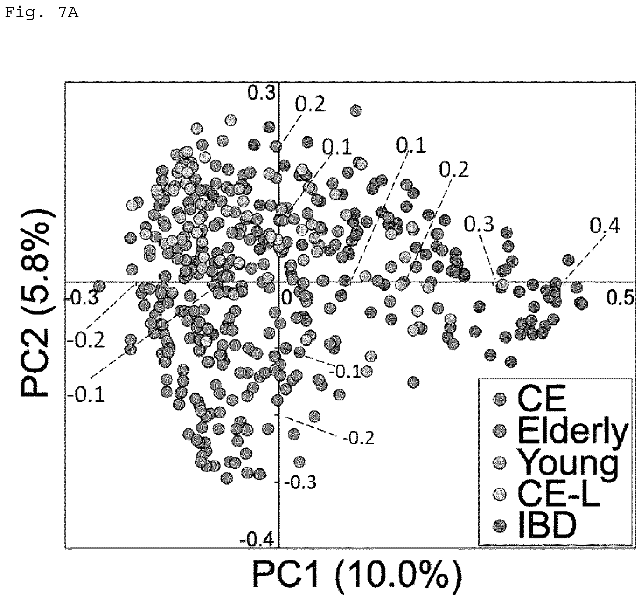

[Fig. 7A] Fig. 7A is a PCoA plot based on an unweighted UniFrac distance matrix between individuals of centenarians, elderly, young, lineal descendants of centenarians, and IBD patients. Under color display, centenarians, elderly, young, lineal descendants of centenarians, and IBD patients are shown in orange, blue, gray, yellow, and red, respectively.

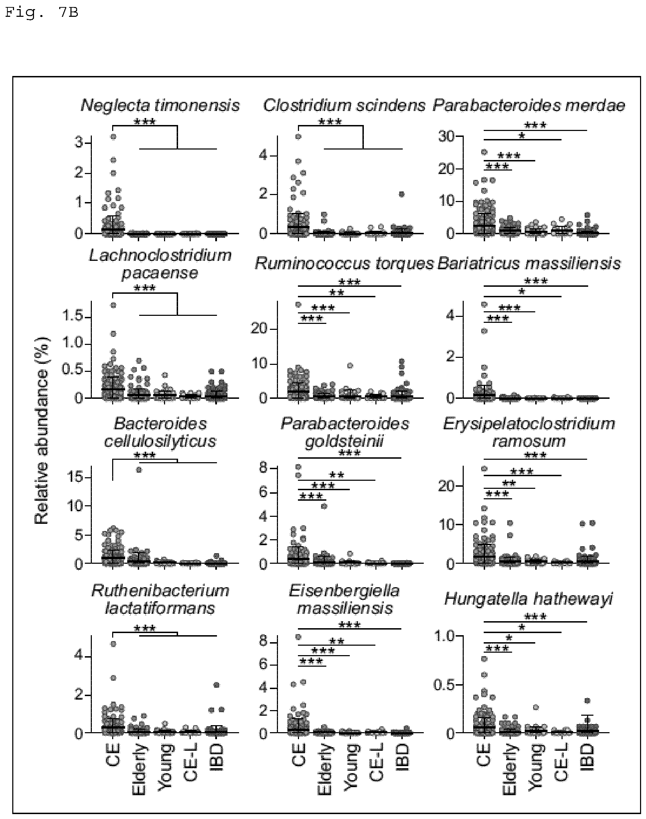

[Fig. 7B] Fig. 7B is a graph showing relative abundance of species significantly increased in centenarians among centenarians, elderly, young, lineal descendants of centenarians, and IBD patients.

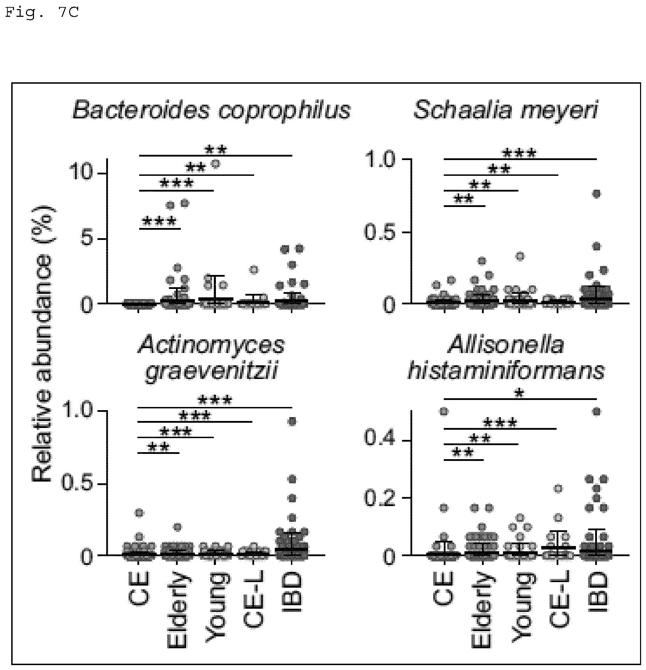

[Fig. 7C] Fig. 7C is a graph showing relative abundance of species significantly decreased in centenarians among centenarians, elderly, young, lineal descendants of centenarians, and IBD patients.

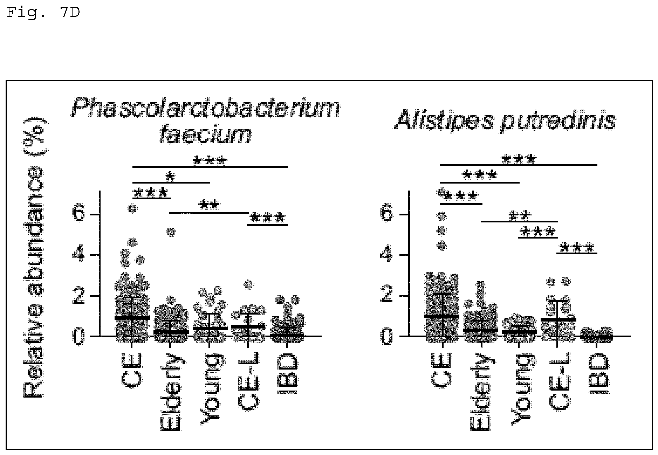

[Fig. 7D] Fig. 7D is a graph showing relative abundance of species characteristic of centenarians and their lineal descendants among centenarians, elderly, young, lineal descendants of centenarians, and IBD patients. In Figs. 7A-7D, the data is shown as the mean + standard deviation. *** P < 0.001; ** P < 0.01; * P < 0.05; post-test of Wilcoxon signed-rank test with Bonferroni correction. ns indicates that there is no significant difference. Dots indicate individuals. Figs. 7A-7D show the results of sequencing 16S rRNA genes in the feces of centenarians (n = 160), elderly (n = 112), young (n = 40), lineal descendants of centenarians (n = 22, 48 to 95 years old, avg. 74.5 + 11.6 years old), and inflammatory bowel disease (IBD) patients (n = 103, 15 to 78 years old, avg. 49 + 1.51 years old).

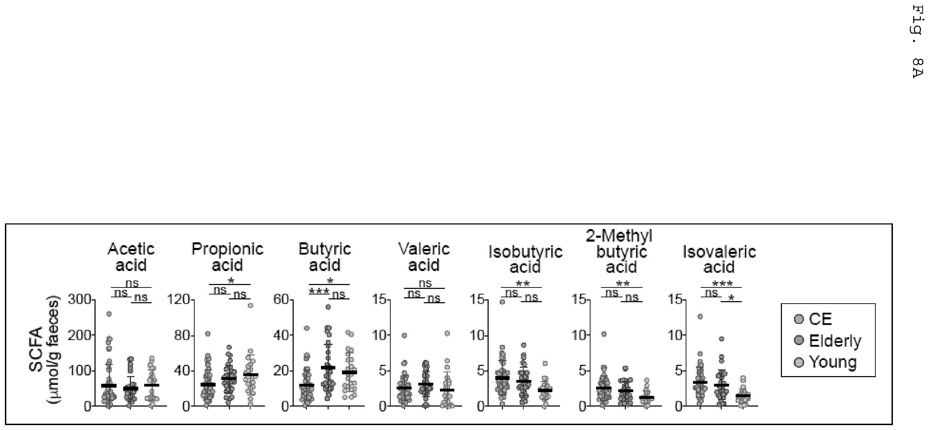

[Fig. 8A] Fig. 8A is a dot plot diagram showing the results of quantifying short-chain fatty acids in the feces of centenarians (n = 47), elderly (n = 31), and young (n = 23) by GC/MS. The amount of short-chain fatty acids in the feces is expressed in µmol/g based on the wet weight of feces.

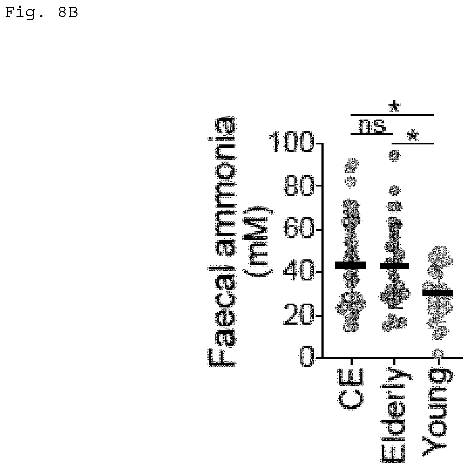

[Fig. 8B] Fig. 8B is a dot plot diagram showing the ammonia concentration in the feces of each centenarian, elderly, and young individual.

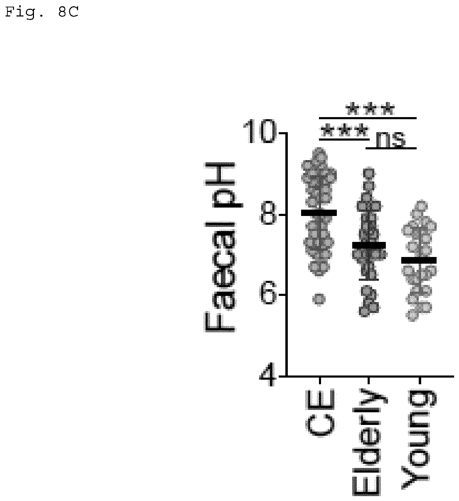

[Fig. 8C] Fig. 8C is a dot plot diagram showing the fecal pH level in the feces of each centenarian, elderly, and young individual. In Figs. 8A-8C, the data is shown as the mean + standard deviation. *** P < 0.001; ** P < 0.01; * P < 0.05; one-way analysis of variance with Tukey's test. ns indicates that there is no significant difference.

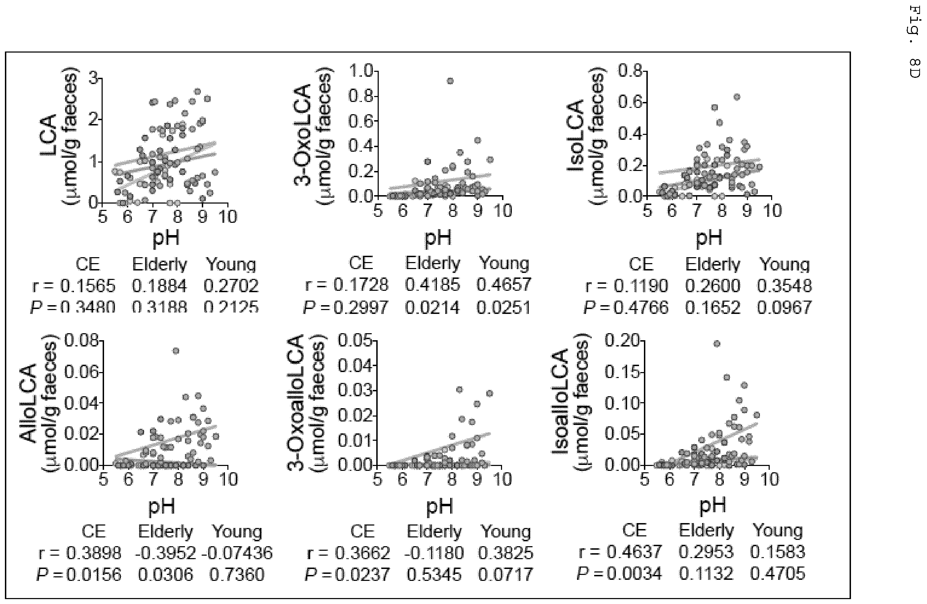

[Fig. 8D] Fig. 8D is a scatter plot diagram showing the correlation between the fecal pH level and each secondary bile acid by a regression line. Each dot indicates an individual result. Spearman's coefficient (r) and its significance (P) were calculated separately for each group of centenarian (under color display, orange), elderly (under color display, blue), and young (under color display, gray) samples. For centenarians, a significant positive correlation was found between pH and fecal alloLCA level (P = 0.0156), 3-oxoalloLCA level (P = 0.00237), or isoalloLCA level (P = 0.0034).

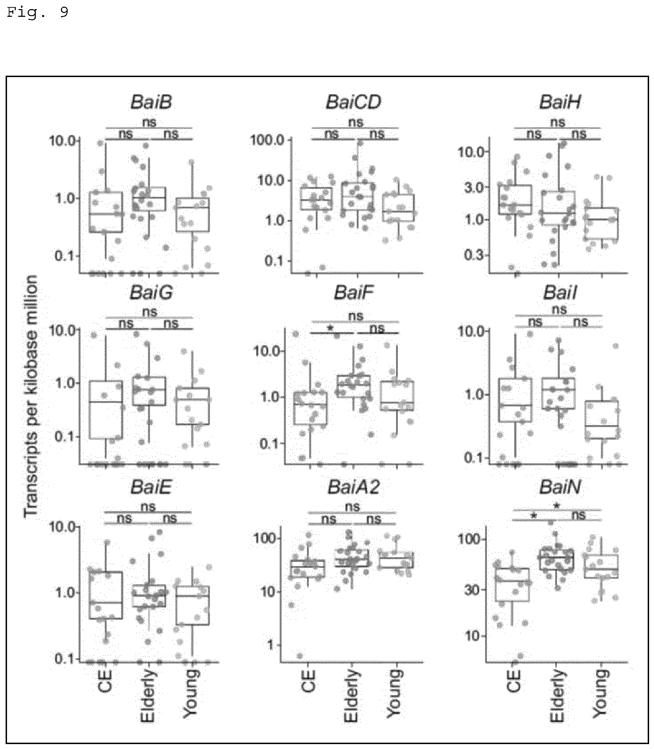

[Fig. 9] Fig. 9 is a dot plot diagram showing the abundance of the C. scindens bai operon gene in a longevity cohort aged 100 years or older in Sardinia. The abundance is shown of homologs for the genes of the C. scindens bai operons (excluding baiA2 and baiN) in the centenarians, elderly control, and young control. Asterisks indicate abundances that are significantly different under FDR P < 0.05 conditions based on the pairwise Wilcoxon test for each abundance.

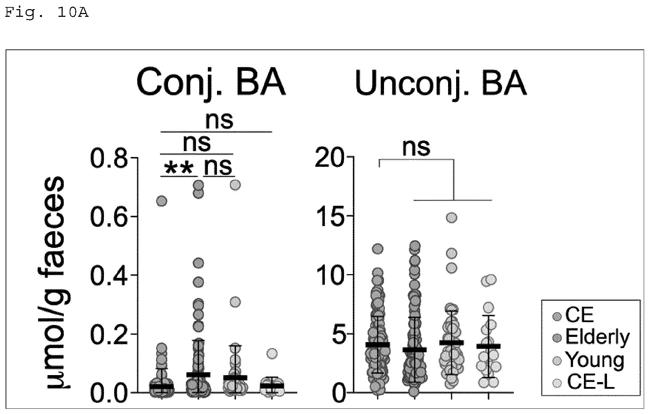

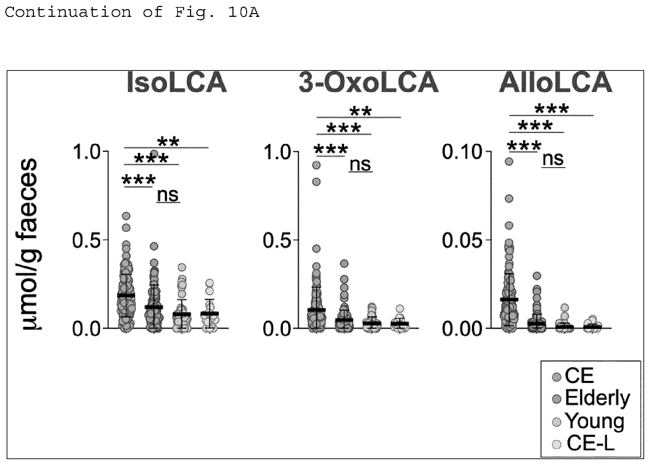

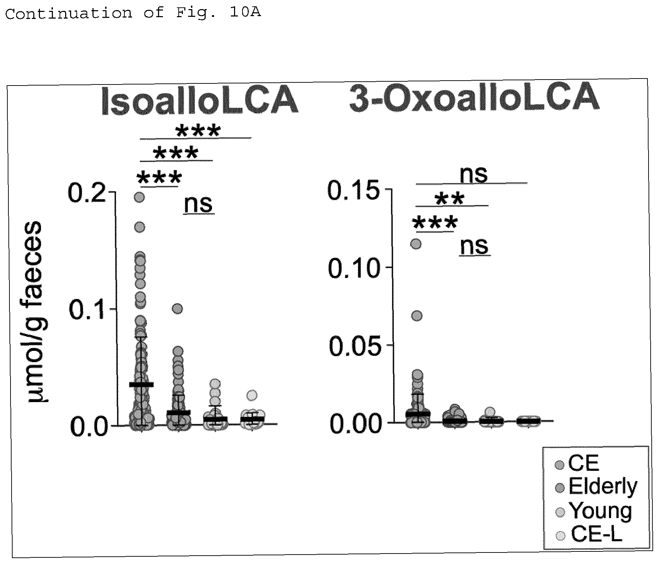

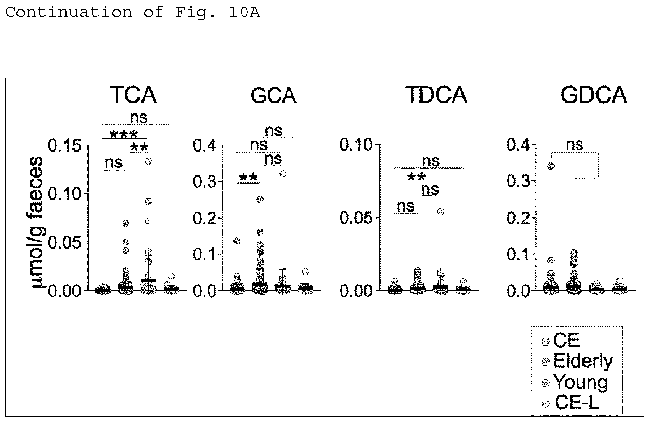

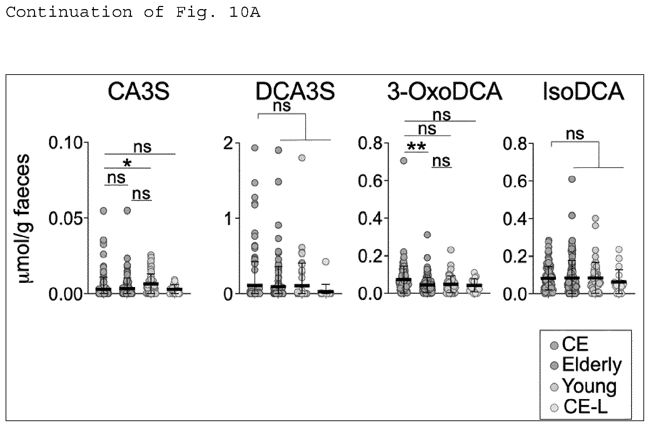

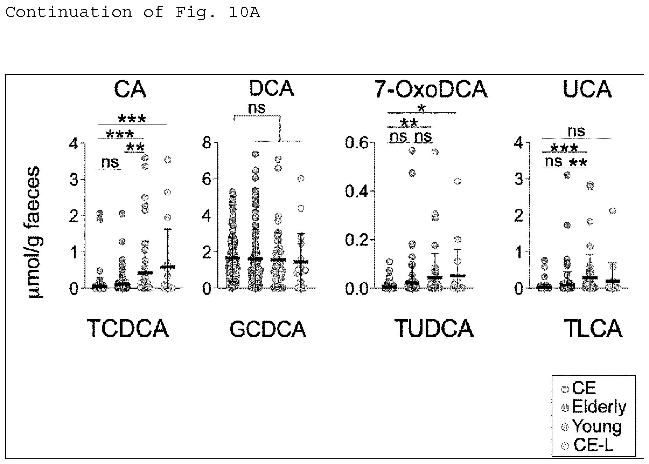

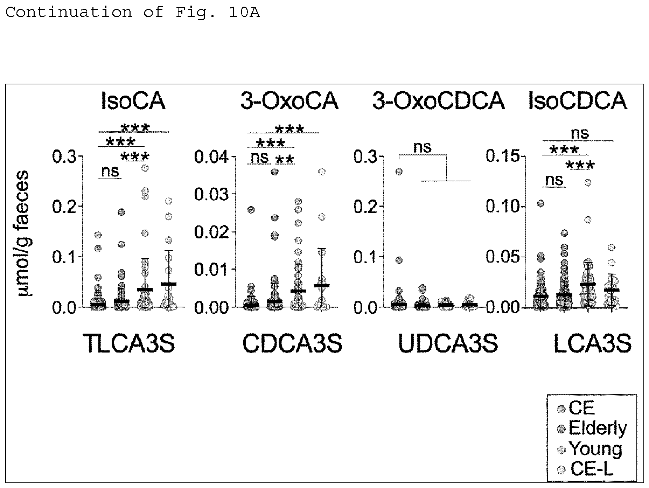

[Fig. 10A] Fig. 10A is a dot plot diagram showing the results of quantifying fecal bile acids in individual centenarians (n = 127), elderly (n = 108), young (n = 47), and lineal descendants of centenarians (n = 18) by LC-MS/MS.

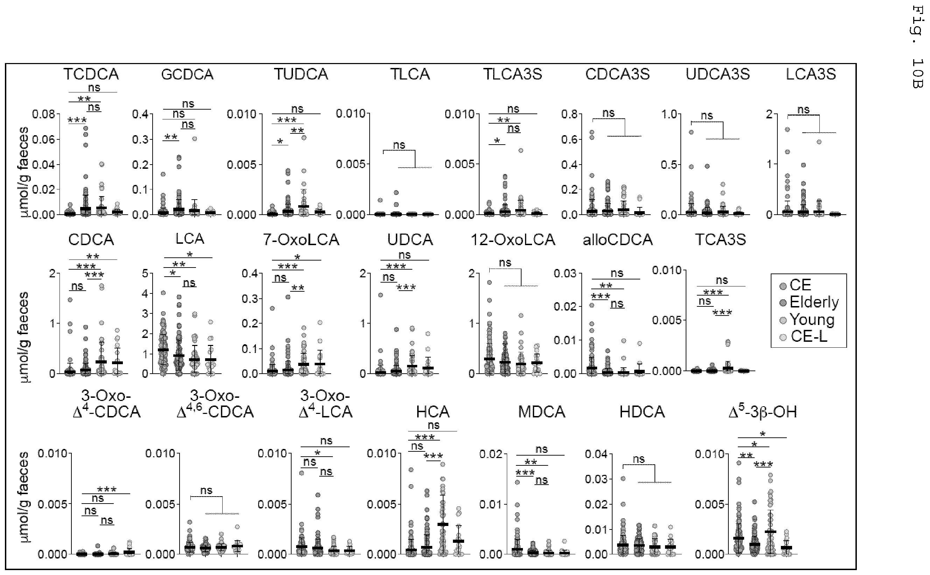

[Fig. 10B] Fig. 10B is a dot plot diagram showing the results of quantifying fecal bile acids in individual centenarians (n = 127), elderly (n = 108), young (n = 47), and lineal descendants of centenarians (n = 18) by LC-MS/MS. In Figs. 10A and 10B, the data is shown as the mean + standard deviation. *** P < 0.001; ** P < 0.01; * P < 0.05; one-way analysis of variance with Tukey's test. ns indicates that there is no significant difference. The total amount of fecal bile acids is expressed in µmol/g based on the wet weight of feces. nd indicates undetected.

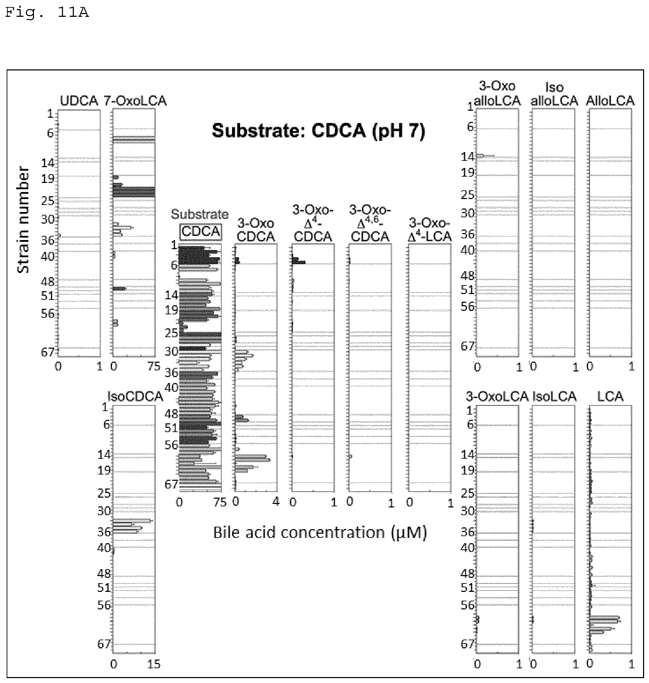

[Fig. 11A] Fig. 11A is a graph showing the results of analyzing bile acid metabolism using CDCA as a substrate at in vitro and pH 7 by 68 strains derived from CE91.

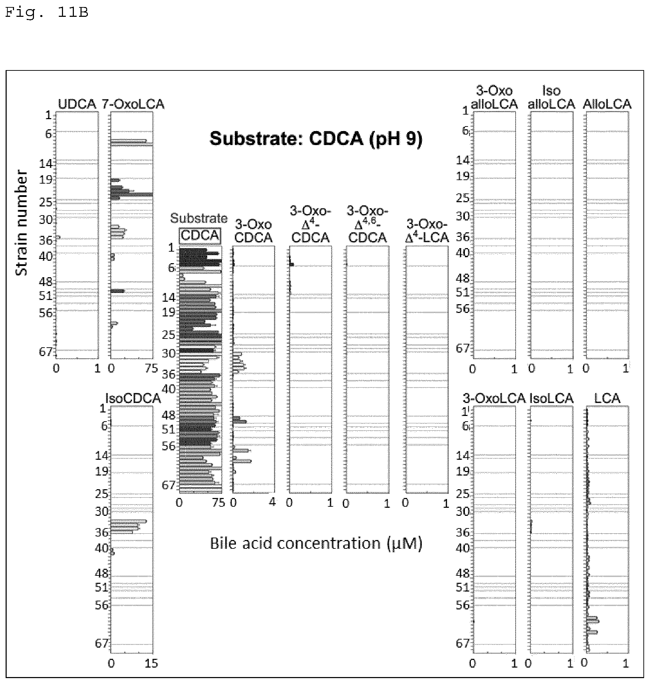

[Fig. 11B] Fig. 11B is a graph showing the results of analyzing bile acid metabolism using CDCA as a substrate at in vitro and pH 9 by 68 strains derived from CE91.

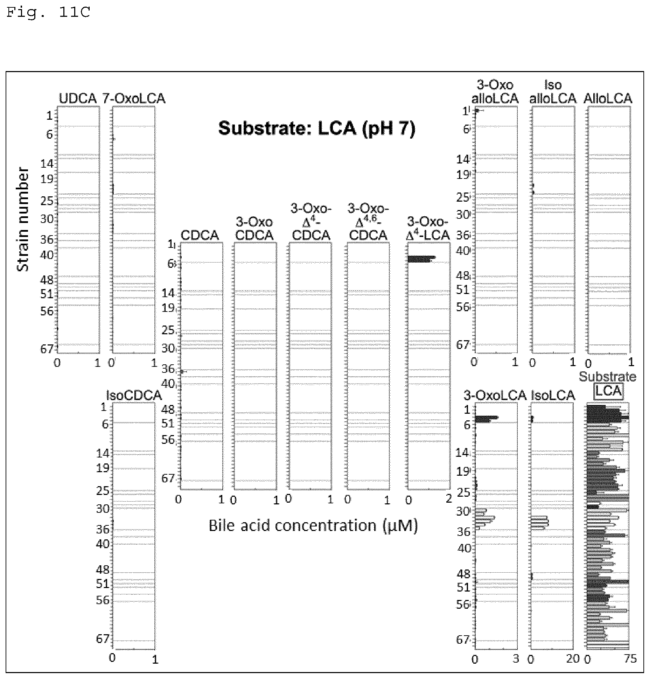

[Fig. 11C] Fig. 11C is a graph showing the results of analyzing bile acid metabolism using LCA as a substrate at in vitro and pH 7 by 68 strains derived from CE91.

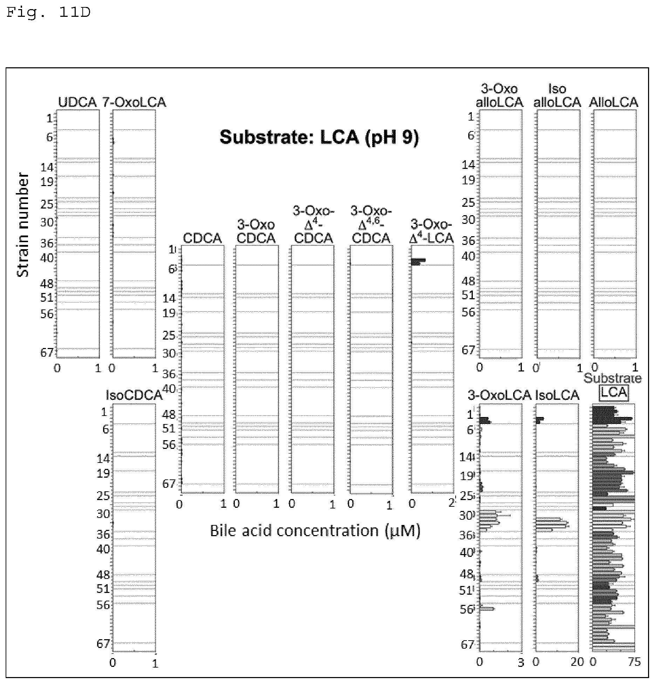

[Fig. 11D] Fig. 11D is a graph showing the results of analyzing bile acid metabolism using LCA as a substrate at in vitro and pH 9 by 68 strains derived from CE91.

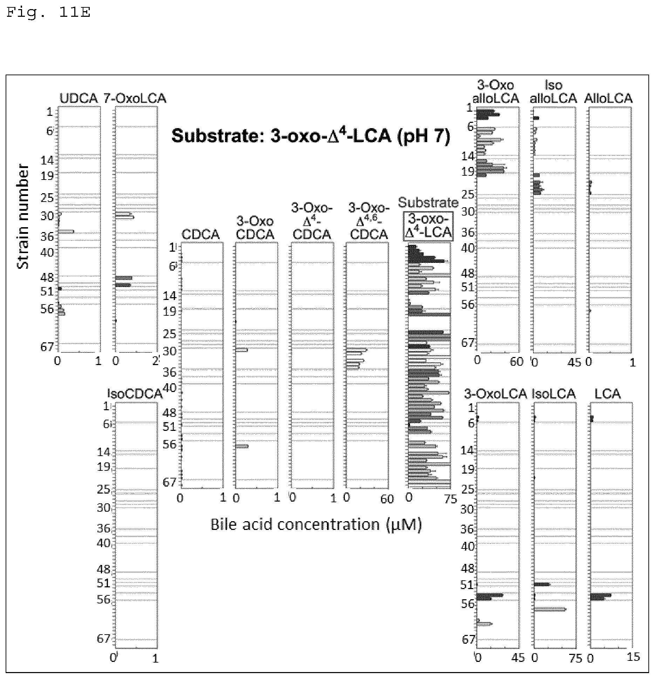

[Fig. 11E] Fig. 11E is a graph showing the results of analyzing bile acid metabolism using 3-oxo-Δ⁴-LCA as a substrate at in vitro and pH 7 by 68 strains derived from CE91.

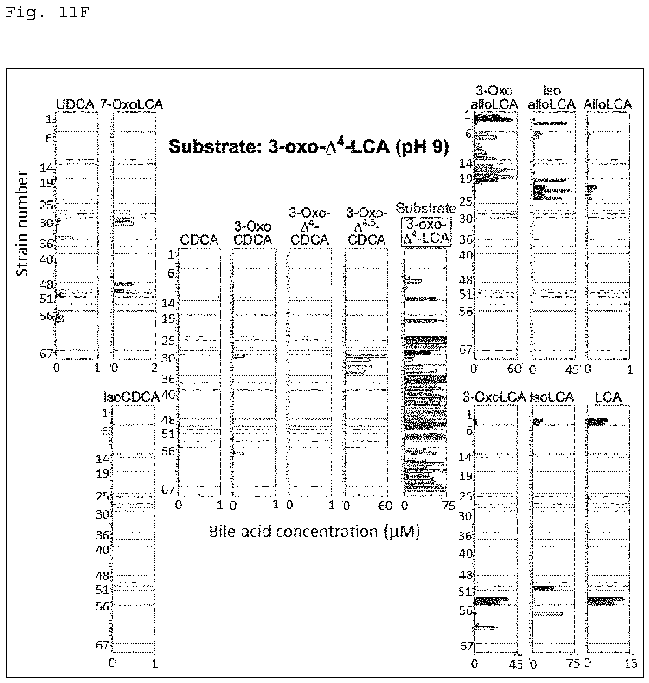

[Fig. 11F] Fig. 11F is a graph showing the results of analyzing bile acid metabolism using 3-oxo-Δ⁴-LCA as a substrate at in vitro and pH 9 by 68 strains derived from CE91. In Figs. 11A to 11F, the concentration of the substrate was 50 µM, and each analysis result was obtained by LC-MS/MS analysis of the supernatant of culture broth cultured for 48 hours. The data is shown as the mean + standard deviation of N = 2. Further, the "Strain number" in each figure and the color coding under color display correspond to Fig. 3A.

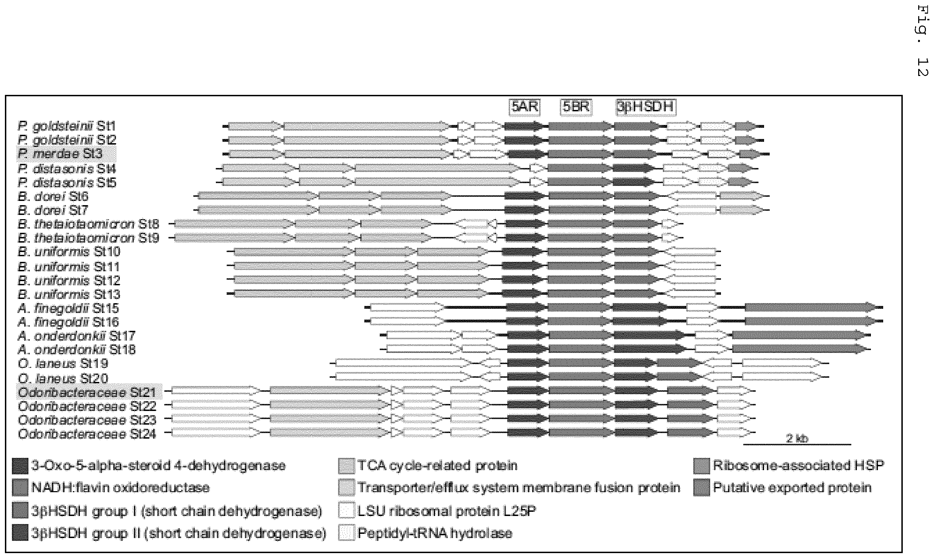

[Fig. 12] Fig. 12 is a diagram showing an overview of gene clusters encoding bile acid metabolizing enzymes of Bacteroidetes strains. The result is shown of analyzing the gene clusters of the Bacteroidetes strains isolated from the centenarian (CE91). Gene clusters containing homologs of 5AR (under color display, magenta), 5BR (under color display, blue), 3βHSDH group 1 (under color display, green), and 3βHSDH group 2 (under color display, purple) are fairly close to genes annotated as functions associated with the TCA cycle (under color display, beige) and/or as membrane fusion proteins of the transporter/efflux system (under color display, yellow). Gene homolog was defined as having <1e⁻¹² E value,> 30% sequence identity, and > 60% query coverage similarity. The arrows indicate coding sequences and are color coded according to the annotated function.

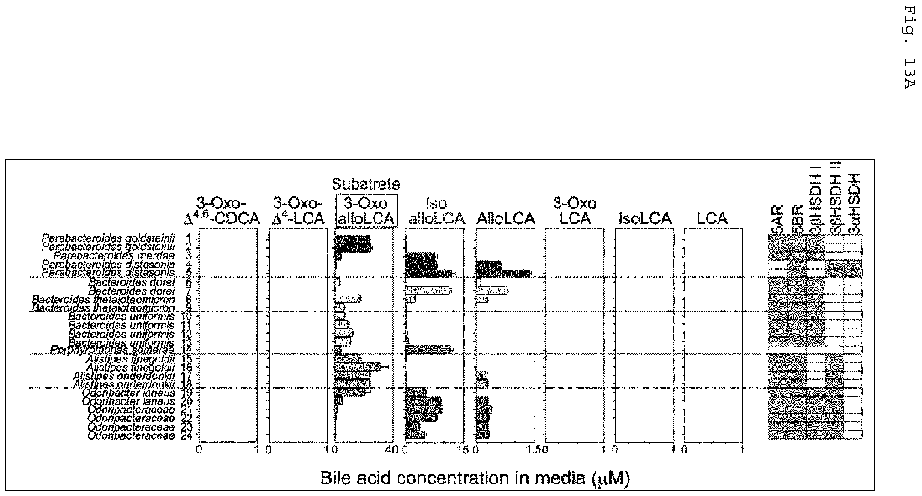

[Fig. 13A] Fig. 13A is a graph showing the results of culturing Bacteroidetes strains in a WCA medium adjusted to pH 9 by adding 3-oxoalloLCA (final concentration 50 *µM) as a substrate and analyzing the metabolism of LCA-related compounds.

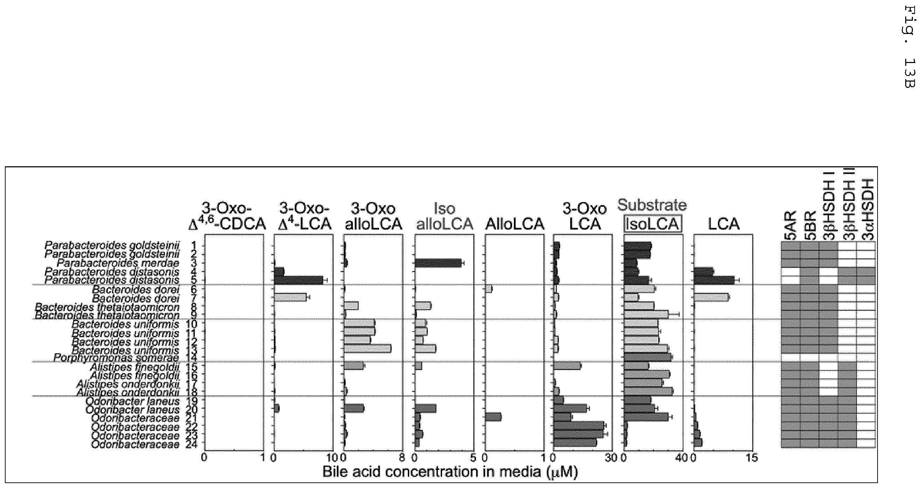

[Fig. 13B] Fig. 13B is a graph showing the results of culturing Bacteroidetes strains in a WCA medium adjusted to pH 9 by adding 3-oxoLCA (final concentration 50 µM) as a substrate and analyzing the metabolism of LCA-related compounds.

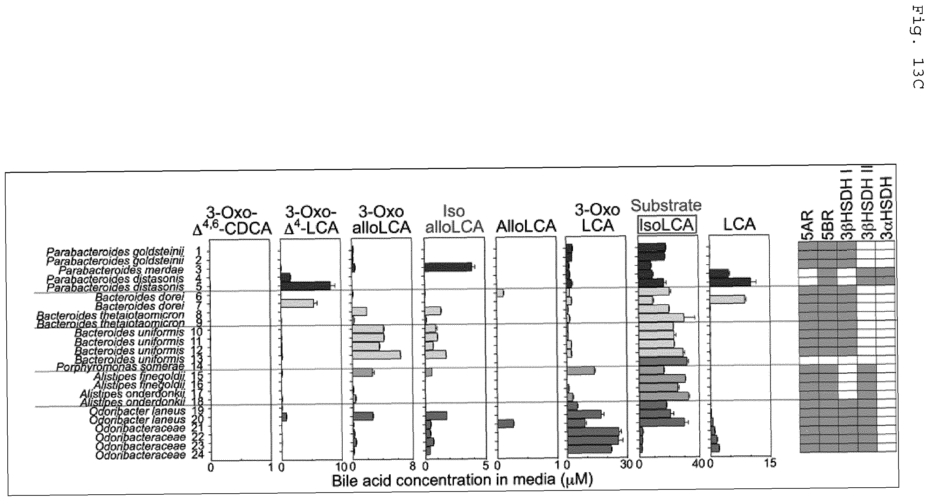

[Fig. 13C] Fig. 13C is a graph showing the results of culturing Bacteroidetes strains in a WCA medium adjusted to pH 9 by adding isoLCA (final concentration 50 µM) as a substrate and analyzing the metabolism of LCA-related compounds. In Figs. 13A to 13C, the culture supernatant was collected after anaerobic culture at 37°C for 48 hours for quantification by LC-MS/MS. The data is shown as the mean + standard deviation of N = 2.

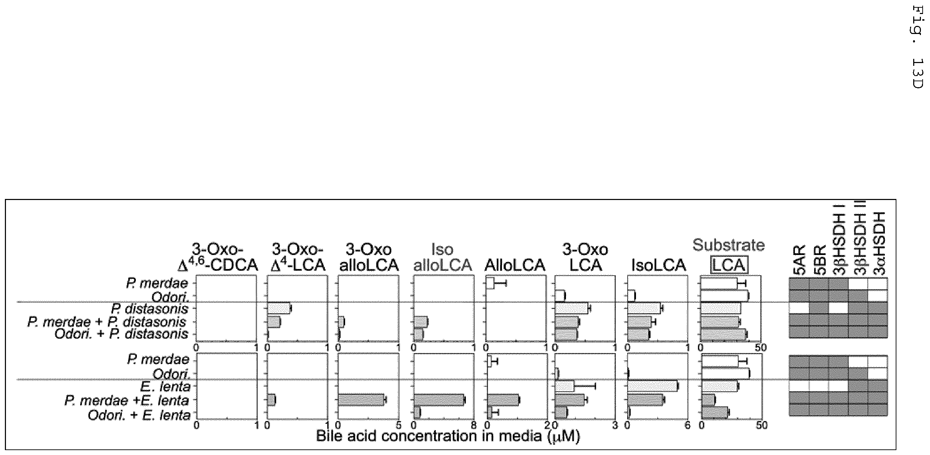

[Fig. 13D] Fig. 13D is a graph showing the results of co-culturing in a WCA medium adjusted to pH 9 by adding 50 µM LCA and analyzing the metabolism of bile acids for P. merdae St3 or Odoribacteraceae St21 and P. distasonis St4 (upper side in the figure) or E. lenta St34 (lower side in the figure). In Figs. 13A to 13D, the presence or absence of homologs for 5AR (under color display, red), 5BR (under color display, blue), 3βHSDH I1 (under color display, green), 3βHSDH2 (under color display, purple) and 3αHSDH (under color display, orange) in the corresponding strains is shown in the waffle chart on the right side of each figure.

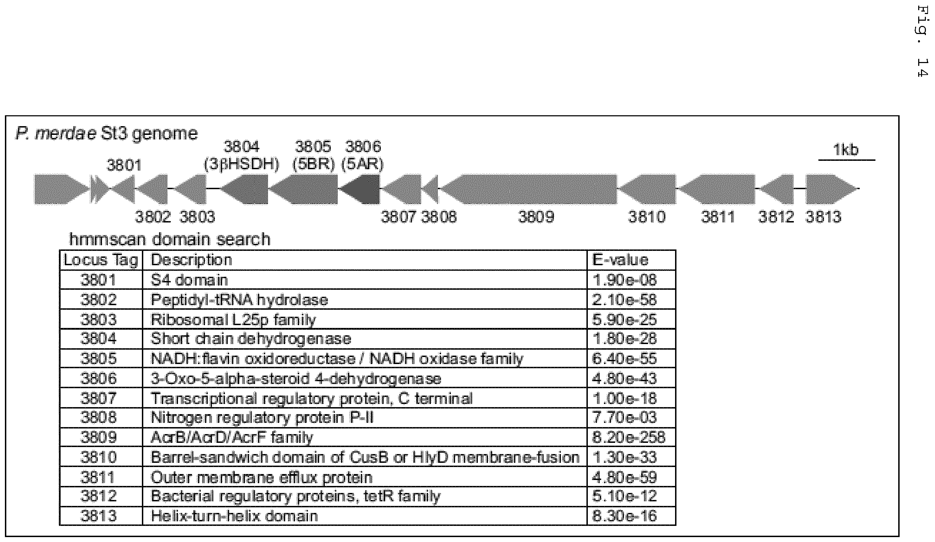

[Fig. 14A] Fig. 14A is a diagram showing an outline of gene organization around the 5AR (PM3806), 5BR (PM3805), and 3βHSDH (PM3804) genes of Parabacteroides merdae St3. Each protein sequence was annotated with HMMER v.3.1b2 hmmscan for Pfam-A HMM domain library.

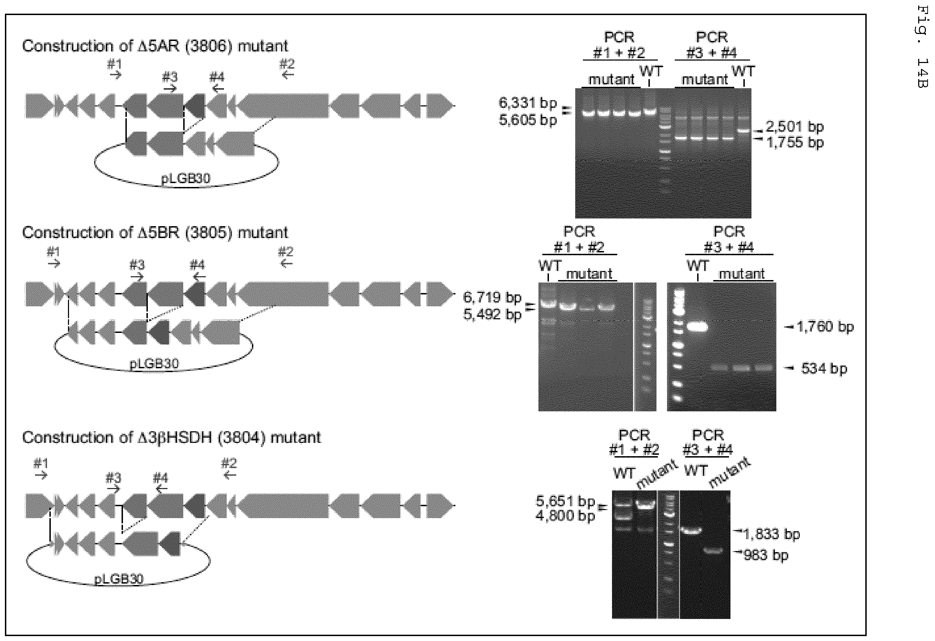

[Fig. 14B] Fig. 14B is a diagram showing an overview of deletion product preparation for 5AR, 5BR, or 3βHSDH. The plasmids for preparing deletion products were prepared by inserting the homologous sequences immediately upstream and downstream of the target gene into the pLGB30 plasmid. The fact that the deletion products had successfully been prepared was confirmed by PCR using the primers inside (#3-#4) and outside (#1-#2) at the homologous sequence site.

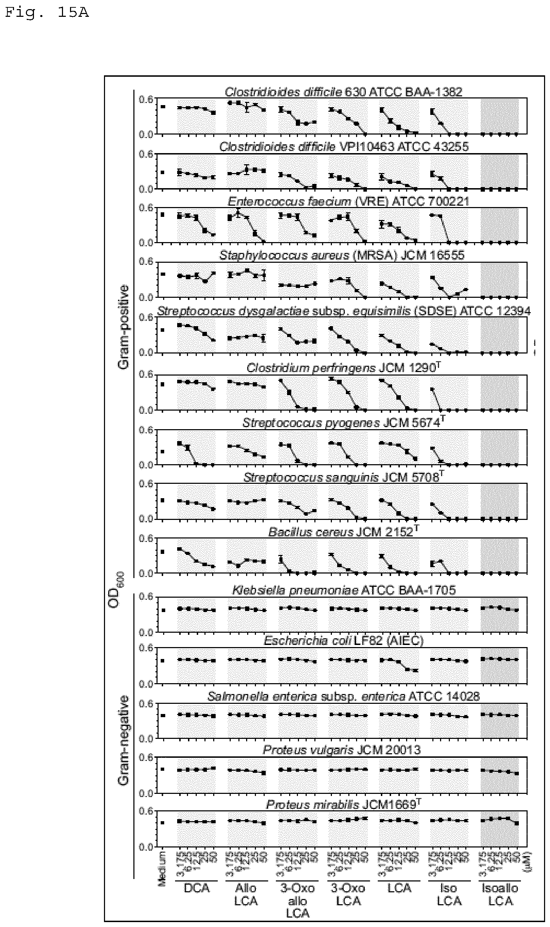

[Fig. 15A] Fig. 15A is a graph showing growth curves of pathogens when cultured with varying secondary bile acid concentrations. The growth was detected by measuring the absorbance (OD 600) of the medium cultured under anaerobic conditions at 37°C using WCA medium. The color-displayed, lightly painted red curves are the results of cultures containing isoalloLCA.

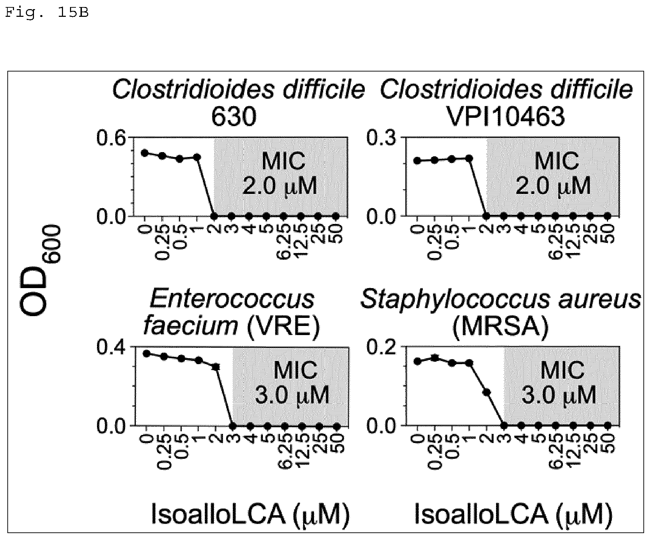

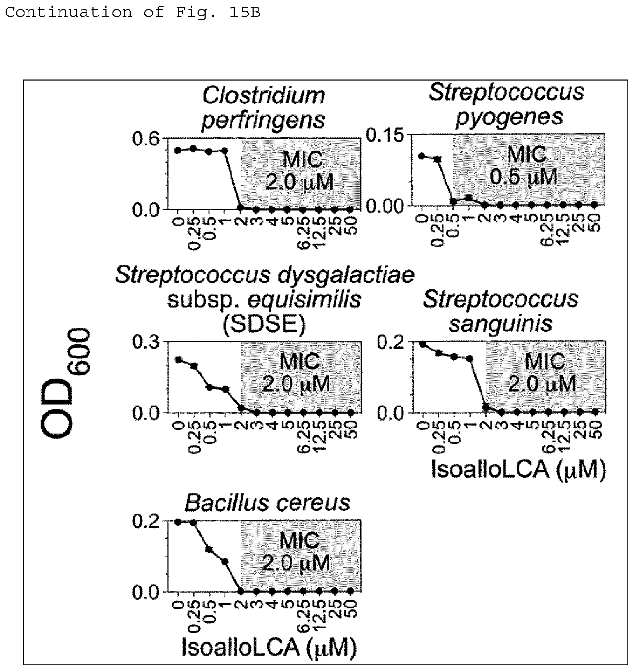

[Fig. 15B] Fig. 15B is a graph showing the effects of isoalloLCA on gram-positive pathogens.

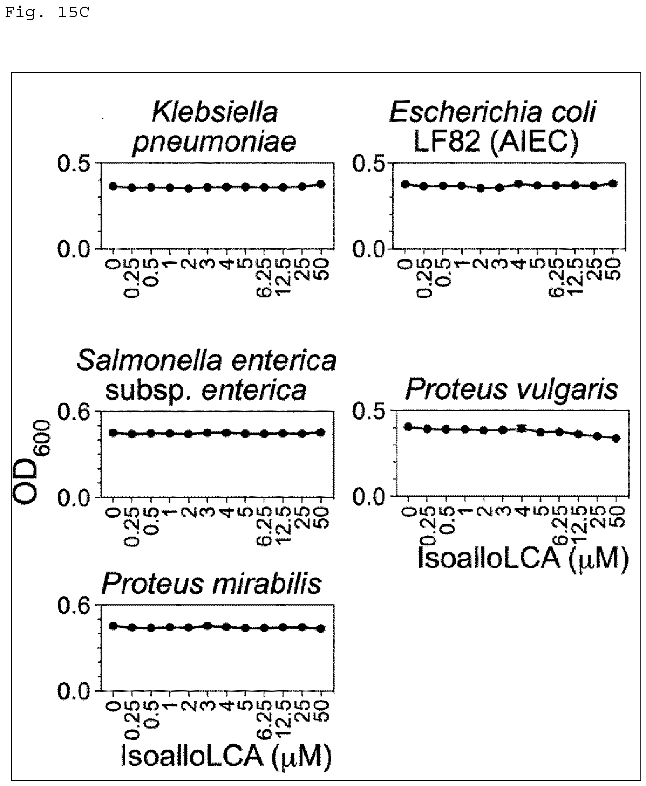

[Fig. 15C] Fig. 15C is a graph showing the effects of isoalloLCA on gram-negative pathogens. In Figs. 15B and 15C, the lightly painted red regions indicate the isoalloLCA concentration at which growth is inhibited, and MIC90 is shown within the area. The data is the mean ± standard deviation of N = 2.

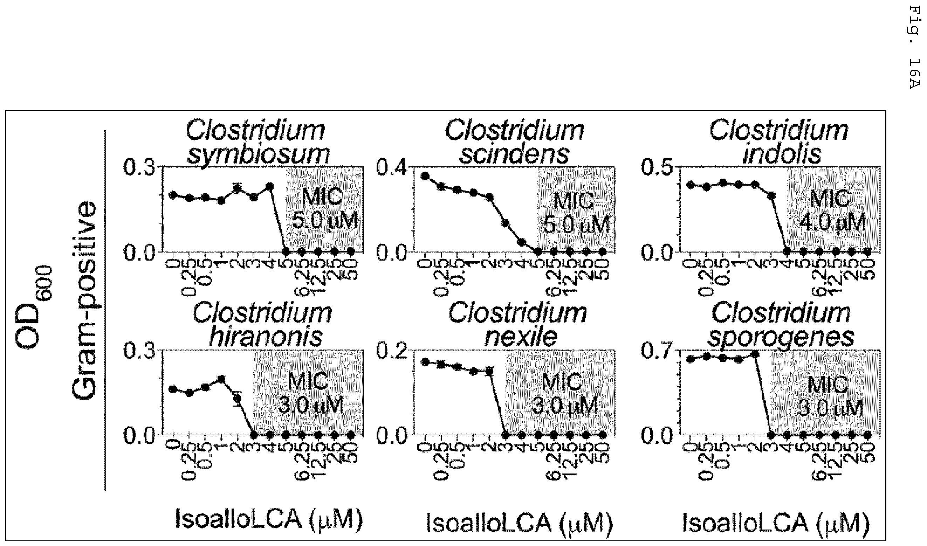

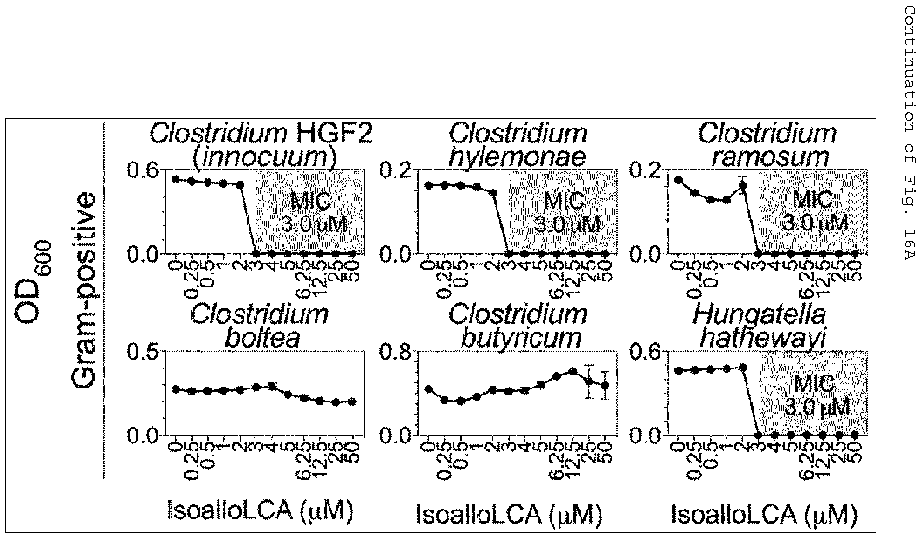

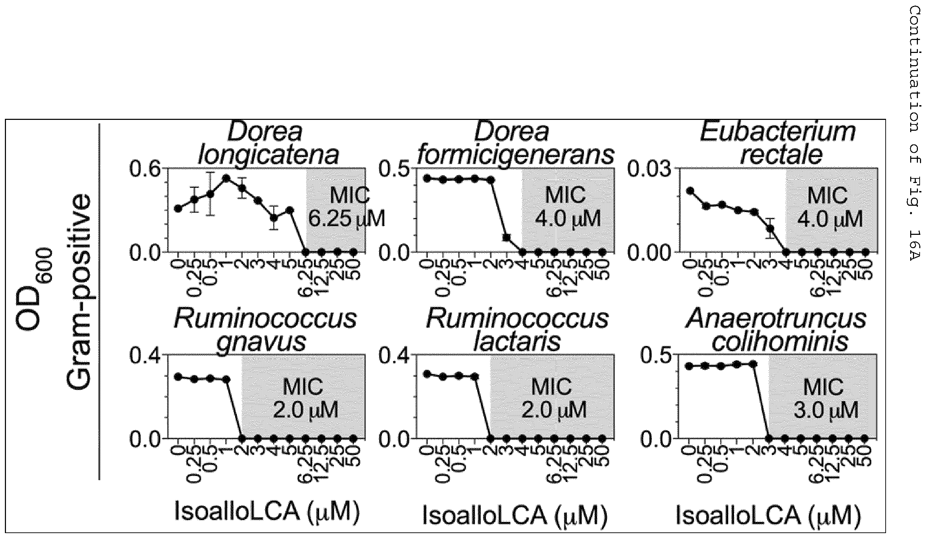

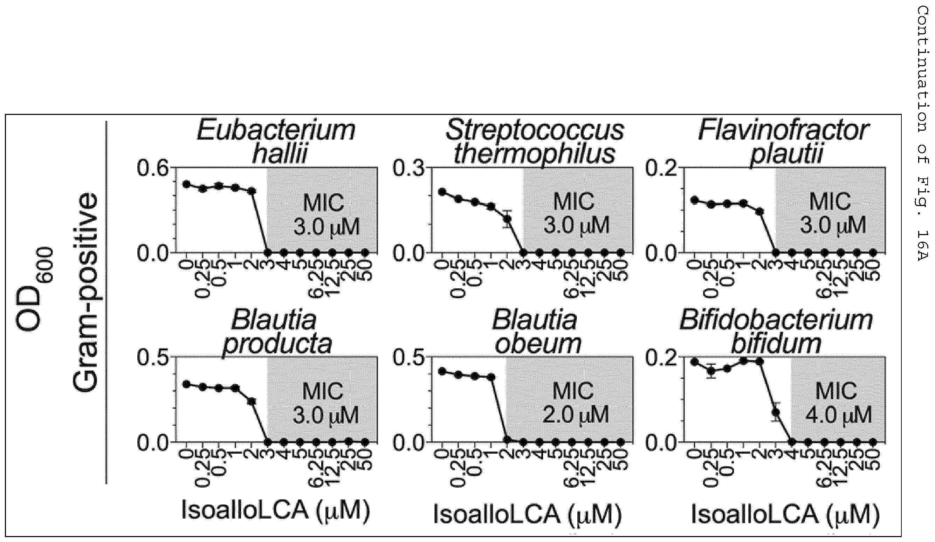

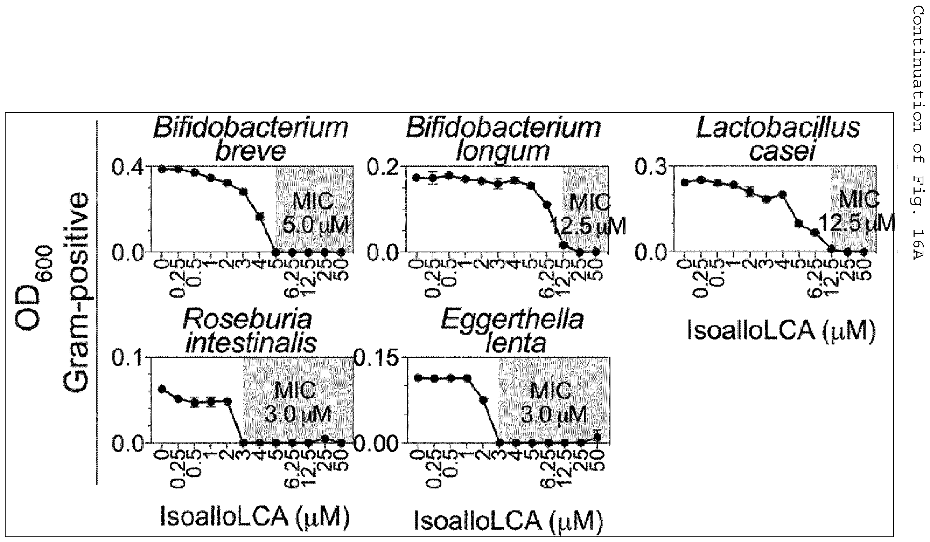

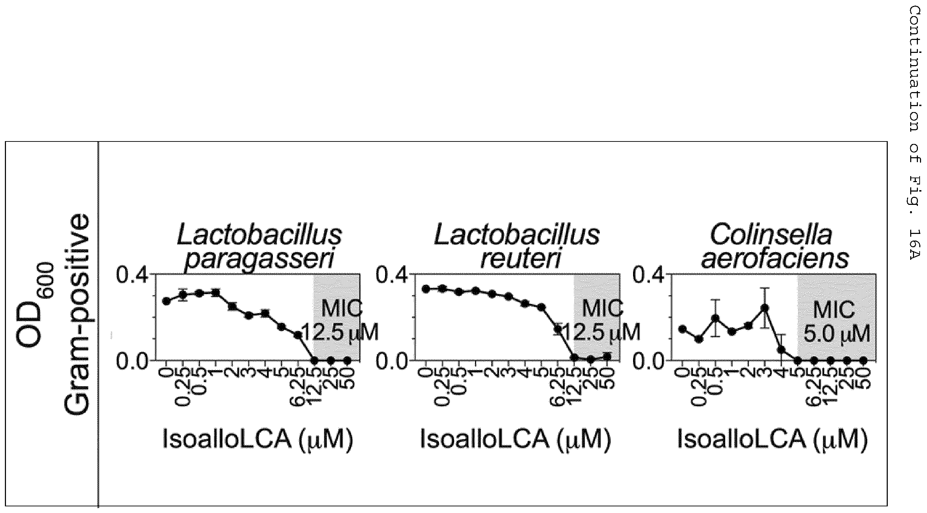

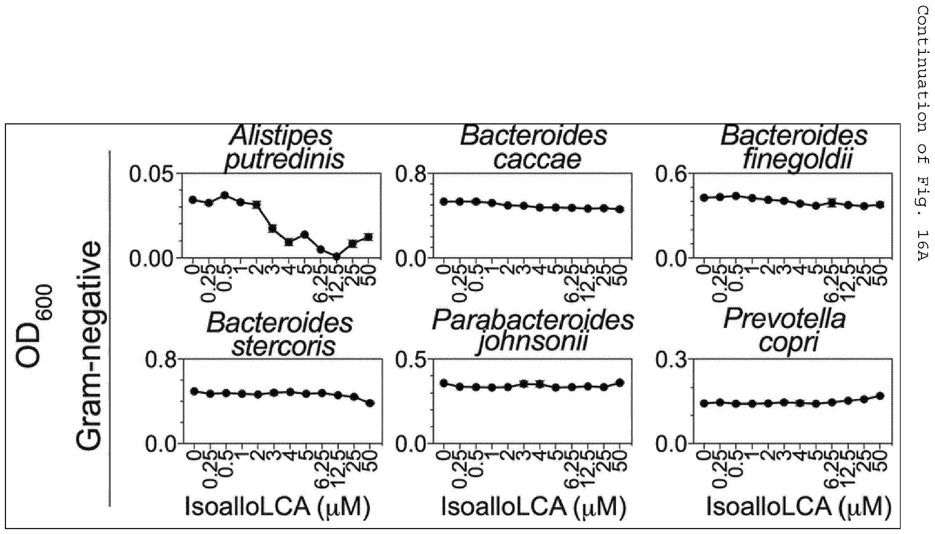

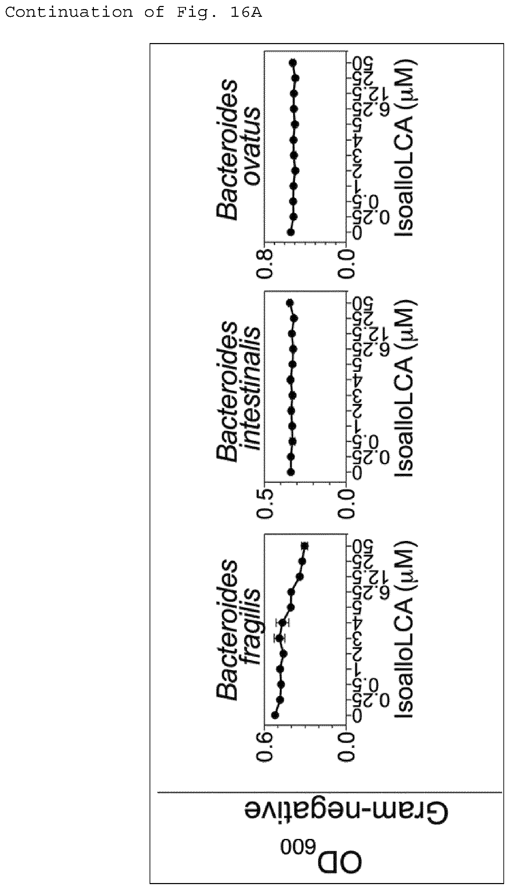

[Fig. 16A] Fig. 16A is a graph showing the growth inhibitory effects of isoalloLCA on intestinal symbiotic bacteria cultured in WCA medium. The growth of both gram-positive symbiotic bacteria and gram-negative symbiotic bacteria was detected by measuring the absorbance (OD 600) of the medium cultured under anaerobic conditions at 37°C. The data is shown as the mean and standard deviation of N = 2. The lightly painted red regions indicate the concentration of isoalloLCA having growth inhibitory effects.

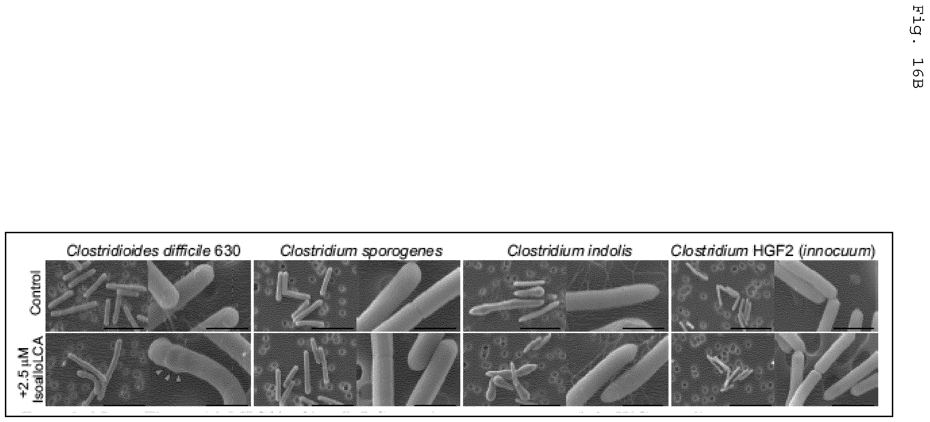

[Fig. 16B] Fig. 16B is a set of photographs showing typical results obtained by observing C. difficile 630, C. sporogenes, C. indoli, and C. HGF2 (innocuum) with a scanning electron microscope after culturing for 5 hours in a WCA medium containing control or 2.5 µM isoalloLCA. Each right panel shows a corresponding high resolution image. Scale bars indicate 5.0 µm and 1.0 µm. The arrowheads indicate a shape change after isoalloLCA treatment.

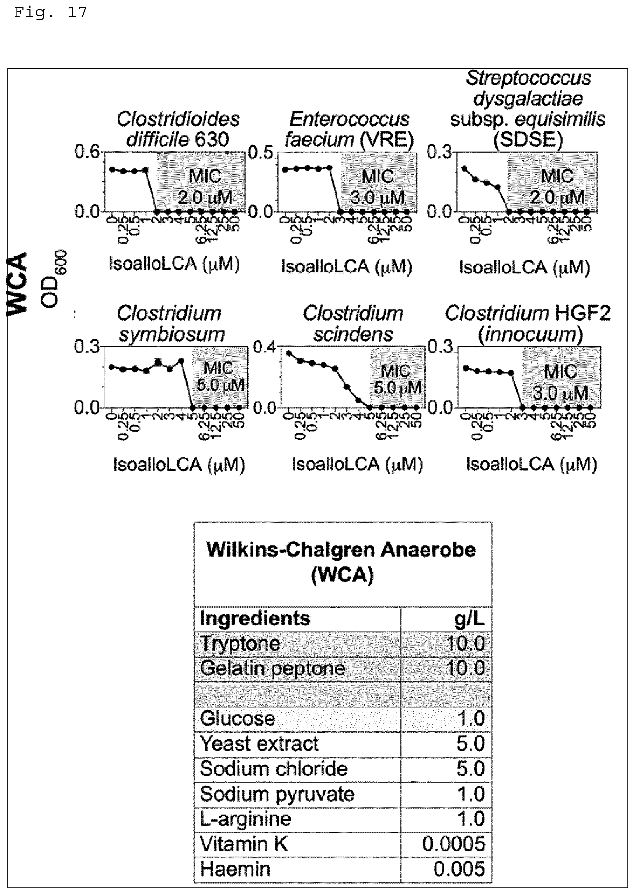

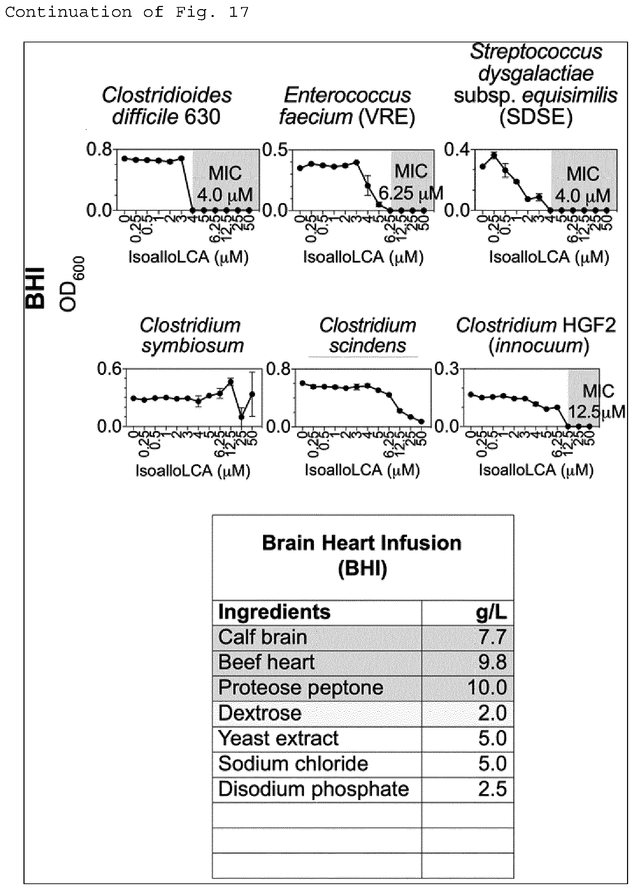

[Fig. 17] Fig. 17 is a graph showing growth curves obtained by culturing C. difficile 630, VRE, SDSE, C. symbiosum, C. scindens, or C. HGF2 (innocuum) in WCA medium and Brain Heart Infusion (BHI) medium containing different concentrations of isoalloLCA. The data is shown as the mean and standard deviation of N = 2. The lightly painted red regions indicate the concentration of isoalloLCA having growth inhibitory effects. In addition, the nutritional compositions of WCA and BHI medium are also shown at the bottom.

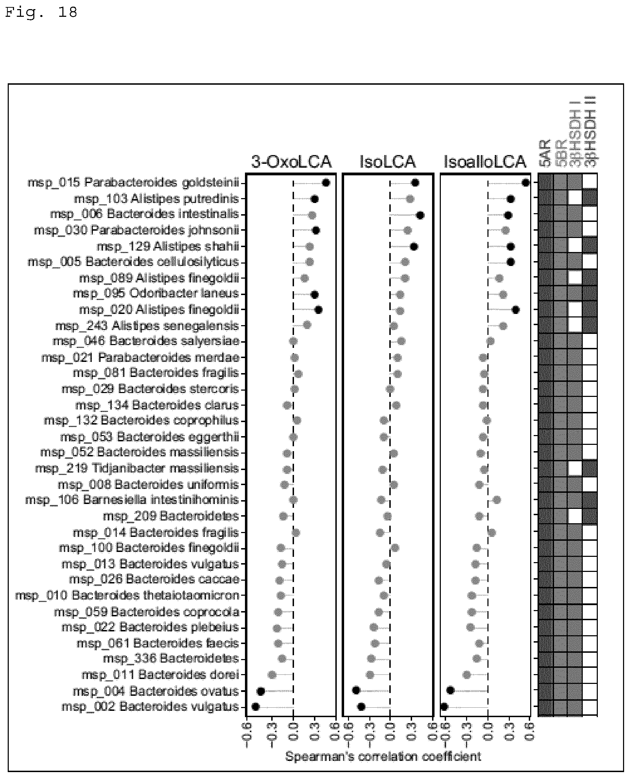

[Fig. 18] Fig. 18 is a diagram showing the correlation between the amount of 3-oxoLCA, the amount of isoLCA, or the amount of isoalloLCA and the relative amount of bacterial species having 5AR, 5BR, and 3βHSDHs. In the figure, the waffle chart on the right side shows the presence or absence of homologs for 5AR (under color display, red), 5BR (under color display, blue), 3βHSDH1 (under color display, green), and 3βHSDH2 (under color display, purple) of the gut microbiome species obtained from the assembled intestinal metagenomes of centenarians. The dot diagrams in the middle are a dot diagram showing the correlation between the amount of secondary bile acids in the feces of centenarians and the relative abundance of the corresponding species. The black dots indicate a significant Spearman's correlation at FDR P < 0.05.

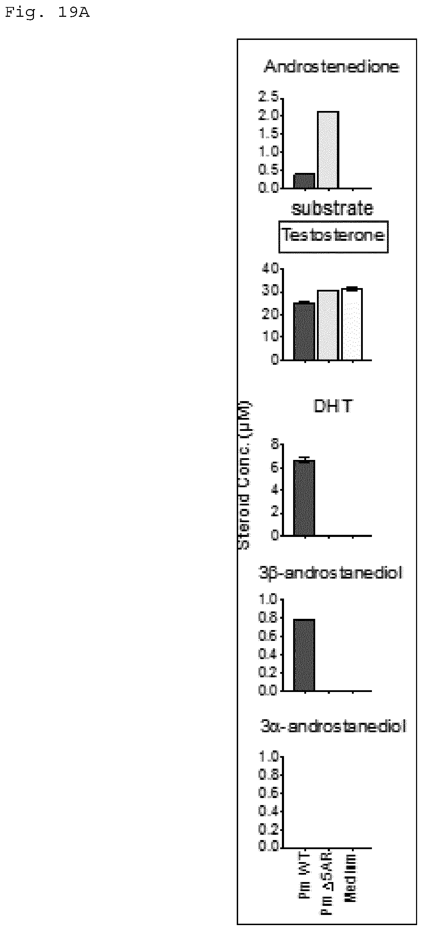

[Fig. 19A] Fig. 19A is a graph showing the results of analyzing testosterone metabolism in vitro by wild-type (WT) P. merdae St3 ("PmWT" in the figure) or 5AR-deficient-type (Δ5AR) P. merdae St3 ("PmΔ5AR" in the figure) . The strains were anaerobically cultured at 37°C for 48 hours in WCA medium adjusted to pH 7.

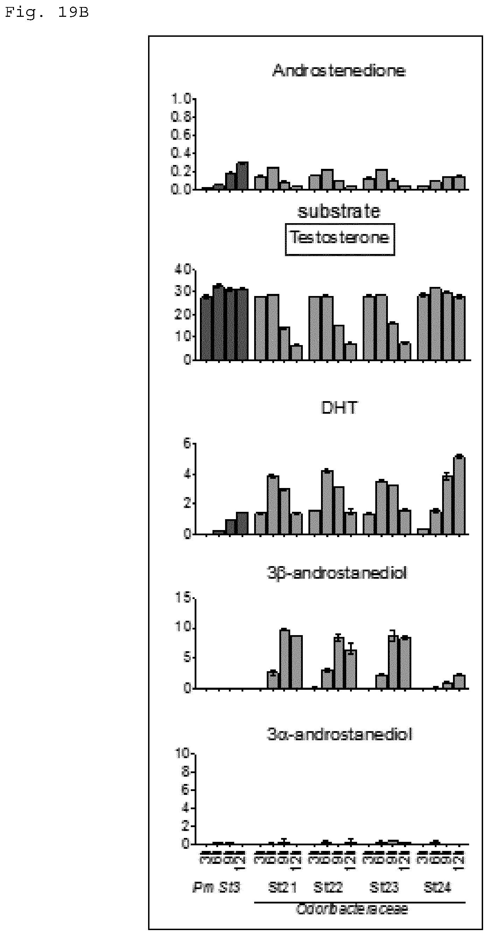

[Fig. 19B] Fig. 19B is a graph showing the results of analyzing testosterone metabolism by P. merdae St3 and Odoribacteraceae St21-24.

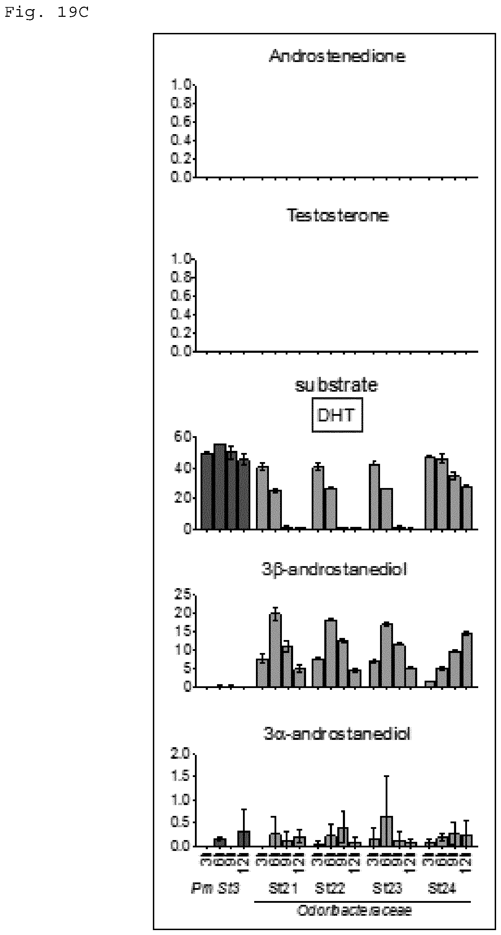

[Fig. 19C] Fig. 19C is a graph showing the results of analyzing metabolism of 5α-dihydrotestosterone(DHT) by P. merdae St3 and Odoribacteraceae St21-24. In Figs. 19B and 19C, the culture supernatant was collected after 3 hours, 6 hours, 9 hours and 12 hours. The concentration of each steroid hormone was determined by LC-MS/MS. The data is shown as the mean ± standard deviation of N = 2.

### [Description of Embodiments]

Hereinafter, the present invention will be described in detail according to preferred embodiments thereof. First, various enzymes according to the present invention having an activity of catalyzing a bile acid producing reaction involved in centenarians and the like will be described.

### <3-Oxo-5α-Steroid-4-Dehydrogenase (5AR)>

The 3-oxo-5α-steroid-4-dehydrogenase (3-oxo-5-alpha-steroid-4-dehydrogenase, 5AR) according to the present invention is also referred to as 5α-reductase and is an enzyme specified by EC1.3.99.5.

The 5AR according to the present invention is an enzyme having an activity of catalyzing a reaction of converting 3-oxo-Δ⁴-LCA to 3-oxoalloLCA (or a reaction of converting 3-oxoalloLCA to 3-oxo-Δ⁴-LCA) and/or a reaction of converting testosterone to 5α-dihydrotestosterone (DHT) (or a reaction of converting DHT to testosterone).

Further, the 5AR according to the present invention is preferably a protein containing an amino acid sequence having high homology with respect to the amino acid sequence set forth in any one of SEQ ID NOs: 69 to 89.

Further, the 5AR according to the present invention may have a natural or non-natural (artificial) mutation introduced into the amino acid sequence set forth in any one of SEQ ID NOs: 69 to 89. That is, the 5AR according to the present invention also includes a protein containing an amino acid sequence in which one or more amino acids are substituted, deleted, added, and/or inserted in the amino acid sequence of 5AR (such as an amino acid sequence set forth in SEQ ID NO: 69). Here, the term "more" is not particularly limited, but is preferably 2 to 150, more preferably 2 to 100, further preferably 2 to 70, more preferably 2 to 50, further preferably 2 to 30, more preferably 2 to 20, further preferably 2 to 10 (for example, 2 to 9, 2 to 8, 2 to 7, 2 to 6, 2 to 5, 2 to 4, 2 to 3, 2) .

Further, the 5AR according to the present invention may be a protein containing an amino acid sequence encoded by a DNA encoding the amino acid sequence set forth in any one of SEQ ID NOs: 69 to 89 and a DNA that hybridizes under stringent conditions.

### <3β-Hydroxysteroid Dehydrogenase (3βHSDH)>

The 3β-hydroxysteroid dehydrogenase (3βHSDH) according to the present invention is an enzyme specified by EC 1.1.1.145 and can be of two isotypes (3βHSDH1 and 3βHSDH2), but is preferably 3βHSDH2.

The 3βHSDH according to the present invention is an enzyme having an activity of catalyzing at least one reaction of a reaction of converting 3-oxoalloLCA to isoalloLCA (or a reaction of converting isoalloLCA to 3-oxoalloLCA), a reaction of converting 3-oxoLCA to isoLCA (or a reaction of converting isoLCA to 3-oxoLCA), and a reaction of converting DHT to 3β-androstanediol (or a reaction of converting 3β-androstanediol to DHT).

The 3βHSDH1 according to the present invention is preferably a protein containing an amino acid sequence having high homology with respect to the amino acid sequence set forth in any one of SEQ ID NOs: 113 to 129.

Further, the 3βHSDH2 according to the present invention is preferably a protein containing an amino acid sequence having high homology with respect to the amino acid sequence set forth in any one of SEQ ID NOs: 130 to 141.

The 3βHSDH1 according to the present invention may have a natural or non-natural (artificial) mutation introduced into the amino acid sequence set forth in any one of SEQ ID NOs: 113 to 129. That is, the 3βHSDH1 according to the present invention also includes a protein containing an amino acid sequence in which one or more amino acids are substituted, deleted, added, and/or inserted in the amino acid sequence of 3βHSDH1 (such as an amino acid sequence set forth in SEQ ID NO: 113). Here, the term "more" is not particularly limited, but is preferably 2 to 200, more preferably 2 to 180, further preferably 2 to 150, more preferably 2 to 100, further preferably 2 to 70, more preferably 2 to 50, further preferably 2 to 30, more preferably 2 to 20, further preferably 2 to 10 (for example, 2 to 9, 2 to 8, 2 to 7, 2 to 6, 2 to 5, 2 to 4, 2 to 3, 2).

The 3βHSDH2 according to the present invention may have a natural or non-natural (artificial) mutation introduced into the amino acid sequence set forth in any one of SEQ ID NOs: 130 to 141. That is, the 3βHSDH2 according to the present invention also includes a protein containing an amino acid sequence in which one or more amino acids are substituted, deleted, added, and/or inserted in the amino acid sequence of 3βHSDH2 (such as an amino acid sequence set forth in SEQ ID NO: 130). Here, the term "more" is not particularly limited, but is preferably 2 to 150, more preferably 2 to 100, further preferably 2 to 70, more preferably 2 to 50, further preferably 2 to 30, more preferably 2 to 20, further preferably 2 to 10 (for example, 2 to 9, 2 to 8, 2 to 7, 2 to 6, 2 to 5, 2 to 4, 2 to 3, 2).

The 3βHSDH1 according to the present invention may be a protein containing an amino acid sequence encoded by a DNA encoding the amino acid sequence set forth in any one of SEQ ID NOs: 113 to 129 and a DNA that hybridizes under stringent conditions.

Further, the 3βHSDH2 according to the present invention may be a protein containing an amino acid sequence encoded by a DNA encoding the amino acid sequence set forth in any one of SEQ ID NOs: 130 to 141 and a DNA that hybridizes under stringent conditions.

### <3-Oxo-5β-Steroid-4-Dehydrogenase (5BR)>

The 3-oxo-5β-steroid-4-dehydrogenase (5BR) according to the present invention is an enzyme specified by EC1.3.99.6.

The 5BR according to the present invention is an enzyme having an activity of catalyzing a reaction of converting 3-oxo-Δ⁴-LCA to 3-oxoLCA (or a reaction of converting 3-oxoLCA to 3-oxo-Δ⁴-LCA).

Further, the 5BR according to the present invention is preferably a protein containing an amino acid sequence having high homology with respect to the amino acid sequence set forth in any one of SEQ ID NOs: 90 to 112.

Further, the 5BR according to the present invention may have a natural or non-natural (artificial) mutation introduced into the amino acid sequence set forth in any one of SEQ ID NOs: 90 to 112. That is, the 5BR according to the present invention also includes a protein containing an amino acid sequence in which one or more amino acids are substituted, deleted, added, and/or inserted in the amino acid sequence of 5BR (such as an amino acid sequence set forth in SEQ ID NO: 90). Here, the term "more" is not particularly limited, but is preferably 2 to 250, more preferably 2 to 200, further preferably 2 to 150, more preferably 2 to 100, further preferably 2 to 70, more preferably 2 to 50, further preferably 2 to 30, more preferably 2 to 20, further preferably 2 to 10 (for example, 2 to 9, 2 to 8, 2 to 7, 2 to 6, 2 to 5, 2 to 4, 2 to 3, 2).

Further, the 5BR according to the present invention may be a protein containing an amino acid sequence encoded by a DNA encoding the amino acid sequence set forth in any one of SEQ ID NOs: 90 to 112 and a DNA that hybridizes under stringent conditions.

Note that in the present invention, "high homology" is 20% or more (for example, 25% or more, 30% or more, 35% or more, 40% or more, 45% or more, 50% or more, 55% or more), preferably 60% or more (for example, 65% or more, 70% or more, 75% or more, 85% or more), more preferably 80% or more (for example, 85% or more, 86% or more, 87% or more, 88% or more, 89% or more), further preferably 90% or more (for example, 91% or more, 92% or more, 93% or more, 94% or more, 95% or more, 96% or more, 97% or more, 98% or more, 99% or more), and particularly preferably 100%.

"Homology" can mean similarity or identity. Further, "homology" may mean, in particular, identity. Amino acid sequence homology can be determined using an alignment program such as BLAST (Basic Local Alignment Search Tool at the National Center for Biological Information). For example, the homology of amino acid sequences includes the homology between amino acid sequences calculated using blastp, and more specifically includes the homology between amino acid sequences calculated by using the default parameters of blastp (for example, using the initially set parameters).

Further, in the present invention, amino acid substitutions and the like are preferably conservative substitutions. "Conservative substitution" means substituting with another amino acid residue having a chemically similar side chain. Groups of amino acid residues having chemically similar amino acid side chains are well known in the art to which the present invention belongs. For example, in terms of acidic amino acids (aspartic acid and glutamic acid), basic amino acids (lysine, arginine, and histidine), and neutral amino acids, classification is possible by amino acids having a hydrocarbon chain (glycine, alanine, valine, leucine, isoleucine, and proline), amino acids having a hydroxy group (serine and threonine), amino acids containing sulfur (cysteine and methionine), amino acids having an amide group (asparagine and glutamine), amino acids having an imino group (proline), and amino acids having an aromatic group (phenylalanine, tyrosine, and tryptophan).

Further, in the present invention, the "stringent conditions" are, for example, hybridization conditions of about "1 XSSC, 0.1% SDS, 37°C", stricter conditions are hybridization conditions of about "0.5 XSSC, 0.1% SDS, 42°C", and further stricter conditions are hybridization conditions of about "0.2 XSSC, 0.1% SDS, 65°C".

Further, the sequence numbers that specify various sequences according to the present invention are shown in Tables 1 to 4 below. Furthermore, in Tables 1 to 4, the presence or absence of possession of various enzymes of the bacterial strains (68 species) peculiar to centenarians is also shown. The numbers in the items of 16S rDNA to 3βHSDH2 indicate the sequence numbers of the DNA sequences that specify 16S rDNA and the sequence numbers of the amino acid sequences that specify various enzymes. Further, in the items of 16S rDNA to 3αHSDH, each black circle indicates that the bacterial strain carries the corresponding enzyme, although the sequence information is not disclosed. Each hyphen indicates that the bacterial strain does not carry the corresponding enzyme.

**[Table 1]**

| Strain number | Strain name | 16SrDNA | 5AR | 5BR | 3βHSDH1 | 3βHSDH2 | 3αHSDH |
|---|---|---|---|---|---|---|---|
| 1 | Parabacteroides goldsteinii St1 | 1 | 69 | 90 | 113 | - | - |
| 2 | Parabacteroides goldsteinii St2 | 2 | 70 | 91 | 114 | - | - |
| 3 | Parabacteroides merdae St3 | 3 | 71 | 92 | 115 | - | - |
| 4 | Parabacteroides distasonis St4 | 4 | - | 93 | - | 130 | • |
| 5 | Para bacteroides distasonis St5 | 5 | - | 94 | - | 131 | • |
| 6 | Bacteroides dorei St6 | 6 | 72 | 95 | 116 | - | - |
| 7 | Bacteroides dorei St7 | 7 | 73 | 96 | 117 | - | - |
| 8 | Bacteroides thetaiotaomicron St8 | 8 | 74 | 97 | 118 | - | - |
| 9 | Bacteroides thetaiotaomicron St9 | 9 | 75 | 98 | 119 | - | - |
| 10 | Bacteroides uniformis St10 | 10 | 76 | 99 | 120 | - | - |
| 11 | Bacteroides uniformis St11 | 11 | 77 | 100 | 121 | - | - |
| 12 | Bacteroides uniformis St12 | 12 | 78 | 101 | 122 | - | - |
| 13 | Bacteroides uniformis St13 | 13 | 79 | 102 | 123 | - | - |
| 14 | Porphyromonas somerae St14 | 14 | - | - | - | - | - |
| 15 | Alistipes finegoldii St15 | 15 | 80 | 103 | - | 132 | - |
| 16 | Alistipes finegoldii St16 | 16 | 81 | 104 | - | 133 | - |
| 17 | Alistipes onderdonkii St17 | 17 | 82 | 105 | - | 134 | - |

**[Table 2]**

| Strain number | Strain name | 16SrDNA | 5AR | 5BR | 3βHSDH1 | 3βHSDH2 | 3αHSDH |
|---|---|---|---|---|---|---|---|
| 18 | Alistipes onderdonkii St18 | 18 | 83 | 106 | - | 135 | - |
| 19 | Odoribacter laneus St19 | 19 | 84 | 107 | 124 | 136 | - |
| 20 | Odoribacter laneus St20 | 20 | 85 | 108 | 125 | 137 | - |
| 21 | Odoribacteraceae St21 | 21 | 86 | 109 | 126 | 138 | - |
| 22 | Odoribacteraceae St22 | 22 | 87 | 110 | 127 | 139 | - |
| 23 | Odoribacteraceae St23 | 23 | 88 | 111 | 128 | 140 | - |
| 24 | Odoribacteraceae St24 | 24 | 89 | 112 | 129 | 141 | - |
| 25 | Campylobacter ureolyticus St25 | 25 | - | - | - | - | - |
| 26 | Akkermansia muciniphila St26 | 26 | - | - | - | - | - |
| 27 | Akkermansia muciniphila St27 | 27 | - | - | - | - | - |
| 28 | Pyramidobacter piscolens St28 | 28 | - | - | - | - | - |
| 29 | Corynebacterium striatum St29 | 29 | - | - | - | ● | - |
| 30 | Raoultibacter timonensis St30 | 30 | - | ● | - | - | ● |
| 31 | Raoultibacter timonensis St31 | 31 | - | ● | - | - | ● |
| 32 | Gordonibacter pamelaeae St32 | 32 | - | - | - | ● | ● |
| 33 | Eggerthella lenta St33 | 33 | - | - | - | ● | ● |
| 34 | Eggerthella lenta St34 | 34 | - | - | - | ● | ● |

**[Table 3]**

| Strain number | Strain name | 16SrDNA | 5AR | 5BR | 3βHSDH1 | 3βHSDH2 | 3αHSDH |
|---|---|---|---|---|---|---|---|
| 35 | Eggerthella lenta St35 | 35 | - | - | - | ● | ● |
| 36 | Christensenellaceae St36 | 36 | - | ● | - | - | - |
| 37 | Christensenellaceae St37 | 37 | - | ● | - | - | - |
| 38 | Desulfovibrionaceae St38 | 38 | - | - | - | - | - |
| 39 | Desulfovibrionaceae St39 | 39 | - | - | - | - | - |
| 40 | Ruminococcaceae St40 | 40 | - | ● | - | - | ● |
| 41 | Ruminococcaceae St41 | 41 | - | ● | - | - | ● |
| 42 | Ruminococcaceae St42 | 42 | - | - | - | ● | - |
| 43 | Ruminococcaceae St43 | 43 | - | - | - | ● | - |
| 44 | Ruminococcaceae St44 | 44 | - | - | - | - | - |
| 45 | Ruminococcaceae St45 | 45 | - | ● | - | ● | - |
| 46 | Clostridiales St46 | 46 | - | - | - | - | ● |
| 47 | Clostridiales St47 | 47 | - | - | - | - | ● |
| 48 | Emergencia timonensis St48 | 48 | - | ● | - | ● | ● |
| 49 | Emergencia timonensis St49 | 49 | - | ● | - | ● | ● |
| 50 | Clostridioides difficile St50 | 50 | - | - | - | - | - |
| 51 | Clostridium innocuum St51 | 51 | - | - | - | - | - |

**[Table 4]**

| Strain number | Strain name | 16SrDNA | 5AR | 5BR | 3βHSDH1 | 3βHSDH2 | 3αHSDH |
|---|---|---|---|---|---|---|---|
| 52 | Phascolarctobacteri um faecium St52 | 52 | - | - | - | - | - |
| 63 | Phascolarctobacteri um faecium St53 | 63 | - | - | - | - | - |
| 54 | Hungatella hathewayi St54 | 54 | - | - | - | - | ● |
| 55 | Hungatella hathewayi St55 | 55 | - | - | - | - | ● |
| 56 | Lachnospiraceae St56 | 56 | - | - | - | ● | ● |
| 57 | Lachnospiraceae St57 | 57 | - | - | - | ● | ● |
| 58 | Lachnospiraceae St58 | 58 | - | ● | - | ● | - |
| 59 | Clostridium scindens St59 | 59 | - | - | - | - | - |
| 60 | Clostridium scindens St60 | 60 | - | - | - | - | - |
| 61 | Lachnospiraceae St61 | 61 | - | ● | - | - | - |
| 62 | Lachnospiraceae St62 | 62 | - | ● | - | - | - |
| 63 | Clostridium hylemonae St63 | 63 | - | - | - | - | - |
| 64 | Faecalicatena contorta St64 | 64 | - | - | - | - | ● |
| 65 | Clostridium symbiosum St65 | 65 | - | - | - | - | ● |
| 66 | Clostridium symbiosum St66 | 66 | - | - | - | - | ● |
| 67 | Methanobrevibacter smithii St67 | 67 | - | - | - | - | - |
| 68 | Methanobrevibacter smithii St68 | 68 | - | - | - | - | - |

Another compound may be directly or indirectly added to "5AR", "3βHSDH", and "5BR" (hereinafter also collectively referred to as "enzyme according to the present invention") according to the present invention. The addition is not particularly limited, and may be an addition at the gene level or a chemical addition. Further, the site for addition is not particularly limited, and may be either the amino terminal or the carboxyl terminal of the enzyme according to the present invention, or both of them. Addition at the gene level is achieved by using a DNA encoding the enzyme according to the present invention to which the reading frame of a DNA encoding another protein is added. The "another protein" added in this way is not particularly limited. For the purpose of facilitating the purification of the enzyme according to the present invention, a purification tag protein such as polyhistidine (His-)tag protein, FLAG-tag protein (registered trademark, Sigma-Aldrich), or glutathione S-transferase (GST) is preferably used. Further, for the purpose of facilitating the detection of the enzyme according to the present invention, a detection tag protein such as a fluorescent protein including GFP or a chemiluminescent protein including luciferase is preferably used. The chemical addition may be a covalent bond or non-covalent bond. The "covalent bond" is not particularly limited, and examples thereof include an amide bond between an amino group and a carboxyl group, an alkylamine bond between an amino group and an alkyl halide group, a disulfide bond between thiols, and a thioether bond between a thiol group and a maleimide group or an alkyl halide group. Examples of the "non-covalent bond" include a biotin-avidin bond. Further, as the "another compound" chemically added in this way, for the purpose of facilitating the detection of the enzyme according to the present invention, a fluorescent dye such as Cy3 or Rhodamine is preferably used, for example.

The enzyme according to the present invention can be obtained by culturing bacteria or the like that produce each of these enzymes. For example, a method can be mentioned in which the cultured bacteria or the like are collected from the medium by filtration, centrifugation, or the like, and the collected bacteria or the like are treated by cell lysis, grinding treatment, pressure crushing, or the like, and further, proteins expressed in bacteria or the like are purified and concentrated by ultrafiltration, salting out, solvent precipitation such as sulfur precipitation, chromatography (such as gel chromatography, ion exchange chromatography, or affinity chromatography), or the like. When the above-mentioned purification tag protein is added to the enzyme according to the present invention, it can be purified and collected using a substrate to which the tag protein is adsorbed. Further, these purification and concentration methods may be carried out alone or in combination as appropriate and may be carried out in multiple steps.

Further, the enzyme according to the present invention is not limited to the above biological synthesis, and can also be produced by using the DNA or the like and the cell-free protein synthetic system according to the present invention described later. The cell-free protein synthetic system is not particularly limited, and examples thereof include wheat germ-derived, Escherichia coli-derived, rabbit reticulocyte-derived, and insect cell-derived synthetic systems. Further, those skilled in the art can chemically synthesize the enzyme according to the present invention using a commercially available peptide synthesizer or the like.

Further, the enzyme according to the present invention may be mixed with other components and used. The other components are not particularly limited, and examples thereof include sterile water, physiological saline, vegetable oils, surfactants, lipids, lysis aids, buffers, protease inhibitors, and preservatives.

### <Polynucleotide>

Next, the polynucleotide encoding the enzyme according to the present invention will be described. The polynucleotide according to the present invention may be a natural DNA or RNA, may be a DNA or RNA in which a mutation has been artificially introduced into a natural DNA or RNA, or may be a DNA or RNA composed of an artificially designed nucleotide sequence, as long as it encodes the enzyme according to the present invention described above. Further, the morphology is not particularly limited, and includes cDNA, genomic DNA, mRNA, chemically synthesized DNA, and chemically synthesized RNA. Preparation of these polynucleotides can be carried out by using conventional means for those skilled in the art. Genomic DNA can be prepared, for example, by extracting genomic DNA from various bacteria, preparing a genomic library (plasmids, phages, cosmids, BAC, PAC, and the like can be used as vectors), developing this, and carrying out colony hybridization or plaque hybridization using a probe prepared based on the nucleotide sequence of the gene encoding the enzyme according to the present invention. The preparation is also possible by preparing a primer specific to the gene encoding the enzyme according to the present invention and perform PCR using the primer. Further, cDNA can be prepared, for example, synthesizing cDNA based on mRNA extracted from various bacteria, inserting it into a vector such as λZAP to prepare a cDNA library, developing it, and performing colony hybridization or plaque hybridization in the same manner as above, or by performing PCR.

Then, those skilled in the art can, if necessary, introduce a mutation encoding the above-mentioned amino acid substitution into the DNA thus prepared by using a known site-directed mutagenesis. Examples of site-directed mutagenesis include the Kunkel method (Kunkel, T. A., Proc Natl Acad Sci USA, 1985, Vol. 82, issue 2, pp. 488 to 492), SOE (splicing-by-overlap-extension)-PCR method (Ho, S. N., Hunt, H.D., Horton, R. M., Pullen, J. K., and Pease, L. R., Gene, 1989, Vol. 77, pp. 51 to 59).

Further, those skilled in the art can also artificially design a nucleotide sequence encoding a protein into which the above-mentioned amino acid substitution has been introduced, and chemically synthesize the polynucleotide according to the present invention using an automatic nucleic acid synthesizer based on the sequence information.

Further, from the viewpoint of further improving the expression efficiency of the encoding enzyme according to the present invention in the host cells, the polynucleotide according to the present invention may also take the form of DNA encoding the enzyme according to the present invention in which codons are optimized according to the type of the host cells.

The present invention may also take the form of a vector into which the DNA is inserted so that the above-mentioned polynucleotide can be replicated in the host cells.

In the present invention, the "vector" can be constructed based on a self-replicating vector, that is, an extrachromosomal independent entity whose replication does not depend on chromosomal replication, for example, a plasmid. The vector may also be one that, when introduced into a host cell, integrates into the genome of the host cell and replicates with the chromosome into which it has been integrated.

Examples of such a vector include a plasmid and phage DNA. Further, examples of the plasmid include Escherichia coli-derived plasmids (such as pET22, pBR322, pBR325, pUC118, pUC119, pUC18, and pUC19), yeast-derived plasmids (such as YEp13, YEp24, and YCp50), and Bacillus subtilis-derived plasmids (such as pUB110 and pTP5). Examples of the phage DNA include λ phages (such as Charon4A, Charon21A, EMBL3, EMBL4, λgt10, λgt11, and λZAP). Further, as a vector according to the present invention, if the host cell is insect-derived, an insect virus vector such as baculovirus can be used, if it is plant-derived, T-DNA or the like can be used, and if it is animal-derived, an animal virus vector such as a retrovirus or an adenovirus vector can be used. Further, as the procedure and method for constructing the vector according to the present invention, those commonly used in the field of genetic engineering can be used. For example, in order to insert the DNA according to the present invention into a vector, a method is employed in which a purified DNA first is cleaved with an appropriate restriction enzyme, inserted into a restriction enzyme site or multiple cloning site of an appropriate vector, and ligated to the vector.

Further, the vector according to the present invention may be in the form of an expression vector containing the enzyme according to the present invention encoded by the DNA in a state capable of being expressed in a host cell. In order to be introduced into a host cell to express the enzyme according to the present invention, the "expression vector" according to the present invention desirably contains, in addition to the above-mentioned DNA, a DNA sequence that controls its expression, a gene marker for selecting a transformed host cell, and the like. The DNA sequence that controls expression includes a promoter, an enhancer, a splicing signal, a poly A addition signal, a ribosome binding sequence (SD sequence), a terminator, and the like. The promoter is not particularly limited as long as it exhibits transcriptional activity in the host cell, and can be obtained as a DNA sequence that controls the expression of a gene encoding a protein homologous or heterologous to the host cell. In addition to the DNA sequence that controls the expression, a DNA sequence that induces expression may be included. When the host cell is a bacterium, examples of the DNA sequence that induces such expression include a lactose operon that can induce the expression of a gene located downstream by adding isopropyl-β-D-thiogalactopyranoside (IPTG). The gene marker in the present invention may be appropriately selected depending on the method for selecting the transformed host cell, and for example, a gene encoding drug resistance or a gene complementing auxotrophy can be used.

A DNA encoding the enzyme according to the present invention may be inserted into a vector one by one, or multiple types of DNAs may be inserted into one vector. In the case of inserting multiple types of DNAs into a vector, it is preferable that these DNAs form an operon when the multiple types of DNA are inserted into one vector. Here, the "operon" is a nucleic acid sequence unit composed of one or more genes transcribed under the control of the same promoter.

### <Bacteria>

Next, the above-mentioned bacterium having the polynucleotide according to the present invention will be described.

The "bacterium having a polynucleotide encoding 5AR" according to the present invention is not particularly limited as long as it is a bacterium having a polynucleotide encoding 5AR according to the present invention described above, and examples thereof include Parabacteroides goldsteinii, Parabacteroides merdae, Bacteroides dorei, Bacteroides thetaiotaomicron, Bacteroides uniformis, Alistipes finegoldii, Alistipes onderdonkii, Odoribacter laneus, and Odoribacteraceae which have the polynucleotide.

### More specific examples include

Parabacteroides goldsteinii having a polynucleotide having a very high identity to the nucleotide sequence set forth in SEQ ID NO: 1 or 2,
Parabacteroides merdae having a polynucleotide having a very high identity to the nucleotide sequence set forth in SEQ ID NO: 3,
Bacteroides dorei having a polynucleotide having a very high identity to the nucleotide sequence set forth in SEQ ID NO: 6 or 7,
Bacteroides thetaiotaomicron having a polynucleotide having a very high identity to the nucleotide sequence set forth in SEQ ID NO: 8 or 9,
Bacteroides uniformis having a polynucleotide having a very high identity to the nucleotide sequence set forth in any one of SEQ ID NOs: 10 to 13,
Alistipes finegoldii having a polynucleotide having a very high identity to the nucleotide sequence set forth in SEQ ID NO: 15 or 16,
Alistipes onderdonkii having a polynucleotide having a very high identity to the nucleotide sequence set forth in SEQ ID NO: 17 or 18,
Odoribacter laneus having a polynucleotide having a very high identity to the nucleotide sequence set forth in SEQ ID NO: 19 or 20, or
Odoribacteraceae having a polynucleotide having a very high identity to the nucleotide sequence set forth in any one of SEQ ID NOs: 21 to 24.

The "bacterium having a polynucleotide encoding 3βHSDH" according to the present invention is not particularly limited as long as it is a bacterium having a polynucleotide encoding 3βHSDH according to the present invention described above, and examples thereof include Parabacteroides goldsteinii, Parabacteroides merdae, Bacteroides dorei, Bacteroides thetaiotaomicron, Bacteroides uniformis, Odoribacter laneus, Odoribacteraceae, Parabacteroides distasonis, Alistipes finegoldii, Alistipes onderdonkii, Corynebacterium striatum, Gordonibacter pamelaeae, Eggerthella lenta, Ruminococcaceae, Emergencia timonensis, and Lachnospiraceae which have the polynucleotide.

### More specific examples include

Parabacteroides goldsteinii having a polynucleotide having a very high identity to the nucleotide sequence set forth in SEQ ID NO: 1 or 2,
Parabacteroides merdae having a polynucleotide having a very high identity to the nucleotide sequence set forth in SEQ ID NO: 3,
Bacteroides dorei having a polynucleotide having a very high identity to the nucleotide sequence set forth in SEQ ID NO: 6 or 7,
Bacteroides thetaiotaomicron having a polynucleotide having a very high identity to the nucleotide sequence set forth in SEQ ID NO: 8 or 9,
Bacteroides uniformis having a polynucleotide having a very high identity to the nucleotide sequence set forth in any one of SEQ ID NOs: 10 to 13,
Odoribacter laneus having a polynucleotide having a very high identity to the nucleotide sequence set forth in SEQ ID NO: 19 or 20,
Odoribacteraceae having a polynucleotide having a very high identity to the nucleotide sequence set forth in any one of SEQ ID NOs: 21 to 24,
Parabacteroides distasonis having a polynucleotide having a very high identity to the nucleotide sequence set forth in SEQ ID NO: 4 or 5,
Alistipes finegoldii having a polynucleotide having a very high identity to the nucleotide sequence set forth in SEQ ID NO: 15 or 16,
Alistipes onderdonkii having a polynucleotide having a very high identity to the nucleotide sequence set forth in SEQ ID NO: 17 or 18,
Corynebacterium striatum having a polynucleotide having a very high identity to the nucleotide sequence set forth in SEQ ID NO: 29,
Gordonibacter pamelaeae having a polynucleotide having a very high identity to the nucleotide sequence set forth in SEQ ID NO: 32,
Eggerthella lenta having a polynucleotide having a very high identity to the nucleotide sequence set forth in any one of SEQ ID NOs: 33 to 35,
Ruminococcaceae having a polynucleotide having a very high identity to the nucleotide sequence set forth in any one of SEQ ID NOs: 42, 43, and 45,
Emergencia timonensis having a polynucleotide having a very high identity to the nucleotide sequence set forth in SEQ ID NO: 48 or 49, or
Lachnospiraceae having a polynucleotide having a very high identity to the nucleotide sequence set forth in any one of SEQ ID NOs: 56 to 58.

The "bacterium having a polynucleotide encoding 5AR and a polynucleotide encoding 3βHSDH" according to the present invention is not particularly limited as long as it is a bacterium having a polynucleotide encoding 5AR and a polynucleotide encoding 3βHSDH according to the present invention described above, and examples thereof include Parabacteroides goldsteinii, Parabacteroides merdae, Bacteroides dorei, Bacteroides thetaiotaomicron, Bacteroides uniformis, Alistipes finegoldii, Alistipes onderdonkii, Odoribacter laneus, and Odoribacteraceae which have these polynucleotides.

### More specific examples include

Parabacteroides goldsteinii having a polynucleotide having a very high identity to the nucleotide sequence set forth in SEQ ID NO: 1 or 2,
Parabacteroides merdae having a polynucleotide having a very high identity to the nucleotide sequence set forth in SEQ ID NO: 3,
Bacteroides dorei having a polynucleotide having a very high identity to the nucleotide sequence set forth in SEQ ID NO: 6 or 7,
Bacteroides thetaiotaomicron having a polynucleotide having a very high identity to the nucleotide sequence set forth in SEQ ID NO: 8 or 9,
Bacteroides uniformis having a polynucleotide having a very high identity to the nucleotide sequence set forth in any one of SEQ ID NOs: 10 to 13,
Alistipes finegoldii having a polynucleotide having a very high identity to the nucleotide sequence set forth in SEQ ID NO: 15 or 16,
Alistipes onderdonkii having a polynucleotide having a very high identity to the nucleotide sequence set forth in SEQ ID NO: 17 or 18,
Odoribacter laneus having a polynucleotide having a very high identity to the nucleotide sequence set forth in SEQ ID NO: 19 or 20, or
Odoribacteraceae having a polynucleotide having a very high identity to the nucleotide sequence set forth in any one of SEQ ID NOs: 21 to 24.

The "bacterium having a polynucleotide encoding 5BR" according to the present invention is not particularly limited as long as it has a polynucleotide encoding 5BR according to the present invention described above, and examples thereof include Parabacteroides goldsteinii, Parabacteroides merdae, Parabacteroides distasonis, Bacteroides dorei, Bacteroides thetaiotaomicron, Bacteroides uniformis, Alistipes finegoldii, Alistipes onderdonkii, Odoribacter laneus, Odoribacteraceae, Raoultibacter timonensis, Christensenellaceae, Ruminococcaceae Emergencia timonensis, and Lachnospiraceae which have the polynucleotide.

### More specific examples include

Parabacteroides goldsteinii having a polynucleotide having a very high identity to the nucleotide sequence set forth in SEQ ID NO: 1 or 2,
Parabacteroides merdae having a polynucleotide having a very high identity to the nucleotide sequence set forth in SEQ ID NO: 3,
Parabacteroides distasonis having a polynucleotide having a very high identity to the nucleotide sequence set forth in SEQ ID NO: 4 or 5,
Bacteroides dorei having a polynucleotide having a very high identity to the nucleotide sequence set forth in SEQ ID NO: 6 or 7,
Bacteroides thetaiotaomicron having a polynucleotide having a very high identity to the nucleotide sequence set forth in SEQ ID NO: 8 or 9,
Bacteroides uniformis having a polynucleotide having a very high identity to the nucleotide sequence set forth in any one of SEQ ID NOs: 10 to 13,
Alistipes finegoldii having a polynucleotide having a very high identity to the nucleotide sequence set forth in SEQ ID NO: 15 or 16,
Alistipes onderdonkii having a polynucleotide having a very high identity to the nucleotide sequence set forth in SEQ ID NO: 17 or 18,
Odoribacter laneus having a polynucleotide having a very high identity to the nucleotide sequence set forth in SEQ ID NO: 19 or 20,
Odoribacteraceae having a polynucleotide having a very high identity to the nucleotide sequence set forth in any one of SEQ ID NOs: 21 to 24,
Raoultibacter timonensis having a polynucleotide having a very high identity to the nucleotide sequence set forth in SEQ ID NO: 30 or 31,
Christensenellaceae having a polynucleotide having a very high identity to the nucleotide sequence set forth in SEQ ID NO: 36 or 37,
Ruminococcaceae having a polynucleotide having a very high identity to the nucleotide sequence set forth in any one of SEQ ID NOs: 40, 41, and 45,
Emergencia timonensis having a polynucleotide having a very high identity to the nucleotide sequence set forth in SEQ ID NO: 48 or 49, or
Lachnospiraceae having a polynucleotide having a very high identity to the nucleotide sequence set forth in any one of SEQ ID NOs: 58, 61, and 62.

The "bacterium having a polynucleotide encoding 5AR, a polynucleotide encoding 3βHSDH, and a polynucleotide encoding 5BR" according to the present invention is not particularly limited as long as it has a polynucleotide encoding 5AR, a polynucleotide encoding, 3βHSDH, and a polynucleotide encoding 5BR according to the present invention described above, and examples thereof include Parabacteroides goldsteinii, Parabacteroides merdae, Bacteroides dorei, Bacteroides thetaiotaomicron, Bacteroides uniformis, Alistipes finegoldii, Alistipes onderdonkii, Odoribacter laneus, and Odoribacteraceae which have these polynucleotides.

### More specific examples include

Parabacteroides goldsteinii having a polynucleotide having a very high identity to the nucleotide sequence set forth in SEQ ID NO: 1 or 2,
Parabacteroides merdae having a polynucleotide having a very high identity to the nucleotide sequence set forth in SEQ ID NO: 3,
Bacteroides dorei having a polynucleotide having a very high identity to the nucleotide sequence set forth in SEQ ID NO: 6 or 7,
Bacteroides thetaiotaomicron having a polynucleotide having a very high identity to the nucleotide sequence set forth in SEQ ID NO: 8 or 9,
Bacteroides uniformis having a polynucleotide having a very high identity to the nucleotide sequence set forth in any one of SEQ ID NOs: 10 to 13,
Alistipes finegoldii having a polynucleotide having a very high identity to the nucleotide sequence set forth in SEQ ID NO: 15 or 16,
Alistipes onderdonkii having a polynucleotide having a very high identity to the nucleotide sequence set forth in SEQ ID NO: 17 or 18,
Odoribacter laneus having a polynucleotide having a very high identity to the nucleotide sequence set forth in SEQ ID NO: 19 or 20, or
Odoribacteraceae having a polynucleotide having a very high identity to the nucleotide sequence set forth in any one of SEQ ID NOs: 21 to 24.

Note that in the present invention, "very high identity" is preferably 95% or more (96% or more, 97% or more, 98% or more, 99% or more), and particularly preferably 100%. The "identity" of a nucleotide sequence can also be determined using an alignment program such as BLAST. For example, the identity of a nucleotide sequence includes the identity between nucleotide sequences calculated using blastn, and more specifically includes the identity between nucleotide sequences calculated by using the default parameters for blastn (for example, using the initially set parameters).

Further, the bacterium according to the present invention preferably has, in addition to each of the above-mentioned enzymes, a polynucleotide encoding a substrate of each enzyme and/or a transporter of bile acids produced by a reaction catalyzed by each enzyme (see Fig. 12).

Further, the bacterium according to the present invention is not particularly limited as long as it has a polynucleotide encoding the enzyme according to the present invention, and may be an innately possessed bacterium, or a host cell (transformant) such as a bacterium possessed by introducing the above-mentioned DNA or vector according to the present invention externally.

The bacterium into which the DNA or vector according to the present invention is introduced is not particularly limited, and examples thereof include the above-mentioned gut microbiomes and other microorganisms (such as Escherichia coli, budding yeast, fission yeast, Bacillus subtilis, actinomycetes, and filamentous fungi). Further, in the present invention, instead of the above bacteria, it is also possible to use a transformant obtained by introducing the above-mentioned DNA or vector according to the present invention into a cell externally. Examples of such cells include plant cells, insect cells, animal cells, and the like.

The introduction of the DNA or vector according to the present invention can also be carried out according to the methods commonly used in the art. Examples of the method of introduction into bacteria such as Escherichia coli include the heat shock method, the electroporation method, the spheroplast method, the lithium acetate method, and the joining method, examples of the method of introduction into plant cells include a method using Agrobacterium and the particle gun method, examples of the method of introduction into insect cells include a method using baculovirus and the electroporation method, and examples of the method of introduction into animal cells include the calcium phosphate method, the lipofection method, the electroporation method, and the microinjection method.

The DNA or the like introduced into a bacterium or cell in this way may be transiently retained in the bacterium or the like, or may be permanently retained in the bacterium or the like (for example, it may be retained by randomly inserting it into its genomic DNA, or it may be retained by homologous recombination), or if it is a vector, it can be replicated and retained as an independent entity outside its genomic DNA.

### <Composition>

Next, the compositions of the present invention will be described. As shown in Examples described later, the present inventors have revealed that the above-mentioned bacteria according to the present invention are involved in the production pathway of bile acids involved in centenarians and the like. Therefore, the present invention provides compositions of the following aspects.
(1) A composition for converting 3-oxo-Δ⁴-LCA to 3-oxoalloLCA, including a bacterium having a polynucleotide encoding 5AR according to the present invention as an active ingredient.
(2) A composition for converting 3-oxoalloLCA to isoalloLCA, including a bacterium having a polynucleotide encoding 3βHSDH according to the present invention as an active ingredient.
(3) A composition for producing isoalloLCA from 3-oxo-Δ⁴-LCA, including a bacterium having a polynucleotide encoding 5AR according to the present invention and a bacterium having a polynucleotide encoding 3βHSDH according to the present invention as active ingredients.
(4) A composition for producing isoalloLCA from 3-oxo-Δ⁴-LCA, including a bacterium having a polynucleotide encoding 5AR according to the present invention and a polynucleotide encoding 3βHSDH according to the present invention as an active ingredient.
(5) A composition for converting 3-oxo-Δ⁴-LCA to 3-oxoLCA, including a bacterium having a polynucleotide encoding 5BR according to the present invention as an active ingredient.
(6) A composition for producing isoalloLCA from isoLCA or 3-oxoLCA, including a bacterium having a polynucleotide encoding 5AR according to the present invention, a polynucleotide encoding 3βHSDH according to the present invention, and a polynucleotide encoding 5BR according to the present invention as an active ingredient.

In addition, as shown in Examples described later, it has been revealed that bile acids (isoalloLCA and isoLCA), enzymes involved in the production of these bile acids (5AR, 3βHSDH, 5BR), and bacteria producing these enzymes exert antibacterial effects against gram-positive bacteria. Therefore, the present invention also provides compositions of the following aspects.
(7) The composition according to any one of (1) to (4) and (6) above, which is an antibacterial composition against gram-positive bacteria.
(8) antibacterial composition against gram-positive bacteria, comprising: isoalloLCA or isoLCA as an active ingredient.
(9) An antibacterial composition against gram-positive bacteria, including at least one enzyme selected from 5AR according to the present invention, 3βHSDH according to the present invention, and 5BR according to the present invention as an active ingredient.

Further, as shown in Examples described later, it has been revealed that the enzyme according to the present invention (5AR, 3βHSDH) and the bacteria producing the enzymes are involved in the metabolism of testosterone and the like. Therefore, the present invention also provides compositions of the following aspects.
(10) A composition for converting 5α-dihydrotestosterone (DHT) to 3β-androstanediol, including a bacterium having a polynucleotide encoding 3βHSDH according to the present invention as an active ingredient.
(11) A composition for producing 3β-androstanediol from testosterone, including a bacterium having a polynucleotide encoding 5AR according to the present invention and a bacterium having a polynucleotide encoding 3βHSDH according to the present invention as active ingredients.
(12) A composition for producing 3β-androstanediol from testosterone, including a bacterium having a polynucleotide encoding 5AR according to the present invention and a polynucleotide encoding 3βHSDH according to the present invention as an active ingredient.
(13) A composition for producing 3β-androstanediol from DHT, including 3βHSDH according to the present invention as an active ingredient.
(14) A composition for producing 3β-androstanediol from testosterone, including 5AR according to the present invention and 3βHSDH according to the present invention as active ingredients.
(15) The composition according to any one of (10) to (14) above, which is a composition for treating or preventing at least one disease selected from the following disease group
   disease group:
   prostate cancer, androgenetic alopecia, polycystic ovary syndrome, acne, hirsutism, ovarian endothelial cancer, neuroinflammation, and GABAA receptor-mediated epilepsy.

The "bacterium" according to the present invention is as described above, and the "bacterium" contained as an active ingredient in the compositions of the present invention may be a live bacterium or a killed bacterium, or may be both a live bacterium and a killed bacterium. Examples of the "killed bacterium" include a heat-treated one, a calcination-treated one, a steam-treated one, a radiation (such as γ-ray) irradiation-treated one, an enzyme-treated one, a drug (such as antibiotic) treated one, a chemical substance (such as formalin) treated one, and an ultrasonic treated one. Further, the form of the bacterium according to the present invention is not particularly limited and may be either liquid or solid, but the form is suitably a dry form (such as powder form and bacterial powder form) from the viewpoint of easy handling during production and storage. The dried product can be prepared by drying a suspension obtained by dispersing bacteria in a solvent (such as water). The drying method is not particularly limited, and examples thereof include the spray drying method and the freeze drying method. The sterilization method is not particularly limited, and examples thereof include the retort sterilization method, the UHT sterilization method, the air sterilization method, the pressure sterilization method, the high pressure steam sterilization method, the dry heat sterilization method, the steam distribution sterilization method, the electromagnetic wave sterilization method, the electron beam sterilization method, the high frequency sterilization method, the radiation sterilization method, the ultraviolet sterilization method, the ethylene oxide gas sterilization method, the hydrogen peroxide plasma sterilization method, the chemical sterilization method (alcohol sterilization method, formalin fixing method, or electrolyzed water treatment method), and the like. Further, in some cases, before and after the sterilization treatment by heating or the like, or before and after the drying treatment, surfactant treatment, grinding and pulverization treatment, enzyme treatment, fractionation treatment, or the like can also be carried out, and the products obtained by these treatments can also be contained in the compositions of the present invention.

The bacteria contained as an active ingredient in the compositions of the present invention may be not only the above-mentioned bacteria, but also the products by the bacteria (such as secretory products of the bacteria and metabolites of the bacteria) or the constituent substances thereof (Components of the bacteria themselves. So-called bacterial components. Examples thereof include amino acids, peptides, nucleic acids, sugars, lipids, vitamins, hormones constituting the bacteria, and complexes thereof, as well as complexes thereof with phosphorus and sulfur metal elements.).

In addition, the active ingredient contained in the compositions of the present invention may be a culture product of the bacterium according to the present invention. The culture product may be one containing that bacterium (culture broth containing the grown bacterium, solid medium, and the like), and the form of the culture product is not particularly limited and may be either liquid or solid, but the form is suitably a dried form (such as a dried culture product) from the viewpoint of easy handling during production and storage. Those skilled in the art can appropriately prepare such a dried culture product or the like by using the above-mentioned drying method.

The "enzyme" contained as an active ingredient in the compositions of the present invention is as described above. Further, the "bile acid" contained as an active ingredient in the compositions of the present invention is as described in Examples described later, but as long as it has its activity, pharmacologically acceptable salts, hydrates, or solvates are also included. Such pharmacologically acceptable salts are not particularly limited and may be appropriately selected depending on the structure and the like of each compound. Examples thereof include acid addition salts (such as inorganic acid salts including hydrochloride, hydrogen bromide, sulfate, hydrogen sulfate, nitrate, carbonate, hydrogen carbonate, phosphate, monohydrogen phosphate, dihydrogen phosphate, and the like and organic acid salts such as acetate, propionate, lactate, citrate, tartrate, and the like), base addition salts (such as alkali metal salts including sodium salt and potassium salt, alkaline earth metal salts including calcium salt and magnesium salt, zinc salt, aluminum salt, ammonium salt, and tetramethylammonium salt), organic amine addition salts (such as addition salts including morpholine and piperidine), amino acid addition salts (such as addition salts including lysine, glycine, and phenylalanine). The hydrate or solvate is not particularly limited, and examples thereof include those in which 0.1 to 10 molecules of water or solvent are added to 1 molecule of bile acid or salt thereof.

Further, the "biliary acid" according to the present invention includes all isomers and isomer mixtures such as tautomers, geometric isomers, asymmetric carbon-based optical isomers, and stereoisomers, as long as they have inhibitory activity. Furthermore, it also includes compounds in which the above substances undergo metabolism such as oxidation, reduction, hydrolysis, amination, deamination, hydroxylation, phosphorylation, dehydroxylation, alkylation, dealkylation, and conjugation in the living body and still exhibits its inhibitory activity, and the present invention also includes compounds that produce the above substances by undergoing metabolism such as oxidation, reduction, and hydrolysis in the living body.

The compositions of the present invention may be in the form of a pharmaceutical composition (such as a pharmaceutical product or a quasi-drug), a food composition (such as food or drink or animal feed), or a reagent used in model animal experiments and the like.

The compositions in the present invention can be formulated by a known pharmaceutical method according to the form of the substance (such as bile acid, enzyme, or bacterium) as an active ingredient. For example, they can be used as capsules, tablets, pills, liquids, powders, granules, fine granules, film coating agents, pellets, troches, sublingual preparations, chewables, buccals, pastes, syrups, suspensions, elixirs, emulsions, coatings, ointments, plasters, poultices, transdermal formulations, lotions, inhalants, aerosols, injections, suppositories, or the like for oral or parenteral administration (such as intestinal, intramuscular, intravenous, intratracheal, intranasal, transdermal, intradermal, subcutaneous, intraocular, vaginal, intraperitoneal, rectal, or inhaled), or for administration by a route composed of a combination of two or more thereof.

In these formulations, they can be appropriately combined with a carrier that is acceptable pharmacologically or as a food or drink, specifically, physiological saline, sterile water, medium, vegetable oil, solvent, excipient, base, emulsifier, suspension, surfactant, stabilizer, flavoring agent, fragrance, vehicle, preservative, binder, diluent, isotonic agent, soothing agent, bulking agent, disintegrant, buffer, coating agents, lubricants, colorants, sweeteners, thickeners, flavor modifier, lysis aid, or other additives.

Further, in the formulation of the above, in view of the fact that the bacterium according to the present invention is a gut microbiome, a pharmaceutical product intended for oral administration may particularly be combined with a composition that enables efficient delivery of the compositions of the present invention into the intestinal tract. Such a composition that enables efficient delivery into the intestinal tract is not particularly limited, and a known composition can be appropriately employed. Examples thereof include pH-sensitive compositions, compositions that suppress release to the intestinal tract (such as cellulosic polymers, acrylic acid polymers and copolymers, and vinyl acid polymers and copolymers), bioadhesive compositions that specifically adhere to the intestinal mucosa (such as polymers described in US Patent No. 6.368.586), and protease inhibitor-containing compositions and compositions specifically degraded by intestinal enzymes.

When the compositions of the present invention are used as a food composition, they can be, for example, a health food, a functional food, a food for specified health use, a food with nutrient function claims, a food with functional claims, a dietary supplement, a food for patients, or animal feed. Note that functional foods are usually classified into four categories, probiotics, biogenics, prebiotics, and symbiotics, based on their mechanism of action, and may take the form of probiotics, biogenics, symbiotics, and prebiotics in the present invention.

The food or drink of the present invention can be ingested as the compositions as described above, and can also be ingested as various foods and drinks. Specific examples of food or drink include liquid foods such as functional beverages, drinks, jelly-like beverages, refreshing beverages, milk beverages, tea beverages, alcoholic beverages, and soups; fermented foods such as yogurt and drink yogurt, and fermented beverages; products containing oils such as cooking oils, dressings, mayonnaise, and margarine; carbohydrate-containing foods such as rice, noodles, and bread; processed livestock foods such as ham and sausage; processed fishery foods such as kamaboko, dried fish, and salted fish; processed vegetable foods such as pickles; semi-solid foods such as jellies; fermented foods such as miso; various confectioneries such as western confectionery, Japanese confectionery, candies, gums, gummies, cold desserts, and frozen desserts; retort products such as curry, ankake sauce, and Chinese soup; and instant foods such as instant soups and instant miso soups, and microwave-ready foods. Further, healthy foods and drinks prepared in the form of powder, granules, tablets, capsules, liquid, paste or jelly can be mentioned. The food or drink according to the present invention can be produced by a production technique known in the art.

Further, as to the food and drink composition proposed in the present invention, it is also possible to provide and sell it as a food or drink for which health use is displayed. The act of "display" includes all acts of informing the consumer of the above-mentioned use, and the composition itself may be provided with an expression that directly explains the use or an expression that is reminiscent of the use, or such expressions may be attached to the container containing the composition, packaging material, or package insert. Further, the "display" may be used as information related to the present invention compositions, such as a storage medium including a leaflet, a pamphlet, a pop, a catalog, a poster, a book, a DVD, or the like, an advertisement on electronic bulletin boards and the Internet, or the like to display or advertise that the compositions of the present invention are effective.

Regarding the compositions of the present invention (7) to (9), their target "gram-positive bacteria" may be any bacteria that are stained dark blue or purple by gram staining, and are not particularly limited, but examples thereof include gram-positive bacteria shown in Figs. 4A and 4E. In the present invention, "antibacterial" means the suppression of the growth of gram-positive bacteria and/or sterilization. In addition, the compositions of the present invention (7) to (9) can also be used as a composition for treating or preventing infectious diseases of gram-positive bacteria. The "infectious diseases" are not particularly limited, and examples thereof include infectious diseases of Clostridium difficile, sepsis, and acute respiratory distress syndrome (ARDS/ALI) having sepsis as an underlying disease.

Further, regarding the composition of the present invention (15), the target diseases are as described above, and in particular, treatment or prevention of "prostate cancer" also includes suppression of prostate cancer progression and/or metastasis, as shown in the Examples described later.

### <Treatment or Prevention Method>

The present invention also relates to a method for treating or preventing infectious diseases of gram-positive bacteria by administering to a subject an effective amount of at least one enzyme selected from 5AR according to the present invention, 3βHSDH according to the present invention, and 5BR according to the present invention.

Furthermore, the present invention relates to a method for treating or preventing infectious diseases of gram-positive bacteria by administering to a subject an effective amount of at least one bacterium selected from the group consisting of a bacterium having a polynucleotide encoding 5AR according to the present invention, a bacterium having a polynucleotide encoding 3βHSDH according to the present invention, a bacterium having a polynucleotide encoding 5AR according to the present invention and a bacterium having a polynucleotide encoding 3βHSDH according to the present invention, a bacterium having a polynucleotide encoding 5AR according to the present invention and a polynucleotide encoding 3βHSDH according to the present invention, and a bacterium having a polynucleotide encoding 5AR according to the present invention, a polynucleotide encoding 3βHSDH according to the present invention, and a polynucleotide encoding 5BR according to the present invention.

Furthermore, the present invention relates to a method for treating or preventing infectious diseases of gram-positive bacteria by administering to a subject an effective amount of isoalloLCA or isoLCA.

Further, the present invention relates to a method for treating or preventing at least one disease selected from the disease group, including administering to a subject an effective amount of at least one substance selected from the group consisting of a bacterium having a polynucleotide encoding 3βHSDH according to the present invention, a bacterium having a polynucleotide encoding 5AR according to the present invention and a bacterium having a polynucleotide encoding 3βHSDH according to the present invention, a bacterium having a polynucleotide encoding 5AR according to the present invention and a polynucleotide encoding 3βHSDH according to the present invention, 3βHSDH according to the present invention, 5AR according to the present invention, and 3βHSDH according to the present invention.

In the present invention, "prevention" includes suppression and delay of infection, suppression of onset of cancer and the like, delay and suppression of recurrence thereof. "Treatment" includes not only complete recovery from infectious diseases and cancer, but also alleviation or amelioration of those symptoms, suppression of their progression, and suppression of their recurrence.

The "subject" in the treatment and the like of the present invention is an animal including a human. The animals other than humans are not particularly limited, and various livestock, poultry, pets, experimental animals, and the like can be targeted. Specific examples include, but are not limited to, pigs, cows, horses, sheep, goats, chickens, wild ducks, ostriches, ducks, dogs, cats, rabbits, hamsters, mice, rats, monkeys, and the like. Further, the subject according to the present invention may be a person suffering from the above-mentioned infectious diseases and cancer, but need not be diagnosed with these diseases. Examples include those who may have these diseases and those who feel they have these diseases. Moreover, even a healthy person can take it on a daily basis for the purpose of preventing these diseases.

The present technique may also be used for therapeutic or non-therapeutic purposes. "Non-therapeutic use" is a concept that does not include medical practice, that is, treatment of the human body by therapy. An example is health promotion.

The method for administering or ingesting the bacterium, enzyme, and bile acid according to the present invention is not particularly limited and may be oral administration or parenteral administration (for example, administration into the intestinal tract). In the case of oral administration, from the viewpoint of further improving the effects of the bacteria or the like according to the present invention, it is preferable to reduce the production of gastric acid in the subject by ingesting a proton pump inhibitor (PPI) or the like. Further, in the administration or ingestion of these substances, the above-mentioned embodiments of the compositions can be preferably used.

When the bacterium, enzyme, and bile acid according to the present invention are administered or ingested, the dose or intake thereof is appropriately selected according to the age, body weight, symptoms of the above-mentioned diseases, health condition, types of compositions (such as pharmaceutical products and food or drinks), types of active ingredients and their forms. In addition, they may be administered or ingested once or multiple times (for example, twice or three times) per day. Further, the period of administration or ingestion may be discontinued depending on the degree of improvement, but from the viewpoint of prevention, administration or ingestion may be continued without discontinuation. Regarding "continuation", it may be continued every day or at intervals, but it is preferable to continuously administer or ingest it every day in terms of effect.

### <Diagnosis Method and the Like>

The present invention provides a method for evaluating at least one disease selected from the following disease group
disease group:
prostate cancer, androgenetic alopecia, polycystic ovary syndrome, acne, hirsutism, ovarian endothelial cancer, neuroinflammation, and GABAA receptor-mediated epilepsy,
(1) quantifying bacteria that produce 3βHSDH in feces of a subject;
(2) comparing a value obtained by quantifying in step (1) with a corresponding value obtained by quantifying the bacteria of a centenarian; and
(3) as a result of the comparison in step (2),
   determining that the subject is unlikely to catch the disease when the quantitative value in the feces of the subject is equal to or higher than the corresponding value, and
   determining that the subject is highly likely to catch or highly likely to have the disease when the quantitative value in the feces of the subject is lower than the corresponding value.

Further, the present invention also provides a method for preventing or treating at least one disease selected from the disease group, comprising:
in the method, administering a bacterium that produces 3βHSDH to the subject who is determined to be highly likely to catch or highly likely to have the disease.

The present invention also provides a method for evaluating a possibility of survival over 100 years old, comprising:
(1) quantifying at least one bile acid selected from the group consisting of 3-oxoLCA, isoalloLCA, 3-oxoalloLCA, alloLCA, 3-oxoLCA, and isoLCA in feces of a subject;
(2) comparing a value obtained by quantifying in step (1) with a corresponding value obtained by quantifying the bile acid of a centenarian; and
(3) as a result of the comparison in step (2),
   determining that the subject has a high possibility of survival over 100 years old when the quantitative value in the feces of the subject is equal to or higher than the corresponding value, and
   determining that the subject has a low possibility of survival over 100 years old when the quantitative value in the feces of the subject is lower than the corresponding value.

Further, the present invention also provides a method for evaluating a possibility of survival over 100 years old, comprising:
(1) quantifying bacteria that produce at least one enzyme selected from 5AR, 3βHSDH, and 5BR (preferably bacteria that produce at least 3βHSDH2) in feces of a subject;
(2) comparing a value obtained by quantifying in step (1) with a corresponding value obtained by quantifying the bacteria of a centenarian; and
(3) as a result of the comparison in step (2),
   determining that the subject has a high possibility of survival over 100 years old when the quantitative value in the feces of the subject is equal to or higher than the corresponding value, and
   determining that the subject has a low possibility of survival over 100 years old when the quantitative value in the feces of the subject is lower than the corresponding value.

Further, the present invention also provides a method for increasing a possibility of survival over 100 years old, comprising:
in the method, administering at least one substance selected from the group consisting of 3-oxoLCA, isoalloLCA, and bacteria that produce at least one enzyme selected from 5AR, 3βHSDH, and 5BR (preferably bacteria that produce at least 3βHSDH2) to the subject who is determined to have a low possibility of survival over 100 years old.

The present invention also provides a method for evaluating resistance to gram-positive bacteria, comprising:
(1) quantifying at least one bile acid selected from the group consisting of 3-oxoLCA and isoalloLCA in feces of a subject;
(2) comparing a value obtained by quantifying in step (1) with a corresponding value obtained by quantifying the bile acid of a centenarian; and
(3) as a result of the comparison in step (2),
   determining that the subject is highly resistant to gram-positive bacteria when the quantitative value in the feces of the subject is equal to or higher than the corresponding value, and
   determining that the subject is lowly resistant to gram-positive bacteria when the quantitative value in the feces of the subject is lower than the corresponding value.

Further, the present invention also provides a method for evaluating resistance to gram-positive bacteria, comprising:
(1) quantifying bacteria that produce at least one enzyme selected from 5AR, 3βHSDH, and 5BR (preferably bacteria that produce at least 3βHSDH2) in feces of a subject;
(2) comparing a value obtained by quantifying in step (1) with a corresponding value obtained by quantifying the bacteria of a centenarian; and
(3) as a result of the comparison in step (2),
   determining that the subject is highly resistant to gram-positive bacteria when the quantitative value in the feces of the subject is equal to or higher than the corresponding value, and
   determining that the subject is lowly resistant to gram-positive bacteria when the quantitative value in the feces of the subject is lower than the corresponding value.

Further, the present invention also provides a method for preventing infectious diseases of gram-positive bacteria, comprising:
in the method, administering at least one substance selected from the group consisting of 3-oxoLCA, isoalloLCA, and bacteria that produce at least one enzyme selected from 5AR, 3βHSDH, and 5BR (preferably bacteria that produce at least 3βHSDH2) to the subject who is determined to be lowly resistant to gram-positive bacteria.

The "subject" in each evaluation method of the present invention is not particularly limited and is the same as the "subject" in the above-mentioned treatment method and the like, but is limited to humans.

In each evaluation method of the present invention, the bacteria to be quantified may be of at least one kind, but preferably 2 or more kinds (for example, 3 or more kinds, 4 or more kinds), more preferably 5 or more kinds (for example, 6 or more kinds, 7 or more kinds, 8 or more kinds, 9 or more kinds), and further preferably 10 or more kinds (for example, 15 or more kinds, 20 or more kinds, 25 or more kinds, 30 or more kinds).

The "quantification of bacteria in feces" does not have to be the amount (number) of the bacteria themselves, and can be performed, for example, by measuring the amount of the substance peculiar to the bacteria that reflects the amount (number) of the bacteria. Examples of such substances include oligonucleotides specific to the above bacteria (such as 16S rRNA gene and transcripts thereof), constituent substances peculiar to the above bacteria (such as peptides, nucleic acids, sugars, lipids, and complexes thereof, which constitute the bacteria), and products of the bacteria (such as secretory products of the bacteria and metabolites of the bacteria).

When the substance is an oligonucleotide, it can be quantified by using, for example, PCR (RT-PCR, real-time PCR, quantitative PCR), DNA microarray analysis method, northern blotting, next-generation sequencing methods (sequencing-by-synthesis (such as sequencing with Illumina Solexa Genome Analyzer or Hiseq (registered trademark) 2000), pyrosequencing method (such as sequencing with sequencer GSLX or FLX manufactured by Roche Diagnostics (454) (so-called 454 sequencing)), ligase reaction sequencing method (such as sequencing with SoliD (registered trademark) or 5500 xl manufactured by Life Technologies), T-RFLP method, bead array method, in situ hybridization, dot blotting, RNase protection assay method, mass spectrometry, genomic PCR method, or southern blotting.

As to other substances, examples include a method of detection using an antibody (immunological method), such as an enzyme-linked immunosorbent assay method (ELISA method), CLEIA (chemiluminescent enzyme immunoassay method), latex aggregation method, antibody array, immunoblotting, immunochromatography, imaging cytometry, flow cytometry, radioimmunoassay, immunoprecipitation method, or immunohistochemical staining method, and a mass spectrometry method.

In the "immunological method", an antibody that binds to each substance is used, the antibody is brought into contact with each substance to which each antibody binds, and the amount of each substance is detected using the binding property of the antibody to each substance as an index.

The "mass spectrometry (MS) method" refers to a measurement method using a mass spectrometer for detection in which the samples are ionized using an ion source, they are moved in a vacuum in the analysis unit, and the ionized samples are separated according to the mass-to-charge ratio using electromagnetic force or by time-of-flight difference, and as a method of ionization using an ion source, a method such as the EI method, the CI method, the FD method, the FAB method, the MALDI method, and the ESI method can be appropriately selected. Further, as a method for separating the ionized samples in the analysis unit, a separation method can be appropriately selected from a magnetic field deflection type, a quadrupole type, an ion trap type, a time-of-flight (TOF) type, a Fourier transform ion cyclotron resonance type, and the like. Further, a combination of two or more mass spectrometry methods, such as tandem type mass spectrometry (MS/MS) or triple quadrupole mass spectrometry, can be used. In particular, various substances can be measured simultaneously by one measurement by selective reaction monitoring (SRM) or multiple reaction monitoring (MRM) by a triple quadrupole mass spectrometer. In addition, the mass spectrometer may be used alone, or by combining liquid chromatography (LC) or high performance liquid chromatography (HPLC), the target substance can be separated and purified to prepare a sample.

In addition, such "immunological method" and "mass spectrometry" can also be used for "quantification of enzymes in feces" and "quantification of bile acids in feces". Further, in the "quantification of bile acids in feces", various types of chromatography (such as gas chromatography, high performance liquid chromatography, LC/MS, LC-MS/MS, and thin layer chromatography) can be preferably used.

The values obtained by quantifying in this way may be relative values as well as absolute quantity. Examples of the relative value include a quantitative ratio (so-called numerical value expressed in an arbitrary unit (AU)) based on a measuring method or a measuring device used for detection.

Further, the relative value in the amount of bacteria may be, for example, a value (relative abundance, occupancy rate) indicating the proportion of the bacteria in the entire intestinal microbiota, as shown in Examples described later. Further, a value calculated based on the amount of reference gut microbiomes may be used. The "reference gut microbiome" according to the present invention may be a bacterium that is stably present in the fecal samples and the difference in the amount thereof is small between different biological samples.

In each evaluation method of the present invention, the value thus quantified and obtained is compared with the corresponding value of the same substance. Note that the "corresponding value" is a quantitative value in a human individual aged 100 years or older, and may be a value that functions as a cutoff value or a reference value in each method of the present invention, and examples thereof include the median or mean of each substance detected in the human population aged 100 years or older. Further, the "corresponding value" may be a preset quantitative value (fixed value), and it is preferable that such a fixed value is described in the instruction manual or the like of the kit relating to the evaluation method. Moreover, the human individuals aged 100 years or older to be compared are preferably those who have the same characteristics of at least one of gender, race, and nationality as the subject. In addition, such "comparison" can be performed by those skilled in the art by appropriately selecting a statistical analysis method suitable for the above detection method. Examples of statistical analysis methods include Mann-Whitney U-test, t-test, analysis of variance (ANOVA), Kruskal-Wallis test, Wilcoxon test, odds ratio, hazard ratio, Fisher's exact test, receiver operating characteristic analysis (ROC analysis), and classification and regression tree analysis (CART analysis). Also, normalized or standardized and normalized data can also be used for comparison.

"Higher than the corresponding value" or "lower than the corresponding value" means that those skilled in the art can appropriately judge based on the above statistical analysis methods. An example is that the amount of substance detected is higher or lower than the corresponding value, and the difference is statistically significant (for example, P < 0.05). Another example is that the amount of substance detected is 2 times or more (preferably 5 times or more and 10 times or more) or 0.5 times or less (preferably 0.2 times or less and 0.1 times or less) the corresponding value. On the other hand, "equivalent to the corresponding value" means, for example, that the difference between the detected substance amount and the corresponding value is not statistically significant (for example, P > 0.05). A further example is that the amount of substance detected is more than 0.5 times and less than 2 times the corresponding value.

"Highly likely" and "less likely" are, for example, 80% or more and less than 20%, preferably 90% or more and less than 10%, and more preferably 95% or more and less than 5%, respectively. Similarly, "highly resistant to gram-positive bacteria" and "lowly resistant to gram-positive bacteria" mean, for example, that the probability of being infected with gram-positive bacteria is 80% or more and less than 20%, preferably the probability is 90% or more and less than 10%, and further preferably the probability is 95% or more and less than 5%, respectively.

The above determination in each evaluation method of the present invention is usually made by a doctor (including a person who has been instructed by a doctor), but the data on the above-mentioned quantitative values and the like are useful for diagnosis including the judgment of the timing of treatment by the doctor. Therefore, the method of the present invention can also be expressed as a method for collecting data on the above-mentioned quantitative values for the diagnosis by a doctor, a method for presenting the data to a doctor, a method for comparing and analyzing the above-mentioned quantitative values and the above-mentioned corresponding values, or a method for assisting the diagnosis by a doctor in consideration of a patient's clinical symptoms and/or other test results.

Further, the administration based on the above determination is not particularly limited and is the same as the above-mentioned treatment methods and the like.

### <Method for Producing isoalloLCA>

As shown in the Examples described later, the present inventors have found a biochemical synthetic pathway that induces isoalloLCA. Therefore, the present invention provides the following method for producing isoalloLCA.

A method for producing isoalloLCA, comprising: culturing a bacterium having a polynucleotide encoding 3βHSDH according to the present invention in the presence of 3-oxoalloLCA and collecting isoalloLCA produced in the bacterium and/or a culture product thereof.

A method for producing isoalloLCA, comprising: culturing a bacterium having a polynucleotide encoding 5AR according to the present invention and a bacterium having a polynucleotide encoding 3βHSDH according to the present invention in the presence of 3-oxo-Δ⁴-LCA and collecting isoalloLCA produced in the bacteria and/or culture products thereof.

A method for producing isoalloLCA, comprising: culturing a bacterium having a polynucleotide encoding 5AR according to the present invention and a polynucleotide encoding 3βHSDH according to the present invention in the presence of 3-oxo-Δ⁴-LCA and collecting isoalloLCA produced in the bacterium and/or a culture product thereof.

A method for producing isoalloLCA, comprising: culturing a bacterium having a polynucleotide encoding 5AR according to the present invention, a polynucleotide encoding 3βHSDH according to the present invention, and a polynucleotide encoding 5BR according to the present invention in the presence of isoLCA or 3-oxoLCA and collecting isoalloLCA produced in the bacterium and/or a culture product thereof.

A method for producing isoalloLCA, including producing isoalloLCA from 3-oxoalloLCA and collecting the isoalloLCA in the presence of 3βHSDH according to the present invention.

A method for producing isoalloLCA, including producing isoalloLCA from 3-oxo-Δ⁴-LCA and collecting the isoalloLCA in the presence of 5AR according to the present invention and 3βHSDH according to the present invention.

A method for producing isoalloLCA, including producing isoalloLCA from isoLCA or 3-oxoLCA and collecting the isoalloLCA in the presence of 5AR according to the present invention, 3βHSDH according to the present invention, and 5BR according to the present invention.

In the present invention, the "bacteria" to be cultured are as described above, and in the production method of the present invention as well, instead of the bacteria, it is possible to use a host cell into which the DNA or vector according to the present invention has been introduced externally.

The conditions for "culturing bacteria (host cells)" may be any conditions as long as the bacteria or the like can produce isoalloLCA, and those skilled in the art can appropriately adjust and set the temperature, the presence or absence of addition of air, the concentration of oxygen, the concentration of carbon dioxide, pH of the medium (for example, pH 7 to 9), the culture temperature (usually 20 to 40°C and preferably 25 to 37°C), the culture time, the humidity, and the like according to the type of bacteria or the like and the medium to be used. Further, the medium may contain a matter that can be assimilated by bacteria or the like, and examples thereof include carbon sources, nitrogen sources, sulfur sources, inorganic salts, metals, peptones, yeast extracts, meat extracts, casein hydrolysates, serum, and the like. In addition, for example, such a medium may be supplemented with IPTG for inducing the expression of the polynucleotide encoding the enzyme according to the present invention, an antibiotic (such as ampicillin) corresponding to the drug resistance gene that can be encoded by the vector according to the present invention, or a nutrient (such as arginine or histidine) corresponding to the gene that complements the auxotrophy that can be encoded by the vector according to the present invention.

In the present invention, the "culture product" is a medium containing proliferated bacteria or the like, secretory products of the bacteria or the like, and metabolites of the bacteria or the like, which are obtained by culturing the bacteria or the like in a medium, and includes dilutions and concentrates thereof.

The "enzyme" used in the present invention is as described above, and the conditions for producing isoalloLCA in the presence of the enzyme according to the present invention may be any conditions as long as the production reaction is accelerated. Those skilled in the art can appropriately adjust and set the composition of the reaction solution (such as buffer solution), the pH of the reaction solution (for example, pH 7 to 9), the reaction temperature (usually 20 to 40°C and preferably 25 to 37°C), the reaction time, and the like.

The collection of isoalloLCA from such bacteria, culture products thereof, reaction solutions, or the like is not particularly limited, and can be carried out by a known collection and purification method. Examples of such a collection method include extraction with a solvent and various types of chromatography (such as gas chromatography, high performance liquid chromatography, LC-MS, LC-MS/MS, and thin layer chromatography). Further, these methods may be carried out alone or in combination as appropriate in multiple steps. In addition, the timing of collection is appropriately adjusted according to the type of bacteria, enzyme, and the like used, and may be at any time for which isoalloLCA can be produced, but it is usually 1 hour to 14 days, preferably 12 hours to 7 days.

### [Examples]

Hereinafter, the present invention will be described in more detail based on Examples, but the present invention is not limited to the following Examples. In addition, the present Examples were carried out using the methods shown below. In the description of [Examples] here, the numbers in [ ] correspond to the numbers of the cited references listed at the end.

### (Human sample collection)

Collection of fecal samples from the young, the elderly, centenarians, their lineal descendants, and inflammatory bowel disease (IBD) patients was carried out according to the following protocols approved by the Keio University School of Medicine Ethics Committee.
The young healthy donors and IBD patients are based on approval number: 20150075.
The elderly cohort is based on approval number: 20160297 (as part of the Kawasaki Aging and Wellbeing Project). The centenarians and their lineal descendants are based on approval number 20022020 (as part of the Japanese Semisupercentenarian Study [15]).

Note that prior to participation, informed consent was obtained from each donor. In addition, all experiments were carried out in accordance with the rules required by these review committees. The Japanese Semisupercentenarian Study [15] and the Kawasaki Aging and Wellbeing Project are registered as observational studies in the University hospital Medical Information Network Clinical Trial Registry System (ID: UMIN000040447 and UMIN000026053).

### (Metagenomic sequencing and 16S rRNA gene pyrosequencing of human stool samples)

Fecal samples were suspended in PBS containing 20% glycerol and 10 mM EDTA and stored at -80°C until use. After thawing, 100 µL of the suspension was gently mixed with 800 µL of TE10 (10 mM Tris-HCl, 10 mM EDTA) buffer containing RNase A (final concentration 100 µg/mL, Invitrogen) and lysozyme (final concentration 15 mg/mL, Sigma) and incubated at 37°C for 1 hour. Purified Achromopeptidase (final concentration 2,000 U/mL, Wako) was added, and the mixture was further incubated at 37°C for 1 hour. SDS (final concentration 1%) and proteinase K (final concentration 1 mg/mL, Roche) were further added to the mixture, and the mixture was incubated at 55°C for 1 hour. Next, high molecular weight DNA was extracted with phenol: chloroform: isoamyl alcohol (25: 24: 1 at pH 7.9), precipitated with isopropanol (equivalent amount to aqueous phase), washed with 1 mL of 70% ethanol, and gently suspended in 30 µL of TE buffer.

The 16S rRNA sequencing was carried out using MiSeq according to the Illumina protocol. PCR was carried out on the V1-V2 region of the 16S rRNA gene using 27Fmod primer 5'-AGRGTTTGATYMTGGCTCAG-3' (SEQ ID NO: 166) and 338R primer 5'-TGCTGCCTCCCGTAGGAGT-3' (SEQ ID NO: 167). The amplicon ( to 330 bp) obtained from each sample was purified using AMPure XP magnetic beads (Beckman Coulter) .

DNA was quantified using Quant-iT Picogreen dsDNA assay kit (Invitrogen) and Infinite M Plex plate reader (Tecan). The purified DNA was stored at -20°C.

The two paired end reads were combined using fastqjoin program based on the overlapping sequences. The reads that did not match for both universal primers with an average quality value of less than 25 were removed. Both primer sequences were removed, and 3000 reads that had passed the quality filter were arranged in descending order according to quality value, and were clustered using the UCLUST program (Edgar 2010) version 5.2.32 with a cutoff of 97% for the matching degree of pairwise sequence to obtain an operational taxonomy unit (OTU).

Taxonomic assignments for each OTU were made by searching for homology to the Ribosomal Database Project (RDP) and the National Center for Biotechnology Information (NCBI) genome database using the GLSEARCH program.

As to the metagenomic sequencing library, Nextera XT DNA Library Preparation kit (Illumina) was used to pool equal volumes of libraries prepared from 2 ng of input DNA according to the manufacturer's recommended protocols, and the insert size and concentration of each pooled library were determined using an Agilent Bioanalyzer DNA 1000 kit (Agilent Technologies). Sequencing was carried out using Illumina NovaSeq 6000 with 151 bp paired end reads so that approximately 10 million paired end reads could be obtained per sample. The data was analyzed for separation, aggregation, and the like using Broad Picard Pipeline (https://broadinstitute.github.io/picard). Quality control for metagenomic data was carried out by using Trim Galore! to detect and remove sequencing adapters (overlap of 5 bp at minimum) and by using kneadData v0.7.2 to remove contamination of human DNA and remove low quality sequences (HEADCROP: 15, SLIDING WINDOW: 1: 20). Then, sequences having a length of less than 50 bp were removed. Metagenomic reads were assembled individually for each sample into contigs using MEGAHIT [46], followed by an open reading frame prediction with Prodigal [47] and retaining predicted genes that had both a start and a stop codon. A nonredundant gene catalogue was constructed by clustering predicted genes based on sequence homology at 95% identity and 90% coverage of the shorter sequence using CD-HIT [48, 49]. Reads were mapped to the gene catalogue with BWA requiring a unique, strong mapping with at least 95% sequence identity over the length of the read [50], counted (count matrix) and normalized to transcript-permillion (TPM matrix) using in-house scripts. Count matrix served as an input for binning genes into metagenomic species pan-genomes (core and accessory genes) using MSPminer with default settings [51]. The present inventors represented the abundance of every metagenomic species in a sample as a median TPM for 30 top representative core genes reported by MSPminer. Assembled genes were annotated at species, genus, and phylum levels with NCBI RefSeq (version May 2018) as described previously [52]. To annotate phylogenetically metagenomic species that had no match to any species from NCBI RefSeq, the present inventors used Phylophlan with default settings [53]. α-diversities were calculated using Shannon index and β-diversity was calculated using Bray-Curtis dissimilarity based on relative abundances at species levels (R package vegan). The nonredundant gene catalogue was queried using USEARCH ublast with proteins in the bai operon of C. scindens [26] or proteins in the bacterial isolates reported here as 5AR, 5BR, 3βHSDH I, or 3βHSDH II to identify and annotate homologous proteins with at least 40% identity and 80% coverage to the query sequence. Identical processing pipeline has been applied to the dataset describing the gut microbiome in Sardinian centenarians [8].

In the subsequent analysis, the present inventors only used samples with at least 4 million reads after the quality control step. Additionally, the present inventors discarded samples that were collected while the subject was undergoing any antibiotic treatment. To test differential abundance of species or phyla and differences in the Shannon diversity index, the present inventors employed linear random effects modelling (centenarians vs. young or elderly controls) or fixed effects modelling (elderly vs. young controls), as implemented in the lmer() and lm() functions in R. Furthermore, for analysis of species differential abundance, the present inventors restricted the analysis to metagenomic species present in at least 10% of samples, zeros were replaced by half of the smallest non-zero measurement on a per-feature basis and log10 transformation was applied on the relative abundances for normality. Linear modelling included fixed effect covariates: sex (male or female) and cohort information (centenarians, elderly or young); random effect included subject information to account for more than one sample among a few centenarians. The PERMANOVA (as implemented in adonis() function vegan-package in R) was applied to the Bray-Curtis dissimilarity to identify correlation cohort and sex information and the composition of the gut microbiome as a whole. Pairwise Wilcoxon rank sum test was used to evaluate differences in the relative abundance of bai operon homologs in centenarians compared to elderly and young controls. Spearman's rank correlation was used to evaluate trends between relative abundance of Bacteroidetes species encoding 5AR, 5BR, 3βHSDH I or 3βHSDH II genes and the abundance of the secondary bile acids in feces. Overall nominal p-values were adjusted for multiple testing using Benjamini-Hochberg correction and associations at FDR < 0.05 (unless stated differently) were considered as significant.

### (Fecal bile acid quantification using LC/MS/MS)

Accurately weighed 10 mg of freeze-dried fecal samples were suspended in 200 µL of water. Next, 250 µL of the fecal suspension was homogenized by ultrasonication for 1 hour in a screw-cap glass vial containing 3 µL deuterium-labeled internal standard and 747 µL of 0.27 N NaOH (deuterium-labeled internal standards: d₄-CA, d₄-GCDCA, d₄-TCDCA, ds-CDCA-3S, and d₄-LCA, 50 µM each) . After incubation for 1 hour at room temperature, pH was adjusted to 8.0 using 8 N HCl, and mixed with 110 µL of 0.5 M EDTA/0.5 M Tris. Then, the solution was subjected to centrifugation at 15000 rpm, and the supernatant was transferred to a solid-phase extraction cartridge (Agilent Bond Elut C18, 100 mg/3mL, pretreated with 1 mL of methanol and 3 mL of water 3 times). The cartridge was washed with 1 mL of water and the captured bile acids were eluted with 300 µL of 90% ethanol. An amount of 5 µL thereof was injected into LC/ESI-MS/MS (Triple Quad 6500+ tandem mass spectrometer, equipped with an ESI probe and Exion LC AD ultra-high pressure liquid chromatography system; SCIEX). A separation column, InertSustain C18 (150 mm × 2.1 mm ID, 3 µm particle size; GL Sciences Inc.), was utilized at 40°C. A mixed solution of 10 mM ammonium acetate and acetonitrile was used as the eluent and separation was carried out by linear gradient at a flow rate of 0.2 mL/min. The mobile phase composition was gradually changed as follows.
Ammonium acetate: acetonitrile (86: 14, v/v) for 0.5 min, (78: 22, v/v) for 0.5-5 min, (72: 28, v/v) for 5-28 min, (46: 54, v/v) for 28-55 min, (2: 98, v/v) for 55-66 min, and (2: 98, v/v) for 66 min-70 min.
The total run time was 70 min.
To operate the LC/ESI-MS/MS, the following MS parameters were used in the positive ion MRM mode.
   ion spray voltage; 5,500 V, interface temperature; 500°C, curtain gas; 30 psi, collision gas (nitrogen); 9 psi, nebulizing gas; 80 psi, and heater gas; 80 psi.
The following MS parameters were used in the negative ion MRM mode.
   ion spray voltage; -4,500 V, interface temperature; 500°C, curtain gas; 30 psi, collision gas (nitrogen); 9 psi, nebulizing gas; 80 psi, and heater gas; 80 psi.
Samples were obtained using Analyst Software ver 1.71 and analyzed using SCIEX OS-MQ Software ver 1.7.0.36606.

### (Fecal SCFA, pH, and ammonia measurement)

Fecal SCFA concentration was determined by GC-MS (Shimadzu QP 2020 system with a flame ionization detector), equipped with PAL RTC autosampler (CTC Analytics). Helium was used as the carrier gas and fused silica capillary columns 30 m x 0.25 mm coated with 0.25 µm film thickness were used. Injection port temperature was set to 250°C. The initial oven temperature was held at 60°C for 2 min and then ramped to 330°C at a rate of 15°C/min. MS parameters were set to: ion source temperature at 200°C, interface temperature at 280°C and loop time of 0.3 sec. For the GC-MS measurement, 50 µL of fecal samples with concentration of 0.5 µg/µL and 20 µg/µL prepared in ethanol were mixed with 10 µL of acetic acid-d₄ (80 µM). Using PAL RTC autosampler, 4-(4,6-dimethoxy-1,3,5-triazin-2-yl)-4 methylmorpholinium (DMT-MM) and n-octylamine (10 µL of each reagent at a concentration of 80 µM) were added to each fecal sample and reacted for 9 hours prior to injection into GC-MS. Samples were analyzed and quantified using LabSolutions Insight GC-MS software (Shimadzu).

Fecal pH was measured from the supernatant from 0.1 mg/µL of fecal suspension in distilled water using a pH meter (Horiba Ltd.) . From the same fecal suspension, fecal ammonia level was quantified using enzymatic ammonia ELISA assay kit (Abcam) according to the manufacture's protocol.

### (Isolation of bacterial strains from a centenarian)

A fecal sample from a centenarian (centenarian 91 (CE91), Japanese, female, age > 110 years) was dispersed in equal volume (w/v) of PBS containing 20% glycerol, snapfrozen in liquid nitrogen, and stored at -80°C until use. 200 µL of thawed fecal suspension was serially diluted with PBS and 100 µL was seeded onto nonselective (Brucella agar plate with hemin, Vitamin K 1, lysed rabbit blood and defibrinated sheep blood (BHK-RS), Kyokuto) and selective agar plates (for gram-negative bacteria: Paramomycin and vancomycin supplemented BHK, Kyokuyo and for Clostridial bacteria: Oxoid Reinforced Clostridial (RC) Agar, Thermofisher) and grown inside an anaerobic chamber (Coy Laboratory Products) under anaerobic conditions (80% N₂, 10% H₂, and 10% CO₂) at 37°C. Individual colonies emerged after 72 hr and up to 10 days of incubation were picked. Isolated strains were identified by PCR amplification of the 16S rRNA gene region with universal primers (27Fmod: 5'-AGRGTTTGATYMTGGCTCAG-3' (SEQ ID NO: 166), 1492R: 5'-GGYTACCTTGTTACGACTT-3' (SEQ ID NO: 168)) for Sanger sequencing and using the NCBI genome database. Individual isolates in the culture collection were given species name with > 98.0% of 16S rRNA sequence homology, family name with > 94.5% homology, and order name with > 86.5% homology. Bacterial isolates were cryo-preserved in 20% glycerol in optimal culture broth at -80°C.

### (In vitro screening of microbial bile acid metabolism)

Under anaerobic conditions, isolated bacteria strains were cultured together with 50 µM of CDCA, 3-oxoΔ⁴-LCA, or LCA to screen for their bile acid metabolism in a 96-deep well plate (Treff Lab) covered with a gas-permeable membrane (Breathe-easier^{™}, Diversified Biotech). 20 µL of bacterial suspension in exponential to stationary phase was inoculated into 1 mL of Wilkins-Chalgren Anaerobe (WCA, Thermofisher) media adjusted to pH 7 (using MOPS buffer solution, Dojindo) or pH 9 (TAPS buffer solution, Dojindo). Several bacterial strains required growth in RC or WCA media supplemented with additional nutrients. Ruminococcaceae St42-43 and 45, Clostridiales St47, and Lachnospiraceae St56 were cultured in RC medium, while Phascolarctobacterium faecium St52-53 were cultured in RC medium supplemented with sodium succinate (20 mmol/L). For the culture of Akkermansia muciniphila St26-27, WCA medium supplemented with ammonium chloride (1.0 g/L), L-cysteine (1.0 g/L), vitamin K (0.5 mg/L), hemin (5 mg/L), and 0.29% volatile fatty acid solution (based on DSMZ 1611 YCFA modified medium) was used. Alistipes finegoldii St16, Campylobacter ureolyticus St25, Christensenellaceae St36-37, and Ruminococcaceae St44 were cultured in WCA medium supplemented with 4% salt solution (0.2 g/L calcium chloride, 0.2 g/L magnesium sulphate, 1 g/L dipotassium hydrogen phosphate, 1 g/L potassium dihydrogen phosphate, 10 g/L sodium hydrogen carbonate, and 2 g/L sodium chloride), ammonium chloride (1.0 g/L), L-cysteine (1.0 g/L), vitamin K (0.5 mg/L), hemin (5 mg/L), sodium acetate (1.0 g/L), sodium formate (0.15 g/L), sodium fumarate (0.15 g/L), sodium thioglycolate (0.3 g/L), 1% ATCC vitamin solution, and 1% ATCC Trace element solution. For the culture of Methanobrevibacter smithii St67-68, the above modified WCA medium was further supplemented with sodium bicarbonate (0.25 g/L), sodium sulfide (0.05 g/L), and sodium formate (1.36 g/L) [54]. After 48 hr of anaerobic incubation at 37°C, culture supernatants were collected and stored at -20°C until sample preparation for the analysis.

For sample preparation, 100 µL of culture supernatant was transferred into a screw-cap glass vial containing 10 µL of deuterium-labeled internal standards (d4-CA, d4-CDCA, and d4-LCA, 1 nmol/mL each) . 400 µL of water was added and sonicated for 10 min, then applied onto the solid-phase extraction cartridge (Agilent Bond Elut C18, 100 mg/I mL, preconditioned with 1 mL of methanol and 3 mL of water). The cartridge was washed with 1 mL of water and captured bile acids were eluted with 1 mL of 90% ethanol. After solvent evaporation, remaining residue was dissolved in 100 µL of 50% ethanol, of which 5 µL of the solution was injected to LC/ESI-MS/MS (LC-MS8040 tandem mass spectrometer, equipped with an ESI probe and Nexera X2 ultra-high-pressure liquid chromatography system; Shimadzu). A separation column, InertSustain C18 (150 mm x 2.1 mm ID, 2 µm particle size; GL Sciences Inc.), was utilized at 40°C. Mixture A (10 mM ammonium acetate, 0.01% formic acid, and 20% acetonitrile) and mixture B (30% acetonitrile and 70% methanol) were used as the eluent, and the separation was carried out by linear gradient elution at a flow rate of 0.2 mL/min.
The mobile phase composition was gradually changed as follows:
Mixture A: B (80: 20, v/v) for 0.1 min, (48: 52, v/v) for 0.1-1 min, (30: 70, v/v) for 1-27 min, (0: 100, v/v) for 27-27.1 min, (0: 100, v/v) for 27.1-33 min, (80: 20, v/v) for 33-33.1 min, and (80: 20, v/v) for 33.1-83 min. The total run time was 83 min.
To operate the LC/ESI-MS/MS, the following MS parameters were used:
   spray voltage; 3000 V, heating block temperature; 400°C, nebulizing gas flow; 3 L/min, drying gas flow; 15 L/min, interface temperature 300°C, collision gas (argon) pressure; 230 kPa, collision energy; negative (11 to -35 eV); and positive (-16 to -19 eV) ion modes.
Samples were analyzed and quantified using LabSolutions Insight LC-MS software (Shimadzu).

### (Bacterial whole-genome sequencing)

The extracted genomic DNA of 68 isolated strains was sheared to yield DNA fragments. The genome sequences were determined by the whole-genome shotgun strategy using PacBio Sequel and Illumina MiSeq sequencers. The library of the Illumina Miseq 2 x 300 bp paired-end sequencing was prepared using TruSeq DNA PCR-Free kit (target length = 550 bp) and all the MiSeq reads were trimmed and filtered with a > 20 quality value using FASTX-toolkit (v. 0.0.13, hannonlab.cshl.edu/fastx_toolkit). The library of the PacBio Sequel sequencing was prepared using SMRTbell template prep kit 2.0 (target length = 10-15 kbp) without DNA shearing. After removal of internal control and adaptor trimming by Sequel, the error correction of the trimmed reads was performed using Canu (v 1.8) with additional options (corOutCoverage = 10000, corMinCoverage = 0, corMhapSensitivity = high). De novo hybrid assembly of the filter-passed Miseq reads and the corrected Sequel reads were performed using Unicycler (v. 0.4.8), which contained checks for overlapping and circularization to generate a circular contigs. The gene prediction and genome annotation of the generated contigs were performed using the Rapid Annotations based on Subsystem Technology (RAST) Prokaryotic Genome Annotation Server [55] and Prokka: rapid prokaryotic genome annotation software tool [56]. Note that default parameters were used unless otherwise specified.

### (Mutant generation)

The deletion mutants (Δ5AR, Δ5BR, and Δ3βHSDH) of P. merdae St3 were generated by a method developed with modifications from those described for generation of Bacteroides mutants [29]. Briefly, approximately 2 kb sequences flanking the coding region were amplified by PCR. Note that for the PCR primers and the like used in this study, see Table 5 below. They were assembled into the PstI and SalI sites of the suicide vector pLGB30 using HiFi DNA Assembly (NEB) as per the manufacturer's protocol. 1 µL aliquots of each reaction were transformed into electro-competent E. coli MFDpir. Transformants were conjugated with P. merdae St3 as follows.

The donor (E.coli MFDpir) and recipient (P. merdae St3) strains were cultured in LB and BHI media, respectively, to an OD 600 of 0.5 and mixed at a ratio of 1: 1. The mixture was dropped onto a BHI agar plate, and incubated anaerobically at 37°C for 16 hr. Transconjugants were selected on BHI agar plates containing tetracycline (6 µg/mL).

Subsequently, to select for loss of plasmid from the genome by a second crossover, transconjugants were plated on M9 agar supplemented with 0.25% (wt/vol) glucose, 50 mg/L L-cysteine, 5 mg/L hemin, 2.5 µg/L vitamin K1, 2 mg/L FeSO₄·7H₂O, 5 µg/L vitamin B12, and 10 mM rhamnose. Successful deletions were confirmed by PCR and Sanger sequencing.

**[Table 5]**

| Primer Name | Sequence (5'-3') | SEQ ID NO: |
|---|---|---|
| 5AR-upstream-Fw | GTGGATCCCCCGGGCTGCACCGTATCGTCCCAAAGTTGT | 142 |
| 5AR-upstream-Rv | TCTAATTGTTATGATTAACTCATAATAGAT | 143 |
| 5AR-downstream-Fw | AGTTAATCATAACAATTAGAAATAAGGTGA | 144 |
| 5AR-downstream-Rv | CTGGAAGATAGGCAATTAGGTAAGCTGGCGCTTGAAATC | 145 |
| 5AR-check#1-Fw | TTGGCGTTCATCAGTTCAAG | 146 |
| 5AR-check#2-Rv | ACCCTGACTCCGATGTTGAG | 147 |
| 5AR-check#3-Fw | GCTCAGACGGGATCATGAAC | 148 |
| 5AR-check#4-Rv | ATCCTGGCAATGGTCGATGA | 149 |
| 5BR-upstream-Fw | GTGGATCCCCCGGGCTGCAAGTTCGCGACGGTCTTTCTT | 150 |
| 5BR-upstream-Rv | CATAATAGATTGGAAAAAGGATTAGCCATC | 151 |
| 5BR-downstream-Fw | CCTTTTTCCAATCTATTATGAGTTAATCAT | 152 |
| 5BR-downstream-Rv | CTGGAAGATAGGCAATTAGGCCCGGAATCAACTGAAGGA | 153 |
| 5BR-check#1-Fw | CGTCTTCCGGATCCTCGAAG | 154 |
| 5BR-check#2-Rv | GCTATGGCTTTGCGTAACCG | 155 |
| 5BR-check#3-Fw | AATACGGCATCCTGGTTGCA | 156 |
| 5BR-check#4-Rv | CGCTTGGTGGACATTTGCAA | 157 |
| 3bHSDH-upstream-Fw | GTGGATCCCCCGGGCTGCAGGATGAAAGCGATTGCCGAC | 158 |
| 3bHSDH-upstream-Rv | AATATAAGTAACCGGCATGCACTTTTAGTT | 159 |
| 3bHSDH-downstream-Fw | GCATGCCGGTTACTTATATTCTAATTTCTC | 160 |
| 3bHSDH-downstream-Rv | CTGGAAGATAGGCAATTAGACGCCCTGCAGATCATATGG | 161 |
| 3bHSDH-check#1-Fw | CTGGTGTCCTCCTTGTCGTC | 162 |
| 3bHSDH-check#2-Rv | TTTGCTTACGTTGCTGTGCC | 163 |
| 3bHSDH-check#3-Fw | TTTCCCATCACACGTACGGG | 164 |
| 3bHSDH-check#4-Rv | CAAAAGCGTTCGGACAAGCA | 165 |
| 27Fmod | AGRGTTTGATYMTGGCTCAG | 166 |
| 338R | TGCTGCCTCCCGTAGGAGT | 167 |
| 1492R | GGYTACCTTGTTACGACTT | 168 |

### (Bacterial growth inhibition assays)

Clostridioides difficile strain 630 (ATCC BAA-1382), Clostridioides difficile VPI 10463 (ATCC 43255), vancomycin-resistant Enterococcus faecium (ATCC 700221), Streptococcus dysgalactiae subsp. equisimilis (ATCC 12394), carbapenemase-resistant Klebsiella pneumoniae (ATCC BAA-1705), and Salmonella enterica subsp. enterica (ATCC 14028) were purchased from the American Type Culture Collection (ATCC).

Clostridium perfringens (JCM 1290T), Bacillus cereus (JCM 2152T), methicillin-resistant Staphylococcus aureus (JCM 16555), Streptococcus pyogenes (JCM 5674T), Streptococcus sanguinis (JCM 5708T), Proteus mirabilis (JCM 1669T), and Proteus vulgaris (JCM 20013) were purchased from the Japan Collection of Microorganisms (JCM) .

Adherent invasive Escherichia coli was kindly gifted from Prof. Nicolas Banich.

For gut commensals, Clostridium scindens (ATCC 35704T), Clostridium sporogenes (ATCC 15579), Dorea formicigenerans (ATCC 27755T), Ruminococcus lactaris (ATCC 29176 T), Bacteroides fragilis (ATCC 25285T), Clostridium indolis (JCM 1380T), Clostridium hiranonis (JCM 10541T), Clostridium hylemonae (JCM 10539T), Clostridium nexile (JCM 31500T), Clostridium butyricum (JCM 1391T), Dorea longicatena (JCM 11232T), Eubacterium hallii (JCM 31263), Streptococcus thermophilus (JCM 17834T), Ruminococcus gnavus (JCM 6515T), Anaerotruncus colihominis (JCM 15631T), Blautia producta (JCM 1471T), Blautia obeum (JCM 31340), Bifidobacterium bifidum (JCM 1254), Bifidobacterium breve (JCM 1192T), Bifidobacterium longum subsp. longum (JCM 1217T), Lactobacillus casei (JCM 1134T), Lactobacillus paragasseri (JCM 1130), Lactobacillus reuteri (JCM 1112T), Collinsella aerofaciens (JCM 10188T), Roseburia intestinalis (JCM 17583T), Eggerthella lenta (JCM 9979T), Bacteroides caccae (JCM 9498T), Bacteroides finegoldii (JCM 13345T), Bacteroides intestinalis (JCM 13265T), Bacteroides ovatus (JCM 5824T), Bacteroides stercoris (JCM 9496T), Parabacteroides johnsonii (JCM 13406T), and Prevotella copri (JCM 13464T) were obtained from ATCC and JCM.

Previously described Treg inducing strains from the laboratory of the present inventors were also included in the commensal panel. Clostridium symbiosum (VE202-16), Clostridium ramosum (VE202-18), Clostridium bolteae (VE202-7), and Flavinofractor plautii (VE202-3). In addition, Hungatella hathewayi, Eubacterium rectale, and Alistipes putredinis isolated from human feces in the laboratory of the present inventors were used.

Clostridium HGF2 (innocuum) HM287 was obtained through BEI Resources, NIAID, and NIH as part of the Human Microbiome Project: Clostridium sp., Strain HGF2, HM-287.

From fresh colonies grown on BHK blood agar plates (Kyokuto), a primary suspension adjusted to OD 600 of 0.63 was prepared in WCA medium. Subsequently, the secondary suspension was prepared by diluting 100 µL of primary suspension into a total of 2.4 mL of medium. 10 µL of secondary suspension was inoculated to a total of 200 µL of medium containing varying concentrations (3.175, 6.25, 12.5, 25, and 50 µM) of bile acids; DCA, LCA, 3-oxoLCA, 3-oxoalloLCA, isoLCA, alloLCA, and isoalloLCA. The growth of bacteria was monitored every 0.5-1 hr by OD 600 measurement using a microplate reader (Sunrise Thermo, Tecan) set at 37°C with a 60 sec shaking before each time point. For determining the minimal inhibitory concentration, 10 µL of secondary suspension was inoculated into a total of 200 µL of medium containing 0.25 to 50 µM of isoalloLCA.

### (Electron microscopy (EM))

Bacterial cultures incubated with or without isoalloLCA were collected after 5 hr incubation for electron microscopy samples. For scanning electron microscopy, 10-30 µL of culture was spotted on the Nano percolator membrane (JEOL) and fixed in freshly prepared 2.5% glutaraldehyde solution. After overnight fixation at 4°C, samples were washed in 0.1 M phosphate buffer (pH 7.4, Muto Pure Chemicals), fixed with 1.0% osmium tetroxide (TAAB Laboratories) for 2 hr at 4°C, and treated with a series of increasing concentrations of ethanol. Samples were dried up with critical point dryer (CPD300, Leica Biosystems) and coated with about 2 nm thickness of osmium using a conductive osmium coater (Neoc-ST, Meiwafosis) . SEM images were acquired using the SU6600 (Hitachi High Tech) at electron voltage of 5 keV.

For transmission electron microscopy, microbial pellets were prepared by centrifugation (13,000 rpm, 2 min) from 25 mL bacterial cultures. Pellets were fixed with 2.5% glutaraldehyde solution overnight at 4°C. After washing with 0.1 M phosphate buffer, samples were fixed with 1.0% osmium tetroxide for 2 hr at 4°C, washed in distilled water and embedded into low gelling temperature Type VII-A agarose (Sigma-Aldrich). Samples were dehydrated by a series of increasing concentrations of ethanol to absolute ethanol, soaked with acetone (Sigma-Aldrich), with n-butyl glycidyl ether (Okenshoji Co., Ltd.), with graded concentration of Epoxy resin, and also with 100% Epoxy resin (100 g Epon (trade name) was composed of 27.0 g MNA, 51.3 g EPOK-812, 21.9 g DDSA, and 1.1 mL DMP-30, all from Okenshoji Co., Ltd.) for 48 hr at 4°C. Polymerization of pure Epoxy resin was completed for 72 hr at 60°C. The ultra-thin sections (70 nm) were prepared on copper grids (Veco Specimen Grids, Nisshin-EM) with ultramicrotome (Leica UC7, Leica Biosystems), and stained with uranyl acetate and lead citrate for 10 min each. TEM images were obtained using the JEM-1400plus (JEOL) at electron voltage of 80-100 keV.

### (Culturing human feces with bile acids)

Human fecal culture was conducted in 96-deep well plates (Treff Lab) using stool samples obtained from young and healthy donors. 5 mg of stool was inoculated for use into 1 mL of WCA medium supplemented with 4% salt solution (0.2 g/L calcium chloride, 0.2 g/L magnesium sulphate, 1 g/L dipotassium hydrogen phosphate, 1 g/L potassium dihydrogen phosphate, 10 g/L sodium hydrogen carbonate, and 2 g/L sodium chloride), ammonium chloride (1.0 g/L), L-cysteine (1.0 g/L), vitamin K (0.5 mg/L), hemin (5 mg/L), sodium acetate (1.0 g/L), sodium formate (0.15 g/L), sodium fumarate (0.15 g/L), sodium thioglycolate (0.3 g/L), 1% ATCC vitamin solution, and 1% ATCC Trace element solution based on media previously used for human fecal batch culture [59, 60]. Fecal cultures with a final concentration of 50 µM bile acids (LCA, 3-oxoLCA, and isoalloLCA) were incubated anaerobically for 48 hr at 37°C. DNA was extracted from the fecal sample culture for 16S metagenomic sequencing as described above.

### (Quantification of steroid hormones using LC-MS/MS LC-MS/MS)

Parabacteroides merdae St3 and Odoribacteraceae St21-24 strains isolated from CE91, together with 50 µM testosterone or 5α-dihydrotestosterone (DHT), were anaerobically cultured in 96-well plates (Treff Lab) covered with a gas-permeable membrane (Breathe-easier^{™}, Diversified Biotech). 20 µL of bacterial culture in exponential to stationary phase was inoculated into 1 mL of Wilkins-Chalgren Anaerobe (WCA, Thermofisher) media adjusted to pH 7 (using MOPS buffer solution, Dojindo) . After culturing for 3 hours, 6 hours, 9 hours, and 12 hours, each culture supernatant was collected for LC-MS/MS quantification.

For sample preparation, 40 µL of culture supernatant was mixed with 160 µL of deuterium-labeled internal standards (testosterone-d 3, 12.5 nM methanol solution) and 800 µL of 80% methanol. The sample was sonicated at room temperature for 10 minutes and then passed through a filter having a pore size of 0.45 µm (Pure Clean for Solvent 0.45 µm, KURABO). 2 µL of solution was injected into LC/ESI-MS/MS (Triple Quad 6500+ tandem mass spectrometer, equipped with an ESI probe and Exion LC AD ultra-high pressure liquid chromatography system; SCIEX). A separation column, InertSustain C18 (150 mm × 2.1 mm ID, 2 µm particle size; GL Sciences Inc.), was utilized at 40°C. Mixture A (5 mM ammonium formate in distilled water) and mixture B (5 mM ammonium formate in methanol) were used as gradients, and the separation was carried out by linear gradient elution at a flow rate of 0.2 mL/min. The mobile phase composition was gradually changed as follows:
Mixture A: B (60: 40, v/v) at 0 min, (100: 0, v/v) at 20-25 min, and (60: 40, v/v) at 25.1-30 min. The total run time was 30 min.
To operate the LC-ESI-MS/MS, the following MS parameters were used:
   ion spray voltage; 5,500 V, interface temperature; 500°C, curtain gas; 30 psi, collision gas (nitrogen); 9 psi, nebulizing gas; 80 psi, and heater gas; 80 psi. Samples were collected using Analyst software ver 1.71 and analyzed using SCIEX OS-MQ software ver 1.7.0.36606.

### (Statistical Analysis)

All statistical analyses were performed using GraphPad Prism software (GraphPad Software, Inc.). Oneway analysis of variance with Tukey's test (parametric) and Kruskal-Wallis with Dunn's test (nonparametric) were used for multiple comparisons. Mann-Whitney test with Welch's correction (nonparametric) was used for all comparison between two groups. Wilcoxon signed-rank test post hoc test with Bonferroni correction (nonparametric) was used to compare group means. Spearman's rank correlation was used to investigate the correlation between two variables.

### (Example 1) Intestinal microbiota signatures of centenarians

Three age groups (centenarian (n = 160), elderly (n = 112), and young (n = 44)) were analyzed. All centenarians were recruited as part of the Japanese Semisupercentenarian Study [15], most of whom live in nursing facilities (85%), and the others live in their homes (9.4%), and are hospitalized in medical facilities (5.6%). It has been reported that centenarians have low activities of daily living (ADL), mini-mental state examination values (MMSE), red blood cell counts and serum albumin levels.

Some centenarians show signs of mild inflammation, as evidenced by elevated levels of serum C-reactive protein and fecal lipocalin [9, 12, 13], which is consistent with the preconceived notion that aging is associated with reduced barrier integrity and chronic inflammation due to immunosenescence (Figs. 5C and 5D). Nevertheless, the majority of centenarians did not have chronic diseases such as obesity, diabetes, hypertension, and cancer, and the prevalence of these diseases was not significantly increased compared to the elderly group (Figs. 5E and 5F, and Table 6).

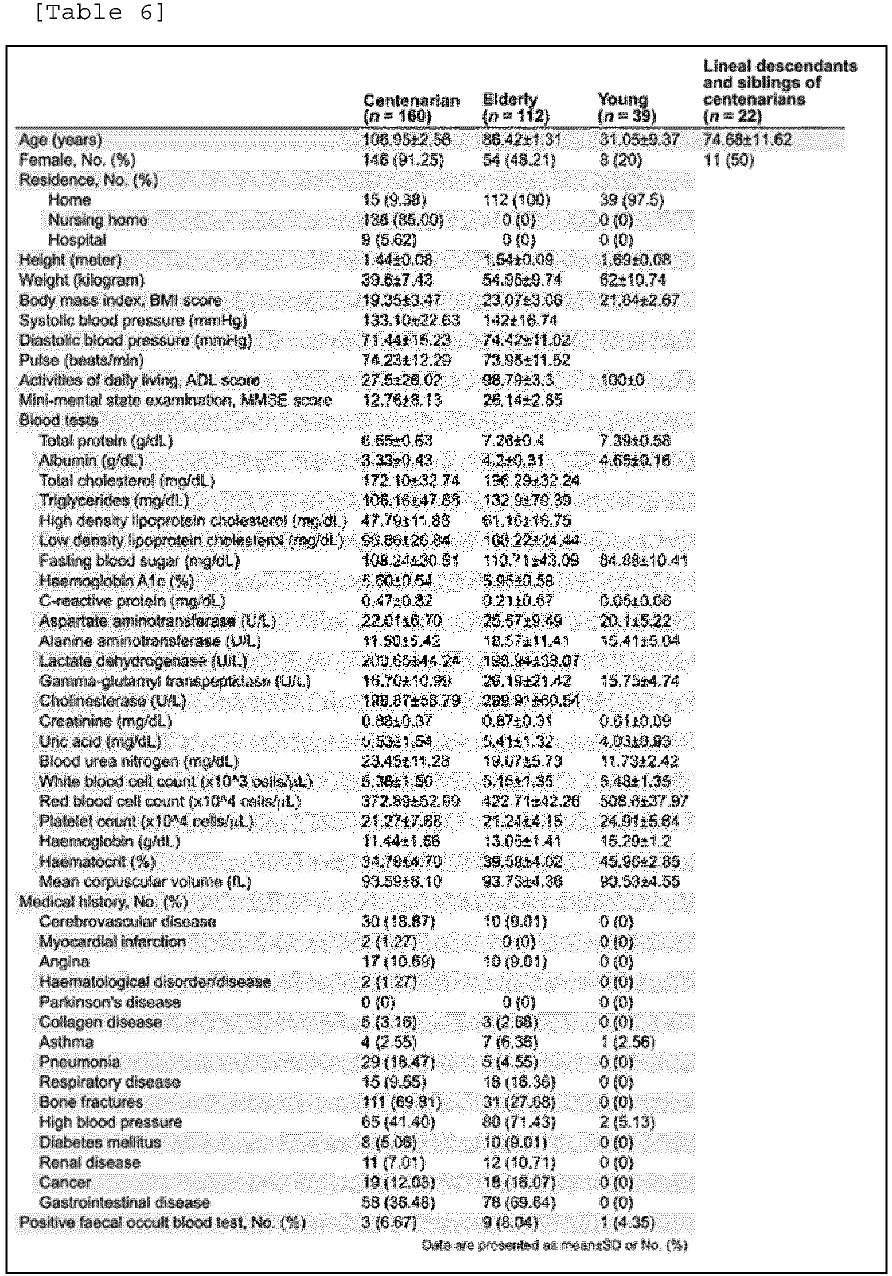

Fecal samples were collected from the three groups and the microbiota was analyzed using both 16S rRNA amplicons and whole metagenomic shotgun sequencing. As a result, the main coordinate analysis (PCoA) based on the Bray Curtis distance revealed that there was a significant difference in the composition of the microbiota between the centenarians and both control groups (PERMANOVA FDR < 0.05, Fig. 1A). At the phylum level, a significant increase in Proteobacteria and Synergistetes, a moderate increase in Verrucomicrobia, and a decrease in Actinobacteria (actinomycetes) were observed in the centenarians compared to the control group (Figs. 1C and 6) .

Such growth of Proteobacteria is common in patients with inflammatory bowel disease (IBD), but in contrast to the commonly observed decrease in gut microbiome diversity in IBD patients, the intestinal microbiota of the centenarians had a high Shannon index on average, even when compared with young people, and diversity was observed (Fig. 1B). Furthermore, the composition of the centenarian microbiota was different from that of IBD patients and was classified as a different set in the main factor analysis (Fig. 7A).

In addition, in some taxa, a relative difference in abundance was observed between the centenarian group and the control groups (Figs. 1D to 1F and Figs. 7B to 7D). These were grouped into three feature groups based on the aging trajectory.

The first feature group is a taxon whose abundance increased or decreased with age (Fig. 1D). For example, the unknown Firmicutes species (msp_161) or Eubacterium siraeum was the most common among the centenarians, followed by the elderly and then the young. On the other hand, Blautia wexlerae showed the opposite tendency, with the youngest being the most, followed by the centenarians, and then the elderly. In addition, Alistipes shahii was relatively predominant in both the elderly and centenarian groups compared to the young. These findings were consistent with previous studies suggesting that the relative abundance of these taxa reflects adaptation to aging and may be related to physical activity, environment, and diet [4-6, 8].

The second feature group is a taxon different from that of the elderly, although the abundance is similar between the centenarians and the young (Fig. IE). These species may contribute to the maintenance of youth and may have anti-aging effects. In particular, the unknown Firmicutes (msp_197), Ruminococcus gnavus, and Eggerthella lenta were relatively predominant in both centenarians and the young. Interestingly, the latter two species are involved in bile acid metabolism and may be involved in the biosynthesis of iso-bile acids in the host intestines [18].

The third feature group is a taxon specific to the centenarians, and its abundance is significantly different between the centenarians and the control groups (elderly and young), so that it is not positioned between the two control groups (Fig. 1F). In this group, Alistipes, Parabacteroides, and Clostridium species, and the major archaea in the human intestine, Methanobrevibacter, were particularly abundant in the centenarians compared to the control groups. One of the most abundant species in the centenarians is Clostridium scindens, which is known to have the relatively rare ability of 7α-dehydroxylation required to convert primary bile acids to secondary bile acids [19, 20]. In contrast, major butyrate-producing bacteria such as Faecalibacterium prausnitzii and Eubacterium rectale were selectively depleted in centenarians (Fig. 1F).

In addition, feces were collected from the lineal descendants and siblings of the centenarians and analyzed by 16S rRNA sequencing (n = 22 from 14 centenarians, 48-95yo, Figs. 7A-7D and Table 6). Some bacterial species such as Phascolarctobacterium faecium and Alistipes putredinis were more abundant in the centenarians and their families than in the control groups (Fig. 7D). The increase in these taxa in the centenarians and their lineal descendants may be due to heredity, lifestyle, and diet. For example, consumption of Brassicaceae vegetables has been reported to favor the expansion of the taxa described above [21].

### (Example 2) Bile acid profile peculiar to centenarians

Next, the results of metabolite analysis in centenarian feces were evaluated in comparison with the elderly and young controls. First, short-chain fatty acids (SCFA) in feces were analyzed and found to have reduced levels of both propionic acid and butyric acid in the centenarians (Fig. 8A). On the other hand, branched short-chain fatty acids such as isobutyric acid and isovaleric acid, and ammonia were elevated in the centenarians (Figs. 8A and 8B).

These metabolic changes may be due to a simultaneous decrease in SCFA-producing bacteria such as R. intestinalis and F. prausnitzii ([22], Fig. 1F) and an increase in protein-fermenting organisms such as Alistipes putredinis ([23], Fig. 1F). This increase in amino acidutilizing bacteria may be the result of a diminished proteolytic capacity of the upper gastrointestinal tract commonly observed in centenarians.

Furthermore, fecal pH was significantly higher in the centenarians than in the controls (Fig. 8C), which may be due in part to lower SCFA concentrations and reduced production of gastric juice characteristic of aging. Given the increased number of species that may metabolize bile acids in centenarians, the present inventors then focused and analyzed the distribution of bile acids in feces.

Primary bile acids are synthesized from hepatic cholesterol, bind to either glycine or taurine, and are secreted into the bile ducts [24, 25]. These primary bile acids are then uncoupled by the intestinal microbiota and converted to various secondary bile acids [19, 25]. The main biochemical conversion is 7α-dehydroxylation of primary bile acids (cholic acid (CA) and chenodeoxycholic acid (CDCA)), and thereby, they are converted into secondary bile acids (deoxycholic acid (DCA) and lithocholic acid (LCA)).

Bile acid metabolism via microbiota is composed of multiple redox reactions catalyzed by enzymes encoded by the bile acid-inducible (bai) operon genes (BaiB, BaiCD, BaiA2, BaiE, BaiF, and BaiH), as shown below [19, 26]. In addition to 7α-dehydroxylation, bile acids undergo oxidation and epimerization to be converted to oxo-(keto-), iso- (3β-hydroxy), and allo- (5α-H-), as well as cis- and trans isomers [25].

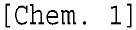

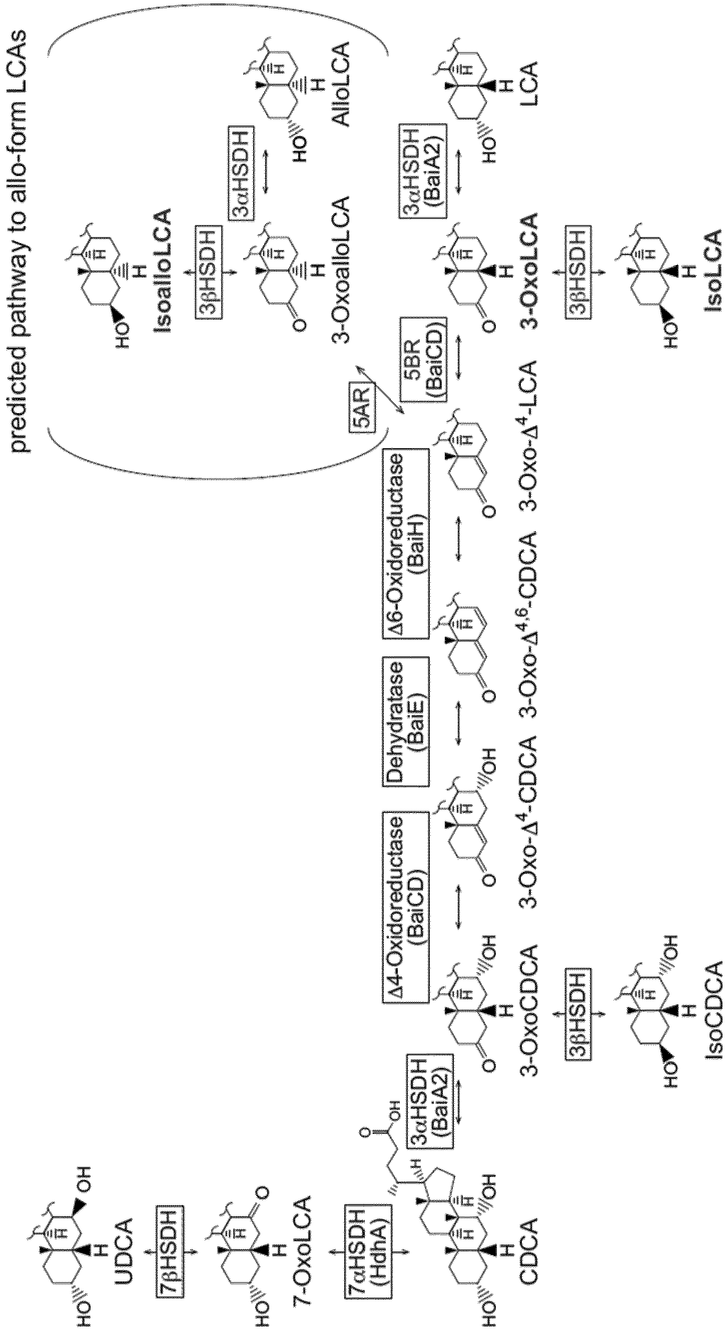

Metagenomic analysis confirmed an increase in the relative abundance of genes homologous to the centenarian bai operons (Fig. 1G). However, the metagenomics of Sardinian centenarians did not yield similar results (Fig. 9) .

High-sensitivity target liquid chromatography-tandem mass spectrometry (LC-MS/MS) was performed to investigate the bile acid distribution of centenarians. Preliminary studies have revealed that 95 of the 132 target bile acids are trace components (< 0.5 µmol/g) in centenarian feces. Therefore, the remaining 37 bile acid compounds were selected for quantitative analysis (Table 7).

**[Table 7]**

| Acronym | Compound Name | Chemical Formula | CAS |
|---|---|---|---|
| CDCA | Chenodeoxycholic acid | C₂₄H₄₀O₄ | 474-25-9 |
| CDCA3S | Chenodeoxycholic acid 3-sulfate | C₂₄H₄₀O₇S | na |
| TCDCA | Tauro-chenodeoxycholic acid (sodium salt) | C₂₆H₄₄NNaO₆S | 6009-98-9 |
| GCDCA | Glyco-chenodeoxycholic acid (sodium salt) | C₂₆H₄₂NNaO₅ | 16564-43-5 |
| 3-Oxo-Δ⁴-CDCA | 3-Oxo-4-cholenoic acid | C₂₄H₃₆O₃ | 1452-29-5 |
| 3-Oxo-Δ^{4,6}-CDCA | 3-Oxo-4,6-choladienoic acid | C₂₄H₃₄O₃ | 88179-71-9 |
| LCA | Lithocholicacid | G₂₄H₄₀O₃ | 434-13-9 |
| | Lithocholicacid3-sulfate (disodiumsalt) | C₂₄H₃₈Na₂O₆S | 64936-81-8 |
| TLCA | Trauro)-lithocholic acid (sodium salt) | C₂₆H₄₄NNaO₅S | 6042-32-6 |
| TLCA3S | Taurolithocholic acid 3-sulfate (disodium salt) | C₂₆H₄₃NNa₂O₈S₂ | 64936-83-0 |
| IsoLCA | Iso-lithocholic acid | C₂₄H₄₀O₃ | 1534-35-6 |
| IsoalloLCA | Iso-allo-lithocholic acid | C₂₄H₄₀O₃ | 2276-93-9 |
| 3-OxoLCA | 3-Oxo-lithocholic acid | C₂₄H₃₈O₃ | 1553-56-6 |
| 7-OxoLCA | 7-Oxo-lithocholic acid | C₂₄H₃₈O₄ | 4651-67-6 |
| UDCA | Ursodeoxycholic acid | C₂₄H₄₀O₄ | 128-13-2 |
| UDCA3S | Ursodeoxycholic acid 3-sulfate | C₂₄H₄₀O₇S | na |
| TUUCA | Tauro-ursodeoxycholic acid (sodium salt) | C₂₆H₄₄NNaO₆S | 35807-85-3 |
| IsoCDCA | Iso-chenodeoxycholic acid | C₂₄H₄₀O₄ | 566-24-5 |
| IsoDCA | lso-deoxycholic acid | C₂₄H₄₀O₄ | 570-63-8 |
| 3-OxoCDCA | 3-Oxo-chenodeoxycholic acid | C₂₄H₃₈O₄ | 4185-00-6 |
| 3-OxoDCA | 3-Oxo-deoxycholic acid | C₂₄H₃₈O₄ | 4185-01-7 |
| CA | Cholic acid | C₂₄H₄₀O₅ | 81-25-4 |
| CA3S | Cholic acid 3-sulfate | C₂₄H₄₀O₈S | na |
| TCA | Tauro-cholic acid | C₂₆H₄₅NO₇S | 81-24-3 |
| GCA | Glyco-cholic acid (sodium salt) | C₂₆H₄₂NNaO₆ | 863-57-0 |
| IsoCA | Iso-cholic acid | C₂₄H₄₀O₅ | 3338-16-7 |
| 3-OxoCA | 3-Oxo-cholic acid | C₂₄H₃₈O₅ | 2304-89-4 |
| 7 -OxoDCA | 7-Oxo-deoxycholic acid | C₂₄H₃₈O₅ | 911-40-0 |
| DCA | Deoxycholic acid (sodium salt) | C₂₄H₃₈NaO₄ | 302-95-4 |
| DCA3S | Deoxycholic acid 3-sulfate | C₂₃H₃₈O₇S | na |
| TDCA | Tauro-deoxycholic acid (sodium salt) | C₂₆H₄₄NNaO₆S | 1180-95-6 |
| GDCA | Glyco-deoxycholic acid | C₂₆H₄₃NO₅ | 360-65-6 |
| UCA | Ursocholic acid | C₂₄H₄₀O₅ | 2955-27-3 |
| Δ5-3β-OH | 3β-Hydroxy-5-cholenoic add | C₂₄H₃₈O₃ | 5255-17-4 |
| HCA | Hyocholic acid | C₂₄H₄₀O₅ | 547-75-1 |
| HDCA | Hyodeoxycholic acid | C₂₄H₄₀O₄ | 83-49-8 |
| MDCA | Murideoxycholic acid | C₂₄H₄₀O₄ | 668-49-5 |

As a result, after filtration, the total bile acid content in the weight-normalized fecal suspension was not significantly different between the centenarians and the control groups. However, a unique distribution was observed in the fecal bile acids of centenarians (Figs. 2A-2F, and Figs. 10A and 10B). For example, centenarians had lower levels of primary bile acids and associated higher levels of CDCA metabolites (Figs. 2D-2F). In particular, the levels of iso-LCA, 3-oxoLCA, isoalloLCA, allo-LCA, and 3-oxoallo-LCA were significantly elevated in centenarians, but these increases were not seen in the elderly and the young, suggesting that these were not merely a by-product of aging (Figs. 2A and 2F). In addition, the concentrations of alloLCA, 3-oxoallo-LCA, and isoalloLCA are positively correlated with fecal pH (Fig. 8D), suggesting that these increases in bile acid levels in centenarians may reflect changes in diet and digestive functions, and the consequent changes in intestinal metabolome. Such an intestinal environment may promote the growth of certain bacterial species and/or the expression of enzymes involved in the production of isoLCA, 3-oxoLCA, and isoalloLCA. Alternatively, colonization of isoLCA-producing bacteria, 3-oxoLCA-producing bacteria, and isoalloLCA-producing bacteria in the intestines may causally affect other members of the intestinal microbiota and their metabolic processes.

### (Example 3) Identification of isoLCA-producing bacterial strains, 3-oxoLCA-producing bacterial strains, and isoalloLCA-producing bacterial strains

Next, the present inventors attempted to identify bacterial strains and enzymes involved in the biosynthesis of isoLCA, 3-oxoLCA, and isoalloLCA in the centenarian microbiota.

Previous reports show that from 3-oxo-Δ⁴-DCA (also referred to as 3-oxo-4, 5-dehydro-DCA), hydrogenation of the beta position of C5 in the C4-C5 double bond makes it possible to produce 3-oxoDCA. It is reported that this reaction is catalyzed by 3-oxo-5β-steroid 4-dehydrogenase (also referred to as 5β reductase, 5BR) encoded by the BaiCD gene carried by a small number of Clostridium species, including C. scindens and C. hylemonae [26, 27].

Furthermore, it has been reported that E. lenta and R. gnavas can generate 3-oxoDCA from DCA and isoDCA from 3-oxoDCA by the action of 3α-hydroxysteroid dehydrogenase (3αHSDH) and 3βHSDH, respectively [18].

Therefore, it was hypothesized that the production of 3-oxoLCA and isoLCA involves 5BR, 3αHSDH, and 3βHSDH as well as 3-oxoDCA and isoDCA, as shown below.

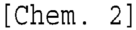

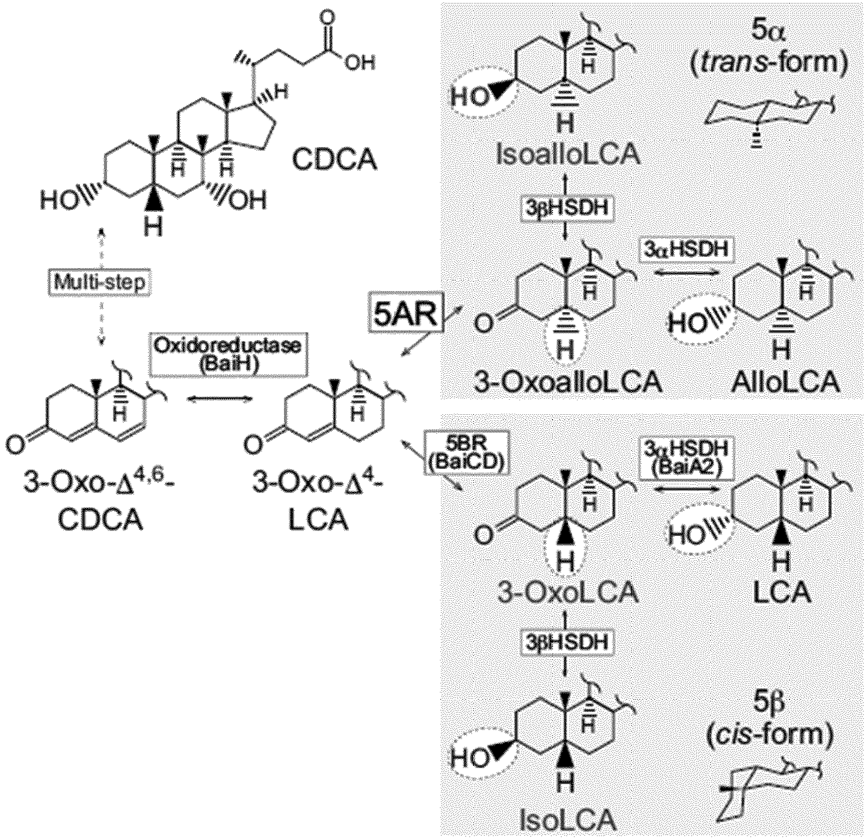

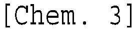

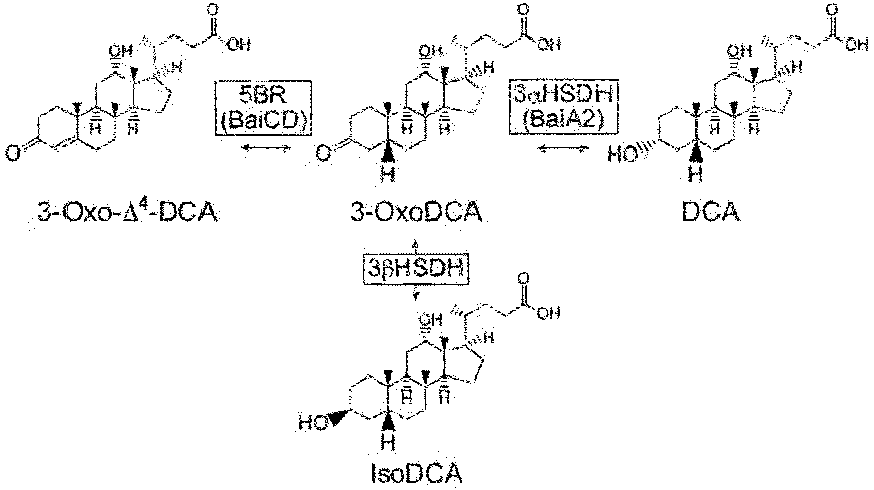

On the other hand, the biosynthetic pathway leading to the production of isoallo bile acid has not been reported so far. The present inventors predicted that isoalloLCA might be produced from 3-oxo-Δ⁴-LCA by the continuous action of a 5α-reductase (5AR) analog and 3βHSDH via the 3-oxo-allolCA intermediate. 5AR is known to mediate the conversion of testosterone to 5α-dihydrotestosterone by hydrogenating the C4-C5 double bond, thereby distorting the A and B steroid rings into a planar (trans) conformation ([28], see below). It was considered that 3-oxoalloLCA (trans bile acid) could arise from 3-oxo-Δ⁴-LCA via a similar pathway.

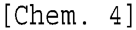

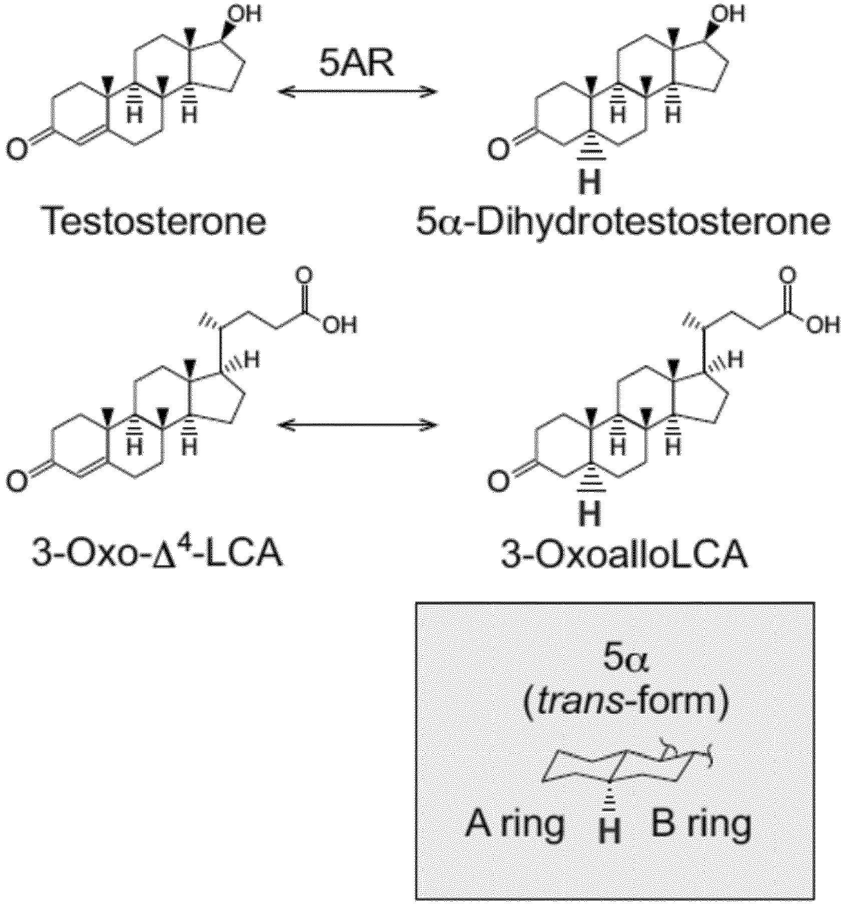

The present inventors also predicted that the subsequent conversion of 3-oxoalloLCA to isoalloLCA might involve 3βHSDH, which is responsible for the previously investigated conversion of 3-oxoDCA to isoDCA [18].

In order to verify the biosynthetic pathways of isoLCA, 3-oxoLCA, and isoalloLCA, and to identify the bacterial strains that produce their secondary bile acids, a detailed investigation was conducted on a centenarian (CE91, 110 years or older) who showed high concentrations of isoLCA, 3-oxoLCA, and isoalloLCA in the feces without showing major abnormalities in blood tests (Fig. 2A).

Specifically, first, CE91 fecal specimens were cultured in various media, and bacterial colonies were analyzed by 16S rRNA gene sequencing to prepare an aggregate of 68 characteristic strains that roughly reproduced the microbiota structure of CE91 (Fig. 3A and tables 8 to 11).

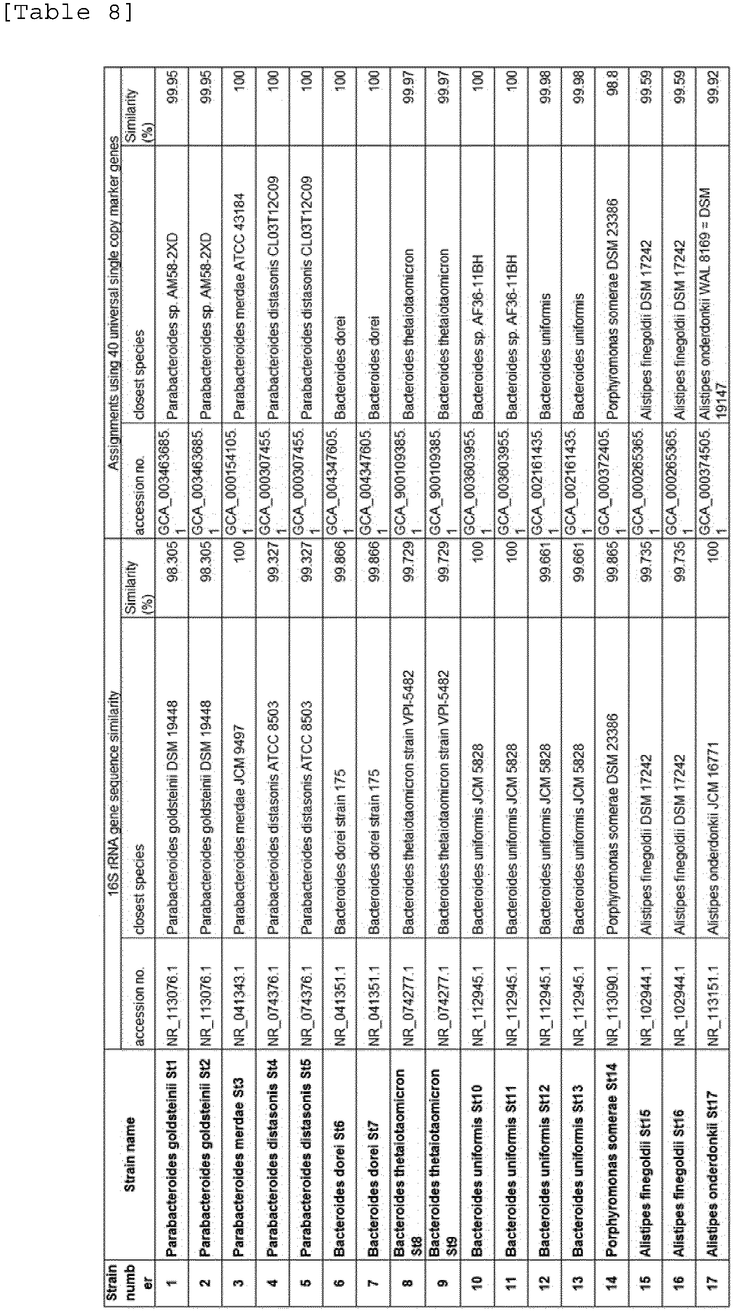

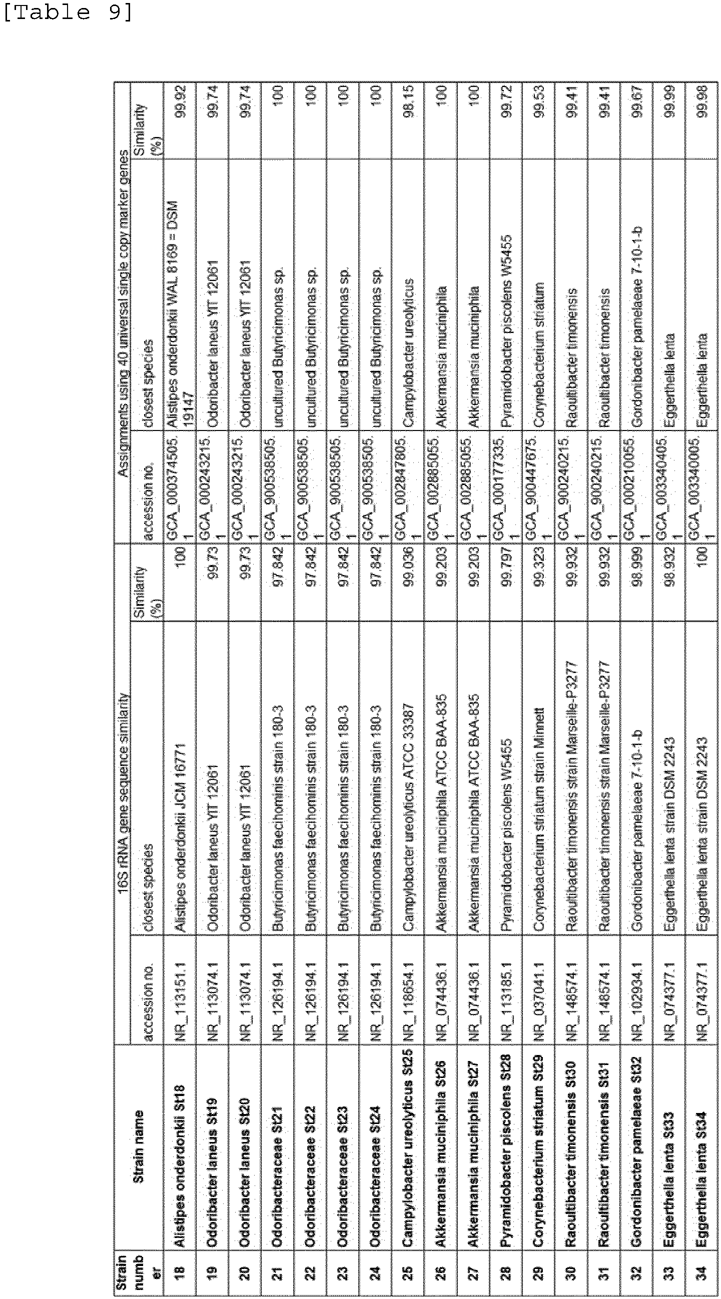

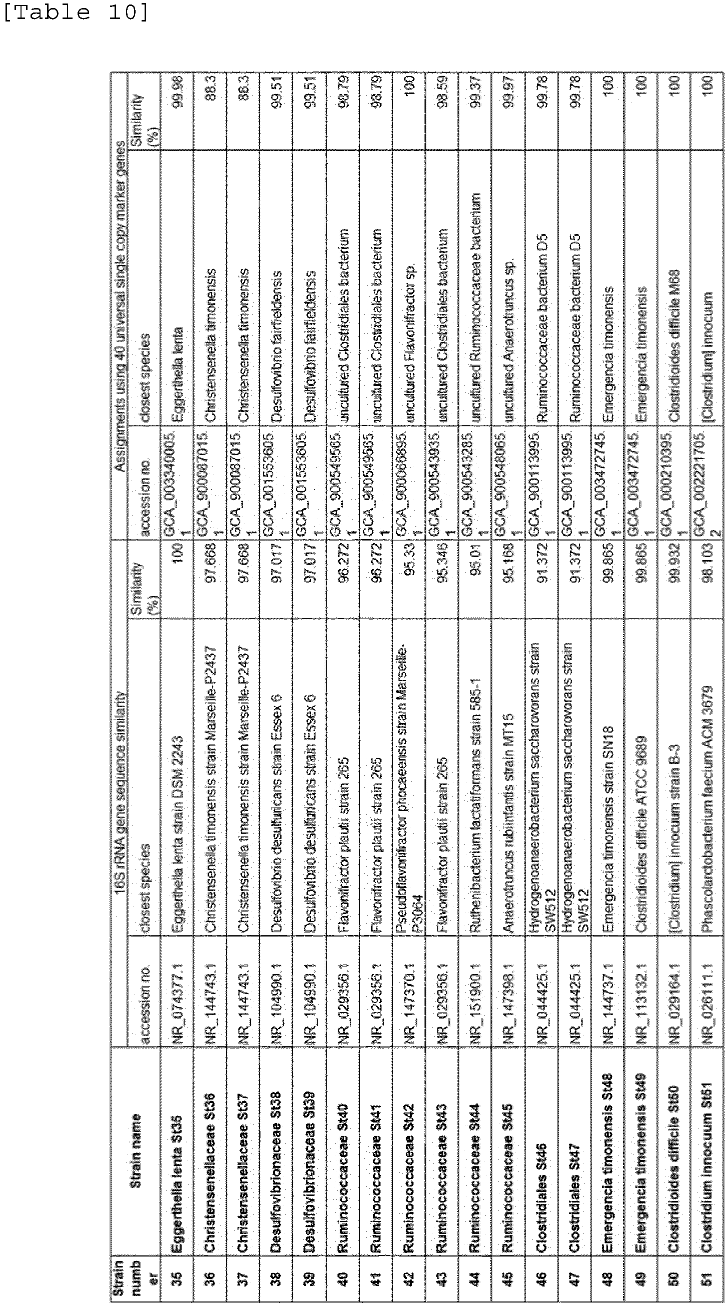

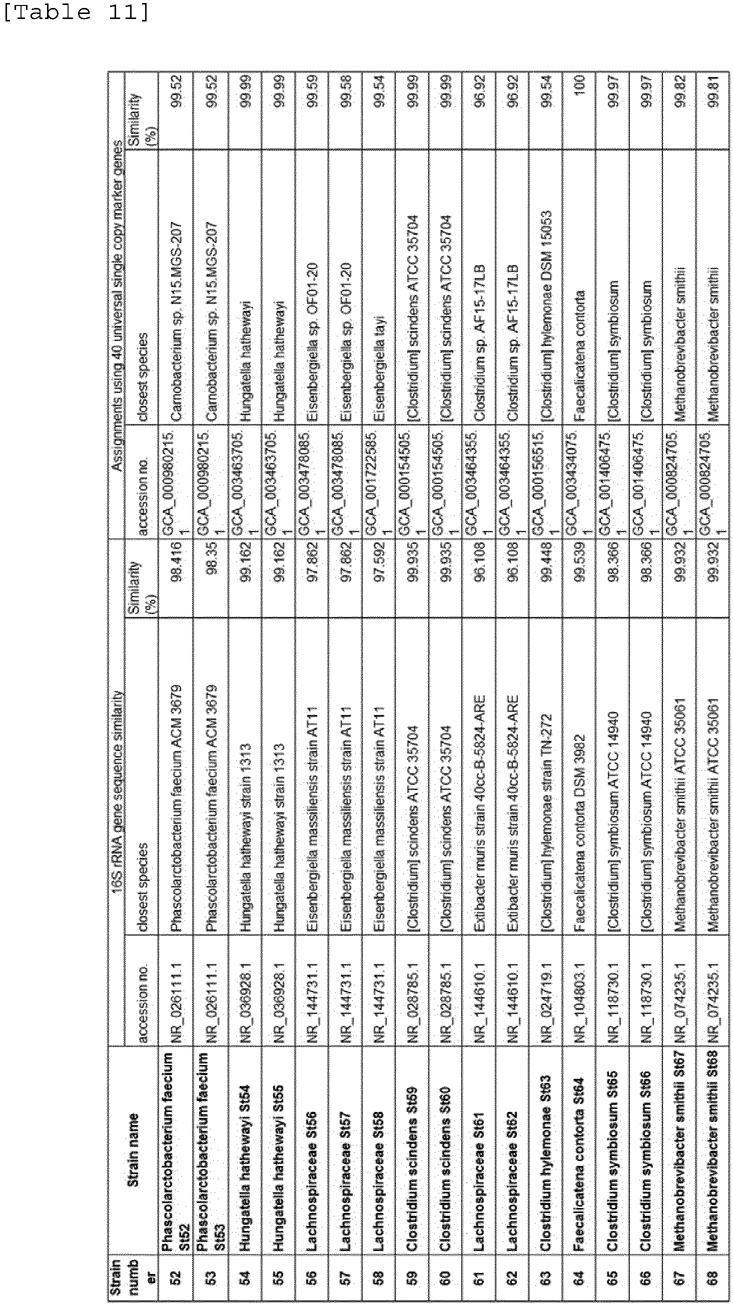

Next, using any of CDCA, LCA, and 3-oxo-Δ⁴-LCA as a starting substrate, the individual isolates were cultured at pH 7 or pH 9, and after 48 hours, the culture supernatant was collected, and the bile acids were quantified by LC-MS/MS (Figs. 3B and 3C, and Figs. 11A to 11F).

As a result, it was confirmed that in the culture using CDCA as a substrate, iso-, 3-oxo-, or isoallo-LCA was not detected in any of the culture supernatants, but the C. scindens strains 59-60 (St59-60) and C. hylemonae St63 previously reported [27] produced LCA, albeit at low levels (Fig. 11A and 11B).

In the case of culture using LCA as a substrate, Gordonibacter pamelaeae St32 and E. lenta St33-35 produced 3-oxoLCA and isoLCA (Figs. 3B and 11C and 11D), and from this, as predicted in the previous study [18], these strains were considered to carry 3αHSDH and 3βHSDH.

Furthermore, Raoulibacter timonensis St30-31 and Lachnospiraceae spp. St57 were also able to convert LCA to 3-oxoLCA, suggesting that these species had 3αHSDH as well as E. lenta.

When 3-oxo-Δ⁴-LCA was used as a substrate, 3-oxoLCA accumulated at high levels in the culture supernatants of Hungatella hathewayi St54-55 and Lachnospiraceae spp. St62, suggesting that these strains had 5BR (Fig. 3C and Figs. 11E and 11F).

Similarly, isoLCA was produced at high levels from 3-oxo-Δ⁴-LCA in the culture supernatants of Clostridium innocuum St51 and Lachnospiraceae spp. St58 (Fig. 3C, and Figs. 11E and 11F), suggesting that these strains carried 5BR and 3βHSDH.

Parabacteroides distasonis St4-5 converted LCA to 3-oxo-Δ⁴-LCA via 3-oxoLCA (Fig. 3B and Figs. 11C and 11D), and furthermore, the conversion from 3-oxo-Δ⁴-LCA to isoLCA and LCA (Fig. 3C, and Figs. 11E and 11F) was noteworthy, suggesting that these strains carried 3αHSDH, 3βHSDH, and 5BR.

As described above, it was revealed that among the 68 strains, at least 12 strains had a strong 3-oxoLCA-producing ability. In addition, it was revealed that the 8 strains had the ability to produce isoLCA from LCA or 3-oxo-Δ⁴-LCA.

Surprisingly, in cultures using 3-oxo-Δ⁴-LCA as a substrate, significant accumulation of isoalloLCA was observed in Parabacteroides merdae St3, Odoribacter laneus St19, and Odoribacteraceae spp. St21-24, and in addition, accumulation of isoalloLCA was also observed at low levels in Bacteroides dorei St6-7 (Fig. 3C and Figs. 11E and 11F), suggesting that these strains carried both 5AR and 3βHSDH activity.

In addition, all culture products of Parabacteroides goldsteinii St1-2, Bacteroides thetaiotaomicron St9, B. Uniformis St10-13, Alistipes finegoldii St15-16, A. onderdonkii St17-18, and O. laneus St20 showed sufficient accumulation of the presumed intermediate 3-oxoalloLCA. Meanwhile, the accumulation level of isoalloLCA was very low (Fig. 3C and Figs. 11E and 11F). It is considered that while these strains probably carried 5AR, they did not have the activity of 3βHSDH or were inactive under these culture conditions.

Therefore, it was revealed that a total of 20 Bacteroides strains (St1-24 strains excluding St4, 5, 8, and 14 strains) could convert 3-oxo-Δ⁴-LCA to 3-oxoalloloLCA, 8 strains of which were found to be able to reliably produce isoalloLCA.

Notably, cultures at pH 9 representing the intestinal environment of centenarians enhanced the accumulation of 3-oxoalloLCA, isoalloLCA, isoLCA, and 3-oxoLCA compared to cultures at pH 7 (Figs. 11A to 11F), which suggests that an alkaline environment may accelerate the activity or expression of the enzymes involved in the production of these bile acids.

### (Example 4) Conversion from 3-oxo-Δ⁴-LCA to isoalloLCA involving 5AR and 3βHSDH

To test the hypothesis that the predicted biosynthetic pathways, especially 5BR and 3α-/3β-HSDH are involved in the conversion of 3-oxo-Δ⁴-LCA to 3-oxoLCA and isoLCA, and 5AR and 3βHSDH are involved in the production of isoalloLCA, Miseq and PacBio were used in an integrated manner to sequence all 68 strains of genes.

When these genomic sequences were queried, it was revealed that the Eggerthella strain carried the 3αHSDH gene as previously reported [18] (Figs. 3B and 3C). It was revealed that P. distasonis St4 and 5 strains carried a gene predicted to be 3αHSDH. This result is consistent with the in vitro evaluation result of bile acid metabolism.

In addition, orthologous sequences with more than 30% amino acid sequence similarity to human 5AR (steroid 5 alpha-reductase 1, SRD5A1) were identified in 21 Bacteroidetes strains (Figs. 3B-3D (under color display, shown in red), and Fig. 12).

Next, genes directly adjacent to the predicted 5AR locus were analyzed. As a result, clusters of genes related to bile acid metabolism function were found in all 21 strains, including the NADH: flavin oxidoreductase sequence predicted to be 5BR (Figs. 3B to 3D (under color display, shown in blue), and Fig. 12).

The present inventors also identified sequences of short-chain dehydrogenase (SDR) that predicted to be 3βHSDH. These SDR sequences were composed of two groups. Group I showed high sequence similarity (> 40%) with 3βHSDH of P. merdae St3. Meanwhile, group II was interrelated with each other, but not with 3βHSDH of P. merdae St3 (Figs. 3B to 3D (under color display, group I is shown in green and group 2 is shown in purple), and Fig. 12).

In addition, the present inventors found sequences near the gene cluster appearing to encode bile acid transporters (Fig. 12).

It was found from the above that with the exception of the St8 strain, the possession of genes expected to be 5AR and 3βHSDH was clearly associated with the production of 3-oxoalloLCA and/or isoalloLCA from 3-oxo-Δ⁴-LCA (Fig. 3C) .

In order to further elucidate the related biosynthetic pathways, 24 Bacteroidales isolates were cultured using 3-oxoalloLCA, 3-oxoLCA, or isoLCA as a substrate, and their bile acid conversion abilities were examined in vitro (Figs. 13A to 13D).

As a result, the efficiency of converting bile acids was different between the substrate specificity and the strains, but the pattern of bile acid conversion was basically consistent with the predicted pathways. For example, P. merdae St3, P. distasonis St4-5, B. dorei St7, and Odoribacteraceae St21 showed strong 3βHSDH activity, showing isoalloLCA production from 3-oxoalloLCA and isoLCA production from 3-oxoLCA (Fig. 13B). Meanwhile, the activity of some other strains such as B. dorei St6 and B. uniformis St10-13 was low with or without the putative 3βHSDH gene (Figs. 13A and 13B).

The intensity of 5BR activity also differed between strains. P. distasonis St4-5 and B. dorei St7 efficiently converted 3-oxoLCA to 3-oxo-Δ⁴-LCA, while other strains showed moderate to weak activity (Fig. 13B).

Porphyromonas somerae St14 lacked the putative 5AR and 3βHSDH genes, but could produce isoalloLCA from 3-oxoalloLCA (Fig. 13A), suggesting that it carried the strain-specific gene having 3βHSDH activity.

To investigate whether isolates with different genes could coordinately metabolize bile acids, E. lenta St34 (having 3αHSDH, 3βHSDH) or P. distasonis St4 (having 3αHSDH, 3βHSDH, 5BR) and P. merdae St3 or Odoribacteraceae St21 (having 5BR, 5AR, 3βHSDH) were co-cultured in the presence of LCA.

As a result, isoalloLCA was produced in a coordinated manner in all combinations, and the highest yield was observed in the co-culture of E. lenta St34 and P. merdae St3 (Fig. 13D).

From the above, although differences were observed between strains in enzyme activity, substrate specificity, and gene locus, the Bacteroides gene clusters identified above may contribute to the coordinated production of bile acids and may at least be involved in the unique fecal bile acid composition distribution found in centenarians.

To further confirm the roles of 5AR and 3βHSDH in bile acid metabolism mediated by microbiota, mutant strains of P. merdae and Odoribacteraceae were prepared.

For the P. merdae St3 strain, by plasmid introduction via conjugation and selection of double-crossover resolvents using rhamnose-inducible ssBfe1 cassette, the present inventors succeeded in producing three mutants lacking the gene encoding the estimated 5AR (PM3806), 3βHSDH (PM3804), or 5BR (PM3805) ([29], Figs. 14A and 14B) .

As a result, as expected, when culturing using 3-oxo-Δ⁴-LCA as a substrate, P. merdae Δ5AR could not produce either 3-oxoalloLCA or isoalloLCA. P. merdae Δ3βHSDH produced 3-oxoalloLCA, but could not generate isoalloLCA (Fig. 3F).

When 3-oxoalloLCA was used as a substrate, P. merdae Δ5AR produced isoalloLCA in the same manner as its parent strain (wild strain), but P. merdae Δ3βHSDH did not produce isoalloLCA (Fig. 3F).

Furthermore, P. merdae Δ3βHSDH was unable to convert 3-oxoLCA to isoLCA, confirming that 3βHSDH can utilize both trans- and cis-bile acids as substrates. P. merdae Δ5BR produced isoalloLCA from 3-oxo-Δ⁴-LCA or 3-oxoalloLCA, but could not convert 3-oxoLCA to 3-oxo-Δ⁴-LCA (Fig. 3F).

These findings support the involvement of 5AR, 3βHSDH, and 5BR in the production of isoalloLCA, 3-oxoLCA, and isoLCA by the human intestinal microbiota.

### (Example 5) The bactericidal effect of isoalloLCA against gram-positive pathogens

Secondary bile acids are known to play several biologically important roles, including regulation of host metabolism and immune response (including regulatory T cell induction, [25, 30-35]) and prevention of pathogen growth in the intestinal tract [36-39]. In particular, DCA, LCA, and isoLCA are known to be involved in inhibiting the growth of Clostridioides difficile, which is an antibiotic-resistant bacterium that requires the most urgent measures [36, 40, 41].

Therefore, next, it was investigated whether 3-oxoLCA and isoalloLCA had the ability to inhibit the growth of C. difficile. Specifically, C. difficile 630 was cultured in the presence of various concentrations of isoLCA, 3-oxoLCA, isoalloLCA, 3-oxoalloLCA, LCA, DCA, or control groups, and growth in vitro was followed over time using optical density measurements.

As a result, surprisingly, isoalloLCA strongly inhibited the growth of C. difficile 630 (Figs. 4A and 4B, and Figs. 13A and 13B). The minimum inhibitory concentration (MIC90) required to prevent growth of 90% or more in WCA medium was 2.0 µM, well below the concentrations of other bile acids tested (Figs. 4A and 4B, and Fig. 13A). Strong growth inhibition by isoalloLCA was also observed in toxin-producing C. difficile VPI10463 and vancomycin-resistant Enterococcus faecium (VRE) (Figs. 4A and 4B, and Figs. 13A and 13B).

Also, observations using scanning and transmission electron microscopy has shown that isoalloLCA is bactericidal and results in morphological changes and hyperfine structure changes in C. difficile 630 and VRE, including cell wall collapse, swelling, and multiple wall penetrations (Fig. 4C). Note that these patterns of damage are often observed with β-lactam antibiotics [42].

Co-culture with Odoribacteraceae St21 in the state of supplementation with 3-oxo-Δ⁴-LCA resulted in significant growth inhibition of C. difficile 630 and VRE as well as isoalloLCA treatment (Fig. 4D).

In contrast, no bacteriostatic effect was observed when co-cultured with C. innocuum St51 (isoLCA-producing bacteria) or P. distasonis St4 (isoLCA and LCA-producing bacteria).

Next, investigation was carried out on the effects of isoalloLCA on gram-positive pathogens including methicillin-resistant Staphylococcus aureus (MRSA), Streptococcus dysgalactiae subsp. equisimilis (SDSE), Clostridium perfringens, Streptococcus pyogenes, Streptococcus sanguinis, and Bacillus cereus and on gram-negative pathogens including Klebsiella pneumoniae, Escherichia coli, Salmonella enterica, Proteus vulgaris, and Proteus mirabilis. Although S. aureus is a well-known skin pathogen, it often forms colonies in the intestines and is known to be resistant to most bile acids [43]. IsoalloLCA strongly inhibited the growth of all gram-positive pathogens tested, including S. aureus, with MIC90 values ranging from 0.5 to 3 µM on WCA medium and 3 to 6.25 µM on BHI medium (Figs. 4A and 4B, and Figs. 15A and 15B) .

In contrast, all bacteria in the gram-negative pathogen panel were found to be resistant to isoalloLCA even at the highest concentrations tested (50 µM) (Figs. 4A and 4B, and Figs. 15A and 15C).

These results suggest that isoalloLCA exerts strong bactericidal/bacteriostatic effects by interfering with the cell wall of bacteria, especially against gram-positive pathogens. It is also suggested that isoalloLCA exerts preventive and therapeutic effects on skin diseases such as atopic dermatitis caused by S. aureus.

### (Example 6) Effects of isoalloLCA on commensal intestinal microbiota

Since intestinal metabolites encounter not only intestinal pathogens but also beneficial symbiotic bacteria, the present inventors investigated how isoalloLCA affected common bacteria in the human intestinal microbiota.

Bacteria constituting a total of 42 common intestinal microbiota composed of both gram-positive bacteria and gram-negative bacteria were selected from the culture collection and cultured in WCA and BHI media with increasing levels of isoalloLCA.

As a result, IsoalloLCA did not significantly affect the growth of most gram-negative symbiotic bacteria such as Bacteroides (Figs. 4E and 16A).

In contrast, it reliably inhibited the growth of gram-positive symbiotic bacteria (Figs. 4E and 16A). Note that the MIC90 value of the symbiotic strains was generally higher than that of the pathogens, and it was clearly confirmed by scanning electron microscopy that the cell wall structure of the symbiotic bacteria was maintained even when C. sporogenes, C. indolis, and C. HGF2 (innocuum) were cultured in 2.5 µM isoalloLCA (C. difficile 1.25 times the MIC90 value) (Fig. 16B). In particular, the Lactobacillus strain was found to be highly resistant to the inhibitory effect of isoalloLCA (Fig. 4E).

In addition, culturing the symbiotic strain in BHI medium rich in peptone and amino acids increased resistance to isoalloLCA compared to culturing in WCA medium, but the pathogens generally maintained susceptibility regardless of the medium (Figs. 4A, 4E, and 17) .

From the above results, it has been shown that the pathogens are always more susceptible than the symbiotic bacteria, although the concentration at which isoalloLCA exerts bactericidal effects on gram-positive bacteria depends on the environmental conditions.

In addition, in order to assess the effects of isoalloLCA on the complex normal intestinal microbiota, human fecal microbiota from young, healthy sample donors were cultured using isoalloLCA, 3-oxoLCA, LCA, or control systems and analyzed for bacterial microbiota changes by 16S rRNA gene sequencing.

As a result, α-diversity was not significantly affected, but isoalloLCA resulted in a clear widespread change in the bacterial community structure at the phylum level (Figs. 4F and 4G).

After culturing together with isoalloLCA, a more pronounced decrease in gram-positive bacteria such as Clostridium, Faecalibacterium, Bifidobacterium, and Streptococcus and a corresponding increase in gram-negative bacteria such as Bacteroides and Alistipes were observed, as compared to other bile acid compounds (Fig. 4G) .

These results are consistent with the increased relative abundance of Bacteroides and Alistipes found in the intestinal microbiota of centenarians, showing that isoalloLCA may directly affect the community structure of gut microbiome.

Since it was confirmed that 5AR, 5BR, and 3βHSDH played an important role in the production of characteristic secondary bile acid derivatives, the present inventors returned to the metagenomic sequence data of Japanese centenarians and examined other species carrying these genes. As a result, the 5AR, 5BR, and 3βHSDH gene clusters were detected in 35 types of bacteria (all Bacteroidetes) (Fig. 18). Then, the abundance of these bacterial species was evaluated in relation to the abundance of isoLCA, 3-oxoLCA, and isoalloLCA.

As a result, in the case of isoalloLCA, a significant positive correlation was observed with a large number of Alistipes sp., Bacteroides cellulosilitycus, B. intestinalis, and P. goldsteinii. In addition, these species were also positively correlated with the levels of isoLCA and 3-oxoLCA (Fig. 18).

In contrast, Bacteroides vulgatus and Bacteroides ovatus were found to be significantly negatively correlated with the three secondary bile acids tested. O. laneus showed a significant or trend-level positive correlation with 3-oxoLCA, isoLCA, and isoalloLCA, but no significant association between P. merdae and these bile acids was observed (Fig. 18).

These results suggest that expression and activity of the identified genes are regulated in vivo through species-specific and intestinal environment-dependent (possibly including interbacterial and bacterial-host interactions) complex mechanisms.

As mentioned above, in the present Examples, the present inventors have succeeded in identifying the signatures of the intestinal microbiota of people aged 100 years or older and identifying the bacterial species and genes/pathways that promote the production of isoLCA, 3-oxoLCA, and isoalloLCA.

It has been reported that isoalloLCA induces Treg cells, and 3-oxoLCA and isoLCA suppress T helper 17 (TH17) cells [30], and accumulation of these bile acids may protect against excessive immune response and inflammation.

The centenarians who participated in this and previous studies [1-3] did not suffer from metabolic diseases or chronic diseases such as cancer, which are thought to be associated with abnormal activation of the immune system and immunosenescence, and this supports the above possibility [12].

In addition to the previously reported antiinflammatory properties, isoalloLCA also exerted very strong antibacterial effects against gram-positive pathogens, as revealed in the present Examples. Bile acids have been reported to contribute to protection against intestinal pathogenic bacterial infections [36, 44, 45]. However, isoalloLCA has been shown to function as the most potent antibacterial agent with high specificity against gram-positive bacteria, including multidrug-resistant pathogens.

It is suggested from the present Examples that accelerating the inhibition of the colonization of gram-positive pathogens by isoalloLCA may be a potential factor contributing to longevity.

These bile acids can be used as biomarkers to monitor health and predict life span, regardless of whether an increase in bacteria that produce isoalloLCA, isoLCA, or 3-oxoLCA contributes to life span as a result of longevity.

In addition, by using the unique ability of the bacterial strains identified in the present Examples to metabolize bile acids, it is possible to logically manipulate bile acid pools and ultimately improve infectious diseases caused by gram-positive pathogens such as antibiotic-resistant C. difficile, VRE, and MRSA.

### (Example 7) Effects of gut microbiomes on testosterone metabolism

As described above, the present inventors have found bacteria and enzymes (5AR) produced by them involved in the reduction of 3-oxo-Δ⁴-LCA to 3-oxoalloLCA. Further, as shown in (Example 3), human 5AR is known to catalyze the enzymatic reduction of testosterone to 5α-dihydrotestosterone (DHT), which is similar to the reduction to 3-oxoalloLCA. Therefore, the present inventors focused on this similarity and predicted that the above-mentioned bacterial-derived enzyme might also be involved in the metabolism of testosterone, as shown below.

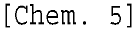

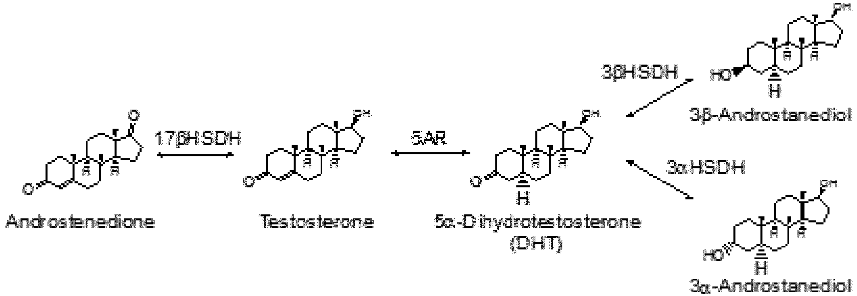

Therefore, the ability of the above-mentioned bacterial-derived enzyme to metabolize steroid hormones (such as testosterone) was verified using the above-mentioned P. merdae St3, Odoribacteraceae St21-24, and their enzyme-deficient mutants.

As a result, the addition of testosterone resulted in the production of DHT and 3β-androstanediol (corresponding to isoalloLCA) in wild-type strains. However, P. merdae Δ5AR was unable to produce them. Instead, androstenedione accumulation was detected in P. merdae Δ5AR (Fig. 19A).

In addition, when Odoribacteraceae St21-24 was cultured in vitro in the presence of testosterone, it was revealed that testosterone was immediately converted to DHT and then converted to 3β-androstanediol within 3 to 12 hours of incubation (Fig. 19B).

Stronger 3βHSDH enzyme activity of Odoribacteraceae St21-24 was detected by the addition of DHT as compared to P. merdae. P. merdae did not produce metabolites, but Odoribacteraceae St21-2 showed a large amount of metabolites within 3 hours of incubation (Fig. 19C).

In addition, in all the strains tested, a preferential metabolic reaction from DHT to 3β-androstanediol rather than to 3α-androstanediol was observed (Figs. 19A-19C). In addition, testosterone was oxidized to androstenedione by P. merdae and Odoribacteraceae St21-24, which are presumed to have the presence of an enzyme with the functions of 17βHSDH.

Prostate cancer is the fifth leading cause of cancer death in men, with an estimated 1.2 million new cases reported worldwide in 2018 [61]. Tumor progression in prostate cancer is mediated by androgen receptor (AR) signaling in glandular cells. Androgen deprivation therapy is currently used as the primary treatment to reduce testicular androgen levels to suppress prostate cancer cells, but the majority of patients eventually develop hormone-refractory tumors [62]. Although both testosterone and DHT are ligands for AR, DHT is a more potent androgen and binds to AR with high affinity [28]. It has been reported that 3β-androstanediol metabolized by 3βHSDH activates estrogen receptor β (ERβ) without binding to AR and inhibits prostate cancer cell migration by reducing E-cadherin expression [63, 64].

Regarding intestinal microbiota and its roles in steroid hormone metabolism the present inventors have demonstrated, as described above, that Odoribacteraceae strains isolated from centenarians have the ability to rapidly metabolize testosterone and DHT to 3β-androstanediol.

Thus, the Odoribacteraceae strain may have a therapeutic role in prostate cancer by depleting tumor-inducing testosterone and DHT while presumably producing metastasis-resistant 3β-androstanediol.

Rapid conversion of DHT by the Odoribacteraceae strain has the potential to treat various diseases requiring anti-androgen treatment, such as androgenetic alopecia, polycystic ovary syndrome, acne, and hirsutism [65].

Furthermore, it is thought that activation of ERβ by 3β-androstanediol has growth-inhibiting and chemotherapy-enhancing effects on ovarian endothelial cancer [66], and activation of ERβ in breast cancer cells generally has an antiproliferative effect on disease progression [67].

In addition to the hormonal effects of the Odoribacteraceae strains, the 3β-androstanediol produced by these strains also has potential therapeutic potential as neurosteroids [68, 69].

In conclusion, hormonal and neurochemical effects of testosterone-derived metabolites produced by certain intestinal microbiota such as the Odoribacteraceae strain can be expected, and this may lead to transformative therapy to improve human health.

### [References]

1. Tindale, L. C., Salema, D. & Brooks-Wilson, A. R. 10-year follow-up of the Super-Seniors Study: Compression of morbidity and genetic factors. BMC Geriatr. 19, 58 (2019).
2. Arai, Y. et al. Inflammation, but not telomere length, predicts successful ageing at extreme old age: A longitudinal study of semi-supercentenarians. EBioMedicine 2, 1549 to 1558 (2015).
3. Andersen, S. L., Sebastiani, P., Dworkis, D. A., Feldman, L. & Perls, T. T. Health span approximates life span among many supercentenarians: Compression of morbidity at the approximate limit of life span. Journals Gerontol. - Ser. A Biol. Sci. Med. Sci. 67 A, 395 to 405 (2012).
4. Barcena, C. et al. Healthspan and lifespan extension by fecal microbiota transplantation into progeroid mice. Nat. Med. 25, 1234 to 1242 (2019).
5. Biagi, E. et al. Gut Microbiota and Extreme Longevity. Curr. Biol. 26, 1480 to 1485 (2016).
6. Claesson, M. J. et al. Gut microbiota composition correlates with diet and health in the elderly. Nature 488, 178 to 184 (2012).
7. Rampelli, S. et al. Shotgun metagenomics of gut microbiota in humans with up to extreme longevity and the increasing role of xenobiotic degradation. mSystems 5, e00124-20 (2020).
8. Wu, L. et al. A cross-sectional study of compositional and functional profiles of gut microbiota in Sardinian centenarians. mSystems 4, e00325-19 (2019).
9. Nicoletti, C. Age-associated changes of the intestinal epithelial barrier: Local and systemic implications. Expert Rev. Gastroenterol. Hepatol. 9, 1467 to 1469 (2015).
10. Huang, Z. & Kraus, V. B. Does lipopolysaccharide-mediated inflammation have a role in OA? Nat. Rev. Rheumatol. 12, 123 to 129 (2016).
11. Morais, L. H., Schreiber, H. L. & Mazmanian, S. K. The gut microbiota-brain axis in behaviour and brain disorders. Nat. Rev. Microbiol. (2020).
12.Franceschi, C., Garagnani, P., Parini, P., Giuliani, C. & Santoro, A. Inflammaging: a new immune to metabolic viewpoint for age-related diseases. Nat. Rev. Endocrinol. 14, 576 to 590 (2018).
13. Santoro, A. et al. Microbiomes other than the gut: inflammaging and age-related diseases. Semin. Immunopathol. (2020).
14. Claesson, M. J. et al. Composition, variability, and temporal stability of the intestinal microbiota of the elderly. Proc. Natl. Acad. Sci. U. S. A. 108, 4586 to 4591 (2011).
15. Hirata, T. et al. Associations of cardiovascular biomarkers and plasma albumin with exceptional survival to the highest ages. Nat. Commun. 11, 1 to 17 (2020).
16. Bloom, D. E. & Cadarette, D. Infectious disease threats in the twenty-first century: Strengthening the global response. Front. Immunol. 10, 549 (2019).
17. Lloyd-Price, J. et al. Multi-omics of the gut microbial ecosystem in inflammatory bowel diseases. Nature 569, 655 to 662 (2019).
18. Devlin, A. S. & Fischbach, M. A. A biosynthetic pathway for a prominent class of microbiota-derived bile acids. Nat. Chem. Biol. 11, 685 to 690 (2015).
19. Ridlon, J. M., Kang, D.-J. J. & Hylemon, P. B. Bile salt biotransformations by human intestinal bacteria. J. Lipid Res. 47, 241 to 259 (2006).
20. Kitahara, M., Takamine, F., Imamura, T. & Benno, Y. Assignment of Eubacterium sp. VPI 12708 and related strains with high bile acid 7α-dehydroxylating activity to Clostridium scindens and proposal of Clostridium hylemonae sp. nov., isolated from human faeces. Int. J. Syst. Evol. Microbiol. 50, 971 to 978 (2000).
21. Li, F., Hullar, M. A. J., Schwarz, Y. & Lampe, J. W. Human gut bacterial communities are altered by addition of cruciferous vegetables to a controlled fruit- and vegetable-free diet. J. Nutr. 139, 1685 to 1691 (2009).
22. Duncan, S. H. et al. Reduced dietary intake of carbohydrates by obese subjects results in decreased concentrations of butyrate and butyrate-producing bacteria in feces. Appl. Environ. Microbiol. 73, 1073 to 1078 (2007) .
23. David, L. A. et al. Diet rapidly and reproducibly alters the human gut microbiome. Nature 505, 559 to 563 (2014).
24. De Aguiar Vallim, T. Q., Tarling, E. J. & Edwards, P. A. Pleiotropic roles of bile acids in metabolism. Cell Metab. 17, 657 to 669 (2013).
25. Wahlstrom, A., Sayin, S. I., Marschall, H.-U. U. & Backhed, F. Intestinal crosstalk between bile acids and microbiota and Its impact on host metabolism. Cell Metab. 24, 41 to 50 (2016).
26. Funabashi, M. et al. A metabolic pathway for bile acid dehydroxylation by the gut microbiome. Nature 582, 566 to 570 (2020).
27. Ridlon, J. M., Kang, D. J. & Hylemon, P. B. Isolation and characterization of a bile acid inducible 7α-dehydroxylating operon in Clostridium hylemonae TN271. Anaerobe (2010).
28. Nixon, M., Upreti, R. & Andrew, R. 5α-Reduced glucocorticoids: A story of natural selection. J. Endocrinol. 212, 111 to 127 (2012).
29. Garcia-Bayona, L. & Comstock, L. E. Streamlined genetic manipulation of diverse bacteroides and parabacteroides isolates from the human gut microbiota. MBio 10, e01762-19 (2019) .
30. Hang, S. et al. Bile acid metabolites control TH17 and Treg cell differentiation. Nature 576, 143 to 148 (2019).
31. Campbell, C. et al. Bacterial metabolism of bile acids promotes generation of peripheral regulatory T cells. Nature 581, 475 to 479 (2020).
32. Song, X. et al. Microbial bile acid metabolites modulate gut RORγ+ regulatory T cell homeostasis. Nature 577, 410 to 415 (2020).
33. Brestoff, J. R. & Artis, D. Commensal bacteria at the interface of host metabolism and the immune system. Nat. Immunol. 14, 676 to 684 (2013).
34. Skelly, A. N., Sato, Y., Kearney, S. & Honda, K. Mining the microbiota for microbial and metabolite-based immunotherapies. Nat. Rev. Immunol. 19, 305 to 323 (2019).
35. Chen, M. L., Takeda, K. & Sundrud, M. S. Emerging roles of bile acids in mucosal immunity and inflammation. Mucosal Immunol. 12, 851 to 861 (2019).
36. Buffie, C. G. et al. Precision microbiome reconstitution restores bile acid mediated resistance to Clostridium difficile. Nature 517, 205 to 208 (2015).
37. Begley, M., Gahan, C. G. M. & Hill, C. The interaction between bacteria and bile. FEMS Microbiol. Rev. 29, 625 to 651 (2005).
38. Sung, J. Y., Shaffer, E. A. & Costerton, J. W. Antibacterial activity of bile salts against common biliary pathogens - Effects of hydrophobicity of the molecule and in the presence of phospholipids. Dig. Dis. Sci. 38, 2104 to 2112 (1993).
39. Urdaneta, V. & Casadesus, J. Interactions between bacteria and bile salts in the gastrointestinal and hepatobiliary tracts. Front. Med. 4, 163 (2017).
40. Thanissery, R., Winston, J. A. & Theriot, C. M. Inhibition of spore germination, growth, and toxin activity of clinically relevant C. difficile strains by gut microbiota derived secondary bile acids. Anaerobe 45, 86 to 100 (2017).
41. Abt, M. C., McKenney, P. T. & Pamer, E. G. Clostridium difficile colitis: Pathogenesis and host defence. Nat. Rev. Microbiol. 14, 609 to 620 (2016).
42. Cushnie, T. P. T., O'Driscoll, N. H. & Lamb, A. J. Morphological and ultrastructural changes in bacterial cells as an indicator of antibacterial mechanism of action. Cell. Mol. Life Sci. 73, 4471 to 4492 (2016).
43. Piewngam, P. et al. Pathogen elimination by probiotic Bacillus via signalling interference. Nature 562, 532 to 537 (2018).
44. Theriot, C. M. et al. Antibiotic-induced shifts in the mouse gut microbiome and metabolome increase susceptibility to Clostridium difficile infection. Nat. Commun. 5, 3114 (2014).
45. Alavi, S. et al. Interpersonal Gut Microbiome Variation Drives Susceptibility and Resistance to Cholera Infection. Cell 181, 1533-1546.e13 (2020).
46. Li, D., Liu, C. M., Luo, R., Sadakane, K. & Lam, T. W. MEGAHIT: An ultra-fast single-node solution for large and complex metagenomics assembly via succinct de Bruijn graph. Bioinformatics 31, 1674 to 1676 (2015).
47. Hyatt, D. et al. Prodigal: Prokaryotic gene recognition and translation initiation site identification. BMC Bioinformatics 11, 119 (2010).
48. Fu, L., Niu, B., Zhu, Z., Wu, S. & Li, W. CD-HIT: Accelerated for clustering the next-generation sequencing data. Bioinformatics 28, 3150 to 3152 (2012).
49. Qin, J. et al. A human gut microbial gene catalogue established by metagenomic sequencing. Nature 464, 59 to 65 (2010).
50. Li, H. & Durbin, R. Fast and accurate short read alignment with Burrows-Wheeler transform. Bioinformatics 25, 1754 to 1760 (2009).
51. Onate, F. P. et al. MSPminer: Abundance-based reconstitution of microbial pan-genomes from shotgun metagenomic data. Bioinformatics 35, 1544 to 1552 (2019).
52. Li, J. et al. An integrated catalog of reference genes in the human gut microbiome. Nat. Biotechnol. 32, 834 to 841 (2014).
53. Segata, N., Bornigen, D., Morgan, X. C. & Huttenhower, C. PhyloPhlAn is a new method for improved phylogenetic and taxonomic placement of microbes. Nat. Commun. 4, 2304 (2013).
54. Khelaifia, S., Raoult, D. & Drancourt, M. A versatile medium for cultivating methanogenic archaea. PLoS One 8, e61563 (2013).
55. Aziz, R. K. et al. The RAST Server: Rapid annotations using subsystems technology. BMC Genomics 9, 75 (2008).
56. Seemann, T. Prokka: Rapid prokaryotic genome annotation. Bioinformatics 30, 2068 to 2069 (2014).
57. Ferrieres, L. et al. Silent mischief: Bacteriophage Mu insertions contaminate products of Escherichia coli random mutagenesis performed using suicidal transposon delivery plasmids mobilized by broad-host-range RP4 conjugative machinery. J. Bacteriol. 192, 6418 to 6427 (2010).
58. Atarashi, K. et al. Treg induction by a rationally selected mixture of Clostridia strains from the human microbiota. Nature 500, 232 to 236 (2013).
59. Quinn, R. A. et al. Global chemical effects of the microbiome include new bile-acid conjugations. Nature 579, 123 to 129 (2020).
60. McDonald, J. A. K. et al. Evaluation of microbial community reproducibility, stability and composition in a human distal gut chemostat model. J. Microbiol. Methods 95, 167 to 174 (2013).
61. Bray, F. et al. Global cancer statistics 2018: GLOBOCAN estimates of incidence and mortality worldwide for 36 cancers in 185 countries. CA. Cancer J. Clin. (2018). doi:10.3322/caac.21492
62. Karantanos, T., Corn, P. G. & Thompson, T. C. Prostate cancer progression after androgen deprivation therapy: Mechanisms of castrate resistance and novel therapeutic approaches. Oncogene (2013).
63. Guerini, V. et al. The androgen derivative 5α-androstane-3β,17β-diol inhibits prostate cancer cell migration through activation of the estrogen receptor β subtype. Cancer Res. (2005).
64. Dondi, D. et al. Estrogen receptor β and the progression of prostate cancer: Role of 5α-androstane-3β,17β-diol. Endocr. Relat. Cancer (2010).
65. Karrer-Voegeli, S. et al. Androgen dependence of hirsutism, acne, and alopecia in women retrospective analysis of 228 patients investigated for hyperandrogenism. Medicine (Baltimore). (2009).
66. Pinton, G., Nilsson, S. & Moro, L. Targeting estrogen receptor beta (ERβ) for treatment of ovarian cancer: Importance of KDM6B and SIRT1 for ERβ expression and functionality. Oncogenesis (2018).
67. Zhou, Y. & Liu, X. The role of estrogen receptor beta in breast cancer. Biomarker Research (2020).
68. Reddy, D. S. & Jian, K. The testosterone-derived neurosteroid androstanediol is a positive allosteric modulator of GABAA receptors. J. Pharmacol. Exp. Ther. (2010) .
69. Huang, Q., Zhu, H., Fischer, D. F. & Zhou, J. N. An estrogenic effect of 5α-androstane-3β, 17β-diol on the behavioral response to stress and on CRH regulation.

Neuropharmacology (2008)

### [Industrial Applicability]

As described above, the present invention makes it possible to reduce the risk of infection with gram-positive bacteria, prostate cancer, and the like. Therefore, the present invention is useful in the development and the like of pharmaceutical products for these diseases.

## Claims

1. A composition for converting 3-oxo-Δ⁴-LCA to 3-oxoalloLCA, comprising: a bacterium having a polynucleotide encoding 3-oxo-5α-steroid-4-dehydrogenase (5AR) as an active ingredient.

2. A composition for converting 3-oxoalloLCA to isoalloLCA, comprising: a bacterium having a polynucleotide encoding 3β-hydroxysteroid dehydrogenase (3βHSDH) as an active ingredient.

3. A composition for producing isoalloLCA from 3-oxo-Δ⁴-LCA, comprising: a bacterium having a polynucleotide encoding 5AR and a bacterium having a polynucleotide encoding 3βHSDH as active ingredients.

4. A composition for producing isoalloLCA from 3-oxo-Δ⁴-LCA, comprising: a bacterium having a polynucleotide encoding 5AR and a polynucleotide encoding 3βHSDH as an active ingredient.

5. A bacterium comprising: a polynucleotide encoding 3-oxo-5β-steroid-4-dehydrogenase (5BR).

6. A composition for converting 3-oxo-Δ⁴-LCA to 3-oxoLCA, comprising: a bacterium having a polynucleotide encoding 5BR as an active ingredient.

7. A composition for producing isoalloLCA from isoLCA or 3-oxoLCA, comprising: a bacterium having a polynucleotide encoding 5AR, a polynucleotide encoding 3βHSDH, and a polynucleotide encoding 5BR as an active ingredient.

8. The composition according to any one of claims 1 to 7, which is an antibacterial composition against gram-positive bacteria.

9. An antibacterial composition against gram-positive bacteria, comprising: isoalloLCA or isoLCA as an active ingredient.

10. An antibacterial composition against gram-positive bacteria, comprising: at least one enzyme selected from 5AR, 3βHSDH, and 5BR as an active ingredient.

11. The composition according to any one of claims 8 to 10, which is a composition for treating or preventing infectious diseases of gram-positive bacteria.

12. The composition according to any one of claims 8 to 10, which is a composition for treating or preventing infectious diseases of Clostridium difficile.

13. The composition according to any one of claims 8 to 10, which is a composition for treating or preventing at least one disease selected from the group consisting of sepsis and acute respiratory distress syndrome (ARDS/ALI) having sepsis as an underlying disease.

14. A composition for converting 5α-dihydrotestosterone (DHT) to 3β-androstanediol, comprising: a bacterium having a polynucleotide encoding 3βHSDH as an active ingredient.

15. A composition for producing 3β-androstanediol from testosterone, comprising: a bacterium having a polynucleotide encoding 5AR and a bacterium having a polynucleotide encoding 3βHSDH as active ingredients.

16. A composition for producing 3β-androstanediol from testosterone, comprising: a bacterium having a polynucleotide encoding 5AR and a polynucleotide encoding 3βHSDH as an active ingredient.

17. A composition for producing 3β-androstanediol from DHT, comprising: 3βHSDH as an active ingredient.

18. A composition for producing 3β-androstanediol from testosterone, comprising: 5AR and 3βHSDH as active ingredients.

19. The composition according to any one of claims 14 to 18, which is a composition for treating or preventing at least one disease selected from the following disease group
disease group:
prostate cancer, androgenetic alopecia, polycystic ovary syndrome, acne, hirsutism, ovarian endothelial cancer, neuroinflammation, and GABAA receptor-mediated epilepsy.

20. A method for evaluating at least one disease selected from the following disease group
disease group:
prostate cancer, androgenetic alopecia, polycystic ovary syndrome, acne, hirsutism, ovarian endothelial cancer, neuroinflammation, and GABAA receptor-mediated epilepsy,
comprising:
(1) quantifying bacteria that produce 3βHSDH in feces of a subject;
(2) comparing a value obtained by quantifying in step (1) with a corresponding value obtained by quantifying the bacteria of a centenarian; and
(3) as a result of the comparison in step (2),
determining that the subject is unlikely to catch the disease when the quantitative value in the feces of the subject is equal to or higher than the corresponding value, and
determining that the subject is highly likely to catch or highly likely to have the disease when the quantitative value in the feces of the subject is lower than the corresponding value.

21. A method for preventing or treating at least one disease selected from the following disease group
disease group:
prostate cancer, androgenetic alopecia, polycystic ovary syndrome, acne, hirsutism, ovarian endothelial cancer, neuroinflammation, and GABAA receptor-mediated epilepsy,
comprising:
in the method according to claim 20, administering a bacterium that produces 3βHSDH to the subject who is determined to be highly likely to catch or highly likely to have the disease.

22. A method for evaluating a possibility of survival over 100 years old, comprising:
(1) quantifying at least one bile acid selected from the group consisting of 3-oxoLCA, isoalloLCA, 3-oxoalloLCA, alloLCA, 3-oxoLCA, and isoLCA in feces of a subject;
(2) comparing a value obtained by quantifying in step (1) with a corresponding value obtained by quantifying the bile acid of a centenarian; and
(3) as a result of the comparison in step (2),
determining that the subject has a high possibility of survival over 100 years old when the quantitative value in the feces of the subject is equal to or higher than the corresponding value, and
determining that the subject has a low possibility of survival over 100 years old when the quantitative value in the feces of the subject is lower than the corresponding value.

23. A method for evaluating a possibility of survival over 100 years old, comprising:
(1) quantifying bacteria that produce at least one enzyme selected from 5AR, 3βHSDH, and 5BR in feces of a subject;
(2) comparing a value obtained by quantifying in step (1) with a corresponding value obtained by quantifying the bacteria of a centenarian; and
(3) as a result of the comparison in step (2),
determining that the subject has a high possibility of survival over 100 years old when the quantitative value in the feces of the subject is equal to or higher than the corresponding value, and
determining that the subject has a low possibility of survival over 100 years old when the quantitative value in the feces of the subject is lower than the corresponding value.

24. A method for increasing a possibility of survival over 100 years old, comprising:
in the method according to claim 22 or 23, administering at least one selected from the group consisting of 3-oxoLCA, isoalloLCA, and bacteria that produce at least one enzyme selected from 5AR, 3βHSDH, and 5BR to the subject who is determined to have a low possibility of survival over 100 years old.

25. A method for evaluating resistance to gram-positive bacteria, comprising:
(1) quantifying at least one bile acid selected from the group consisting of 3-oxoLCA and isoalloLCA in feces of a subject;
(2) comparing a value obtained by quantifying in step (1) with a corresponding value obtained by quantifying the bile acid of a centenarian; and
(3) as a result of the comparison in step (2),
determining that the subject is highly resistant to gram-positive bacteria when the quantitative value in the feces of the subject is equal to or higher than the corresponding value, and
determining that the subject is lowly resistant to gram-positive bacteria when the quantitative value in the feces of the subject is lower than the corresponding value.

26. A method for evaluating resistance to gram-positive bacteria, comprising:
(1) quantifying bacteria that produce at least one enzyme selected from 5AR, 3βHSDH, and 5BR in feces of a subject;
(2) comparing a value obtained by quantifying in step (1) with a corresponding value obtained by quantifying the bacteria of a centenarian; and
(3) as a result of the comparison in step (2),
determining that the subject is highly resistant to gram-positive bacteria when the quantitative value in the feces of the subject is equal to or higher than the corresponding value, and
determining that the subject is lowly resistant to gram-positive bacteria when the quantitative value in the feces of the subject is lower than the corresponding value.

27. A method for preventing infectious diseases of gram-positive bacteria, comprising:
in the method according to claim 25 or 26, administering at least one selected from the group consisting of 3-oxoLCA, isoalloLCA, and bacteria that produce at least one enzyme selected from 5AR, 3βHSDH, and 5BR to the subject who is determined to be lowly resistant to gram-positive bacteria.

28. A method for producing isoalloLCA, comprising: culturing a bacterium having a polynucleotide encoding 3βHSDH in the presence of 3-oxoalloLCA and collecting isoalloLCA produced in the bacterium and/or a culture product thereof.

29. A method for producing isoalloLCA, comprising: culturing a bacterium having a polynucleotide encoding 5AR and a bacterium having a polynucleotide encoding 3βHSDH in the presence of 3-oxo-Δ⁴-LCA and collecting isoalloLCA produced in the bacteria and/or culture products thereof.

30. A method for producing isoalloLCA, comprising: culturing a bacterium having a polynucleotide encoding 5AR and a polynucleotide encoding 3βHSDH in the presence of 3-oxo-Δ⁴-LCA and collecting isoalloLCA produced in the bacterium and/or a culture product thereof.

31. A method for producing isoalloLCA, comprising: culturing a bacterium having a polynucleotide encoding 5AR, a polynucleotide encoding 3βHSDH, and a polynucleotide encoding 5BR in the presence of isoLCA or 3-oxoLCA and collecting isoalloLCA produced in the bacterium and/or a culture product thereof.
